# EUROPÄISCHE PATENTSCHRIFT

(11) **EP 2 094 711 B1**
(45) Veröffentlichungstag und Bekanntmachung des Hinweises auf die Patenterteilung: **19.03.2014**
(21) Anmeldenummer: 07817610.4
(22) Anmeldetag: 25.09.2007
(51) Int. Cl.: C07D 487/14, A61K 31/55, A61P 25/00, A61P 31/00, A61P 33/00

(54) **STRUKTURMIMETIKA PROLINREICHER PEPTIDE UND IHRE PHARMAZEUTISCHE VERWENDUNG**
STRUCTURAL MIMETICS OF PROLINE-RICH PEPTIDES AND THE PHARMACEUTICAL USE THEREOF
STRUCTURES MIMÉTIQUES DE PEPTIDES RICHES EN PROLINE, ET LEUR UTILISATION PHARMACEUTIQUE

(30) Priorität: 25.09.2006 EP 06090178; 25.09.2006 EP 06090179; 29.11.2006 DE 102006057070; 07.05.2007 EP 07090095
(43) Veröffentlichungstag der Anmeldung: 02.09.2009
(73) Patentinhaber: Forschungsverbund Berlin e.V., 12489 Berlin (DE)
(72) Erfinder: KÜHNE, Ronald, 12683 Berlin (DE); OSCHKINAT, Hartmut, 13098 Berlin (DE); BROCKMANN, Christoph, Cambridge CB1 3QB (GB); SCHMALZ, Hans-Günther, 50321 Brühl (DE)
(74) Vertreter: Boeckh, Tobias
(86) Internationale Anmeldenummer: PCT/DE2007/001768
(87) Internationale Veröffentlichungsnummer: WO 2008/040332

(56) Entgegenhaltungen:
- EP-A- 1 077 218
- WO-A-2006/092722

## Beschreibung

Die Erfindung betrifft Verbindungen, die insbesondere als Strukturmimetika prolinreicher Peptide eingesetzt werden können und demgemäß in der Lage sind, PRM-Bindungsdomänen (proline-rich-motif binding domains) von Proteinen zu binden, die Erfindung betrifft weiterhin die Verwendung dieser Verbindungen als pharmazeutische Wirkstoffe sowie die Verwendung der pharmazeutischen Wirkstoffe zur Behandlung von bakteriellen Erkrankungen, neurodegenerativen Erkrankungen sowie Tumoren.

Peptide und Proteine sind essentielle Bestandteile von Organismen mit einer Vielzahl verschiedener Funktionen. Während den Proteinen vor allem biokatalytische Aufgaben (Enzyme) sowie solche als wichtige Gewebebestandtele zukommen, erfüllen die Peptide vor allem als Hormone, Neurotransmitter und Neuromodulatoren wichtige Funktionen im Organismus. Durch Bindung an membrangebundene Rezeptoren und dadurch vermittelte zellphysiologische Folgereaktionen beeinflussen Peptide die Zell-Zell-Kommunkaton und kontrollieren eine Vielzahl vitaler Prozesse wie Stoffwechsel, Immunabwehr, Verdauung, Atmung, Schmerzempfinden, Reproduktion, Verhalten, Elektrolythaushalt und mehr.

Es besteht daher im Stand der Technik das Bedürfnis, die genauen Zusammenhänge im Organismus aufzuklären und gleichzeitig eine notwendige Grundlage für die Therapierbarkeit pathogener Zustände bereitzustellen. Mit einem zunehmenden Verständnis biologischer Prozesse auf molekularer Ebene hat auch die Verflechtung von Biologie und Chemie zugenommen, unterstützt durch große Fortschritte bei den analytischen Verfahren und den rechnergestützten theoretischen Methoden. All dies sind wichtige Voraussetzungen für die erfolgreiche Identifizierung von Leitstrukturen in der Wirkstoffentwicklung. Dennoch ist man vom eigentlichen Ziel, dem einfachen und effizienten de *novo*-Design von Wirkstoffen noch weit entfernt. Vielmehr bedarf es in der Regel eines großen empirischen Forschungsaufwandes, um ausgehend von natürlichen Strukturen Bibliotheken möglicher Zielsubstanzen zu synthetisieren und diese auf eine bestimmte Wirkung hin zu optimieren. Neben hohem Zeitaufwand und enormen Kosten stellt sich zudem oft heraus, dass mithilfe von Computern entwickelte Wirkstoffe in realen, sehr komple-xen biologischen Systemen (z. B. Menschen) die erwünschte Wirkung nur unzureichend erzielen oder nicht zu tolerierende Nebenwirkungen haben.

Im Stand der Technik sind peptidomimetische Komponenten und die Herstellung von biologisch aktiven Derivaten, die die Sequenz RGD umfassen, beschrieben worden. Diese zyklischen peptidomimetischen Komponenten haben eine azabicycloalkane Struktur und können zur Behandlung von angiogenetischen Phänomenen eingesetzt werden. Besonders gute Eigenschaften weisen diese als Antagonisten bestimmter Integrine auf (WO 2006/092722, WO 2005/042531, EP 1 077 218).

Vor diesem Hintergrund bleibt die Entwicklung besonders auch peptidischer bzw. peptidmimetischer Wirkstoffe eine große Herausforderung, auch in synthetischer Hinsicht; denn im vielfältigen interdisziplinären Zusammenspiel bestimmt nicht zuletzt die organische Chemie mit ihren Möglichkeiten und Beschränkungen den Zugang zu den gewünschten Zielmolekülen. Da diese in möglichst wenigen Stufen und in der Regel stereoselektiv aufgebaut werden müssen, bedarf es stetig neuer und besserer Synthesemethoden, um dieses Ziel nicht nur im Labor, sondern nachfolgend auch im Hinblick auf eine großtechnische Anwendung zu erreichen.

Es war daher die Aufgabe der Erfindung, Verbindungen bereitzustellen, die als Mimetika für Prolinreiche Peptide eingesetzt werden können, insbesondere wenn diese eine PPII-Helix-Konformation aufweisen. Die Prolin-Prolin-Dipeptideinheiten, insbesondere mit einer PPII-Helix-Konformation, können bevorzugt als Liganden für so genannte PRM-Bindungsdomänen fungieren (PRM = proline-rich motifs).

Das erfindungsgemäße Problem wird überraschend gelöst durch die Bereitstellung einer Verbindung gemäß der allgemeinen Formel 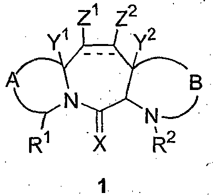 mit einem gesättigten oder ungesättigten zentralen siebengliedrigen Ring, wobei
X = O und/oder S sind;
A, B = Ringbrücken sind;
Y¹ , Y² = H, Alkyl, Fluoralkyl, Aryl und/oder Heteroaryl sind;
Z¹ , Z² = H; Carbonyl; OH; O-Alkyl; O-Acyl; N-R¹R² (mit R¹ bzw. R² = H, Alkyl, Acyl, Sulfonyl); Alkyl; Acyl; Fluoralkyl; Aryl und/oder Heteroaryl sind;
R¹ =Alkyl, Acyl, Alkoxycarbonyl, Aryloxycarbonyl und/oder Aminocarbonyl (CONH₂, CONHR, CONH-Peptidyl, (mit R)) sind;
R² = H, Alkyl, Aryl, Alkylcarbonyl, Arylcarbonyl, Alkoxycarbonyl, Aminocarbonyl, Aryloxycarbonyl, Alkyl-sulfonyl, Arylsulfonyl, Aminoacyl und/oder Peptidyl sind.

Es war völlig überraschend, dass die erfindungsgemäßen Verbindungen die Nachteile des Standes der Technik nicht aufweisen. Neben den o. g. Nachteilen des Standes der Technik war bisher der Einsatz peptidischer Wirkstoffe als Medikamente durch eine Reihe weiterer Faktoren eingeschränkt
1) geringe metabolische Stabilität durch Proteolyse im Magen-Darm-Trakt und im Serum,
2) schlechte Resorption nach oraler Einnahme, vor allem aufgrund der hohen Molekülmasse,
3) schnelle Ausscheidung durch Leber und Nieren und
4) mangelnde Selektivität durch Wechselwirkung mit unterschiedlichen Rezeptoren.

Die erfindungsgemäßen Verbindungen können überraschend als Mimetika eingesetzt werden, die als künstlich hergestellte Stoffe in der Lage sind, insbesondere die Funktion eines Rezeptorliganden auszuüben und den biologischen Effekt eines Peptides zu imitieren (Agonist) oder zu blockieren (Antagonist). Mimetika im Sinne der Erfindung sind chemische Verbindungen, die funktionsanalog zu Prolinen oder prolinreichen Peptiden sind und demgemäß mit den entsprechenden Domänen interagieren, wie es Proline oder prolinreiche Peptide tun würden und bevorzugt die gleiche Wirkung wie die Proline oder prolinreichen Peptide auslösen.

Die Begriffe "Funktionsanaloge" oder "Mimetika" sind keine relativen Begriffe, da die betreffenden Ausdrücke auf dem Fachgebiet der Biologie eine allgemein anerkannte Bedeutung haben. Die Begriffe "Funktionsanaloge" oder "Mimetika" können gemäß der Erfindung bevorzugt als Äquivalente verstanden werden. Das heißt, Mimetika sind im Vergleich zur natürlichen Struktur Varianten, die im wesentlichen dieselbe Funktion auf im wesentlichen demselben Weg mit im wesentlichen demselben Ergebnis hervorbringen.

Obwohl das Prinzip der Bereitstellung von Peptidmimetika bekannt ist, sind erst sehr wenige Beispiele für PPII-Struktur-Mimetika offenbart worden, die allerdings zahlreiche Nachteile aufweisen. Auch wenn es verschiedene Ansätze gab, das Strukturmotiv der PPII-Helix ganz oder teilweise durch synthetische Analoga zu ersetzen, war es mit den bekannten Strukturen nicht oder nur sehr bedingt möglich, ihre Wechselwirkung mit verschiedenen Proteindomänen zu untersuchen. Der Stand der Technik lehrt einen Fachmann mit durchschnittlichem Wissen auch, dass die Wechselwirkung prolinreicher Helicis mit verschiedenen Proteindomänen keine besondere biologische Bedeutung aufweist. Es ist das Verdienst der Erfinder gezeigt zu haben, dass diese Wechselwirkung bzw. ihre Modifikation mit zahlreichen Erkrankungen wie bakteriellen oder viralen Infektionen, neurodegenerativen Erkrankungen oder der Ausbildung von Tumoren einhergeht. Zahlreiche im Stand der Technik bekannte Mimetika können nur mithilfe von Katalysatoren bereitgestellt werden, die aber oft nur mit Schwierigkeiten oder gar nicht aus dem System entfernt werden können. Weiterhin sei darauf hingewiesen, dass die bekannten Produkte keine hohe Lagerbeständigkeit aufweisen und oft mit Produkten, die für die Synthese notwendig sind, kontaminiert vorliegen, was insbesondere einen Einsatz in der Medizin nicht ermöglichen oder erschweren würde. Des weiteren ist die Bindungsaffinität der bisher bekannten Mimetika unzureichend.

Die Wechselwirkung von Peptidliganden mit Proteinrezeptoren spielt eine wichtige Rolle bei der Regulation biologischer Prozesse und hängt in entscheidender Weise von der Peptidgeometrie ab. Unter physiologischen Bedingungen steht die Konformation eines linearen Peptids durch Rotation um einzelne Bindungen in einem dynamischen Gleichgewicht, das vom pH-Wert und von der Temperatur abhängt. Dies führt dazu, dass die biologische Reaktivkonformation nur zu einem geringen Prozentsatz vorliegt.

Die Konformation des Peptidrückgrats wird üblicherweise durch die drei Winkel ϕ (phi), ψ (psi) und ω (omega) beschrieben. Aufgrund des partiellen Doppelbindungscharakters ist die Peptidbindung in ihrer Rotation gehindert und weist eine planare Geometrie auf, was zu zwei Vorzugskonformationen führt: der *trans-* und der *cis*- Peptidbindung mit w = 180° bzw. ω = 0°, wobei die *trans*-Konformation energetisch günstiger ist und deshalb überwiegt.

Daher genügen zur Konformationsbeschrebung des Peptidrückgrats in erster Näherung die Torsionswinkel ϕ und ψ der Aminosäurereste. Der Winkel ϕ, der die Rotation entlang der N-C_{α}-Bindung beschreibt, wird durch die vier Atome C(=O)-N-C_{α}-C(=O) definiert. In gleicher Weise definieren N-C_{α}-C(=O)-N den Winkel ψ, der die Rotation um die C_{α}-C(=O)-Bindung beschreibt Obwohl theoretisch eine große Anzahl verschiedener Kombinationen aus ϕ und ψ möglich ist, existieren im allgemeinen in Peptiden bestimmte Vorzugskonformationen in Abhängigkeit von Größe, Polarität und Ladung der Seitenketten, was zur Ausbildung der bekannten Sekundärstrukturen wie α-Helix, β-Faltblatt, β-Tum etc. führt.

### Die Aminosäure Prolin als Bestandteil von Peptiden

Unter den zwanzig natürlich vorkommenden Aminosäuren nimmt Prdin als einzige sekundäre Aminosäure eine Sonderstellung ein. Durch die Cyclisierung der α-Seitenkette an den Amidstickstoff ist der Torsionswinkel ϕ = (-65 ± 15°) als Bestandteil des fünfgliedrigen Ringes relativ eingeschränkt, das Peptid hat folglich weniger Rotationsfreiheitsgrade. Die zweifache Alkylierung des Stickstoffs hat einerseits zur Folge, dass das sonst übliche Amidproton (im Peptidrückgrat) fehlt und Prolin deshalb als Wasserstoffbrücken-Donor ausscheidet, andererseits ist die Carbonylgruppe besonders elektronenreich und deshalb ein besserer Wasserstoffbrücken-Akzeptor als bei anderen Aminosäuren. Durch diese geometrischen und elektronischen Eigenschaften kann Prolin keine α-Helix stabilisieren ("α-Helix-Brecher") und bildet auch keine β-Faltblattstruktur ("β-Faltblatt-Brecher") sondern ist bevorzugt in anderen typischen Sekundärstrukturen anzutreffen: den sogenannten β-Turns und der Poly-Prolin-Helix (PPII-Helix).

### Prolinreiche Motive und die PPII-Helix als Sekundärstruktur

Prolinreiche Aminosäuresequenzen findet man häufig in Peptiden, die in intrazelluläre Signaltransduktionsvorgänge involviert sind. Dabei handelt es sich um Sequenzen, in denen ausschließlich oder überwiegend Prolin auftritt (oft vier und mehr Prolineinheiten in Folge).

Dadurch wird die charakteristische Sekundärstruktur, die Polyprolin-Helix oder kurz PPII-Helix induziert, worunter man eine gestreckte linksgängige Helix mit den Torsionswinkeln ϕ =-78° und ψ =+146° des Peptidrückgrats versteht. Als Konsequenz ergibt sich eine pseudo=C₃-Rota-tionssymmetrie um die Helixachse mit genau drei Prolinresten pro Drehung im Querschnitt (siehe Figuren), weshalb in prolinreichen Sequenzen die Prolinreste sich bevorzugt mindestens mit einer Periodizität von drei wiederholen (z.B. PxxPxxP oder PPxPPxPPx).

Auf diese Weise sind die Prolin-Seitenketten und die Carbonylgruppen des Peptidrückgrats in regelmäßigen Intervallen dem Lösungsmittel ausgesetzt. Bedingt durch das Fehlen intramolekularer Wasserstoffbrücken sind die Carbonylgruppen besonders geeignet, intermolekulare Wasserstoffbrückenbindungen zu Rezeptorproteinen einzugehen.

Das Strukturmotiv der PPII-Helix kann aber auch dann induziert werden, wenn nicht ausschließlich Prolin anwesend ist. Besonders häufig tritt in PPII-Helices und in deren Nähe die Aminosäure Glu auf, aber auch Gln, Arg, Ala, Leu, Ser, Asp und His werden gefunden. Der bevorzugte Bindungsmodus zwischen Domäne und Ligand bestimmt, welche Prolin-Positionen streng konserviert sein müssen und welche ggf. durch andere Aminosäuren ersetzt sein können.

In einer bevorzugten Ausführungsform der Erfindung ist diese so ausgewählt, dass A und/oder B 5 und/oder 6 Ringatome sind, wobei die durch A und B repräsentierten Ringglieder ausgewählt sind aus der Gruppe umfassend C-, O-, S- und/oder N-Atome. Selbstverständlich kann es auch bevorzugt sein, dass die Ringbrücken A, B so gewählt sind, dass sich ein 4-, 5- oder 6-gliedriger Ring ergibt, wobei die Ringglieder aus -CH2-, -O-, -S- und -N-R mit R = H, Alkyl, oder Acyl bestehen.

In einer weiteren bevorzugten Ausführungsform der Erfindung besitzt die Verbindung die allgemeine Formel 2 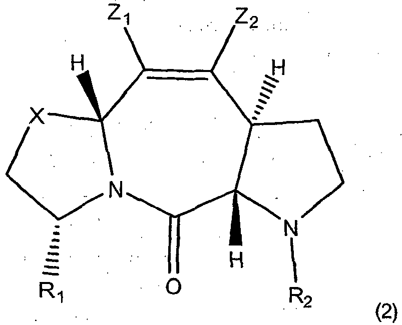
mit Z¹, Z² wie für Struktur 1 angegeben und der im Formelbild 2 gezeigten Konfiguration,
mit R¹, R² =Alkyl, Acyl, Hetaryl und/oder Sulfonyl,
mit X =-CH₂-, -O-, -S- und/oder-NH-R.

In einer weiteren bevorzugten Ausführungsform der Erfindung entspricht die Verbindung der Formel 3 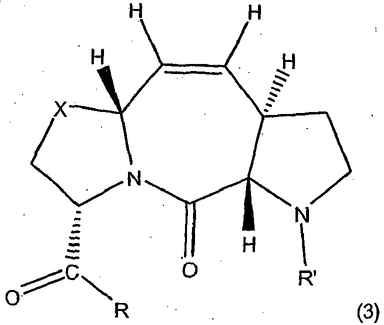
mit X = -CH₂-, -O- und/oder -S-,
mit R = NH-R", -O-R", mit R" = Peptidyl, substituierte Alkyle und/oder Hetaryl,
mit R' = Acyl, Peptidyl und/oder Sulfonyl.

Besonders bevorzugt ist in der Formel 3 R' ein Peptidyl.

Die Erfindung betrifft in einem bevorzugten Aspekt die Verwendung der genannten Verbindungen als pharmazeutische Wirkstoffe. Die Verwendung als pharmazeutischer Wirkstoff betrifft die Verwendung für chirurgische, therapeutische oder diagnostische Verfahren.

Die Erfindung betrifft in einem weiteren Aspekt ein pharmazeutisches Mittel, das die erfindungsgemäßen Verbindungen gegebenenfalls zusammen mit einem pharmazeutisch verträglichen Träger umfasst.

Bevorzugte pharmazeutische Träger sind beispielsweise Füllmittel, Streckmittel, Bindemittel, Feuchthaltemittel, Lösungsverzögerer, Sprengmittel, Resorptionsbeschleuniger, Netzmittel, Absorptionsmittel und/oder Gleitmittel.

In einem weiteren Aspekt betrifft die Erfindung die Verwendung der erfindungsgemäßen Verbindungen als Ligand für eine Domäne ausgewählt aus der Gruppe umfassend Src-Homologie-3-Domänen, WW-Domänen, Ena-VASP-Homologie-1-Domänen, GYF-Domänen, UEV-Domänen und/oder Profilin.

Bevorzugt interagieren alle diese Domänen inbesondere mit prolinreichen Sequenzen mit Affinitäten zwischen 1 und 500 µM und benötigen in bestimmten Ausfühurngsformen der Erfindung gegebenenfalls weitere flankierende Epitope, um die nötige Spezifität zu erreichen. Die Bindungen zwischen Liganden, Peptid und Domäne kommt bevorzugt vor allem durch die Interaktion zweier vorgeformter hydrophöber Oberflächen zustande. An der Oberfläche der Domänenproteine kommt es vorteilhafterweise zu einer Ansammlung aromatischer Aminosäuren, deren Reste eine hydrophobe Bindungstasche bilden. Der prolinreiche Peptidligand weist durch die starre Natur der PPII-Helix eine geometrisch fixierte, komplementäre Struktur auf, die mit der Domänenoberfläche in Kontakt tritt. Dabei kommt es vorteilhafterweise nicht über die ganze Länge des Kernmotivs gleichermaßen zu hydrophoben Kontakten, vielmehr bildet das Ligandenpeptid eine schirmartige Struktur, die die Domäne überspannt. Einige der Prolin-Reste werden in den hydrophoben Bindungstaschen aufgenommen und interagieren so mit den aromatischen Resten der Domäne. Wenn diese Kontakte für eine biologisch relevante Bindungsstärke nicht ausreichen, werden mit Vorteil zusätzlich stabilisierende Wasserstoff-Brückenbindungen ausgebildet, was durch die elektronenreiche Carbonylgruppe des Prolins besonders gut ermöglicht wird.

Energetisch wird die intermolekulare Bindung durch die eingeschränkte Flexibilität der PPII-Helix begünstigt, da durch den relativ hohen Ordnungsgrad die Entropieabnahme bei der Bindungsbildung geringer ist als bei einem gewöhnlichen linearen Peptid. Zur Quantifizierung dieses Energiebeitrages kann man ein Dipeptid xP betrachten, das nur zwei der sonst üblichen vier Rotationsfreiheitsgrade um das Peptidrückgrät besitzt. Da jeder Rotationsfreiheitsgrad bei 300 K etwa 3,5 kJ/mol entspricht ergibt sich pro xP-Dipeptid ein Energievorteil von ca. 7 kJ/mol bei der Komplexbildung.

Eine weitere Affinitätssteigerung beobachtet man auch durch die mehrfache Wiederholung der prolinreichen Sequenzen in einem einzigen Peptid, wie z.B. dem bakteriellen Oberflächenprotein ActA, das an die EVH1-Domäne (Ena-VASP-Homologie-1-Domäne) bindet

Domänen im Sinne der Erfindung sind insbesondere Src-Homologie-3-Domänen, WW-Domänen, EnaVASP-Homologie-1-Domänen, GYF-Domänen, UEV-Domänen und/oder Profilin.

Die EVH1-Domäne besteht aus etwa 115 Aminosäuren und kommt in einer Vielzahl signalgebender Multidomänenproteine vor. Neben einigen weiteren gehört dazu auch die Familie der EnaNASP-Proteine, die als molekulare Adaptoren wirken und die Actindynamik des Cytoskeletts modulieren. Man unterteilt die EVH1-Domänen nach ihrer Ligandenpräferenz in drei Klassen. Die erste Klasse erkennt insbesondere spezifisch ein FPPPP-Kernmotiv, das sich in allen fokalen Adhäsionsproteinen wie Vinculin und Zyxin wie auch im ActA-Protein des intrazellulären Bakteriums *Listeria monocytogenes* wieder findet

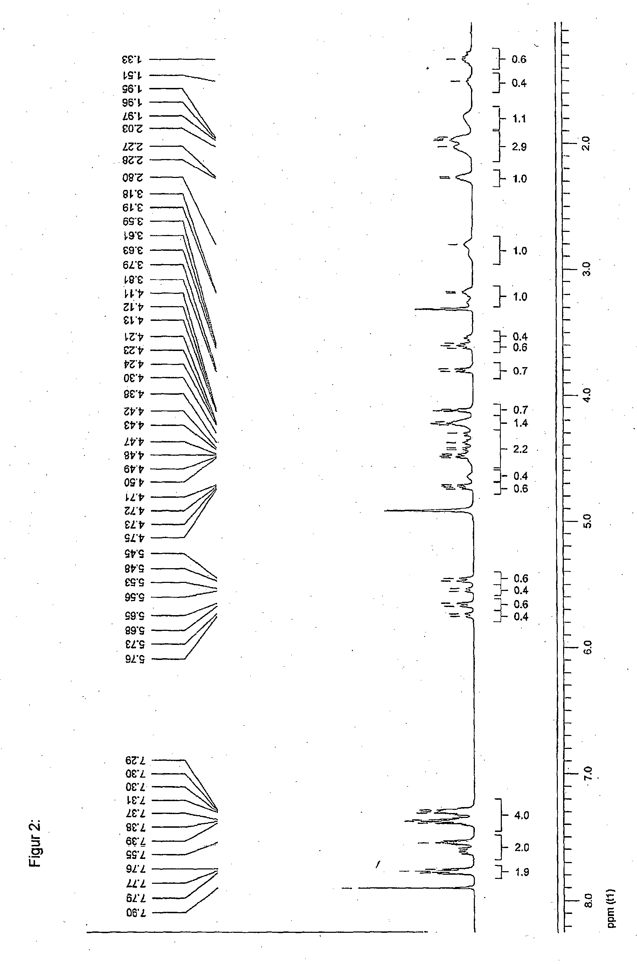

Zur Erforschung der Vorgänge auf molekularer Ebene und der resultierenden biologischen Funktions-weise wurde die dreidimensionale Domänenstruktur aufgeklärt, aber auch die Wechselwirkung mit ist verschiedenen Ligandenpeptiden untersucht. Dabei zeigte sich, dass die EVH1-Domänen ein Konsens-Kemmotiv FPx*ϕ*P erkennen, bei dem Phenylalanin (F) und die beiden äußeren Prolinpositionen (P) essentiell für eine Bindungsbildung sind, die beiden inneren Positionen aber durchaus Variationen zulassen (x = beliebige Aminosäure, *ϕ* = hydrophobe Aminosäure). Dies ist in Figur 2 zu sehen, in der die Klasse-I-EVH1-Domäne des menschlichen VASP-Proteins mit einem kurzen Ausschnitt aus dem ActA-Peptid (₃₃₂SFEFPPPPTEDEL₃₄₄) zusammen untersucht wurde. Das zentrale FPPPP-Motiv und ein affinitätssteigerndes EL-Epitop sind umrandet und zeigen eine deutliche Beeinflussung der Bindungs-stärke bei Substitution der einzelnen Positionen durch andere natürliche Aminosäuren (dunkel = gute Bindung, hell = keine Bindung).

Dass die Positionen P₀ und P₁ durch andere Aminosäuren ersetzt werden können, wobei besonders P₀ völlig unspezifisch ist, lässt sich durch die Betrachtung des Ligandenpeptids im Bindungsmodus erklä-ren: während P₋₁ und P₂ in einer hydrophoben Bindungstasche der Domäne aufgenommen werden, befinden sich die mittleren beiden Proline in einer schirmartigen Position über der Domäne und haben fast keinen Kontakt mit der Domänenoberfläche. Die Carbonylgruppe von P₀ bildet allerdings eine essentielle Wasserstoffbrücke zum NH des Tryptophan-Restes W23 der EVH1-Domäne.

Weiterhin konnte anhand einer Reihe verschiedener Test-Ligändenpeptide ein Überblick über die Bindungsaffinitäten gewonnen werden. Die höchste beobachtete Bindungsaffinität (K_{D} = 45 µM) liefert der Ligand ₃₃₂SFEFPPPPTEDEL₃₄₄, (dritter der vier prolinreichen Repeats des ActA-Proteins). Bei einer Verkürzung des Liganden- auf das Kernmotiv FPPPPT hingegen ließ sich keine messbare Affinität zur VASP-EVH1-Domäne mehr feststellen, eine Bindung zur Mena-EVH1-Domäne dagegen war sehr schwach, aber noch nachweisbar (417 µM).

In einer bevorzugten Ausführungsform der Erfindung werden die Verbindungen als Poly-Prolin-Mimetika eingesetzt. Vorteilhafterweise findet man prolinreiche Aminosäurensequenzen insbesondere in Peptiden, die in Signaltransduktionsvorgängen, insbesonder intrazellulären Signaltransduktionsvorgängen beteiligt sind. Im Sinne der Erfindung kann der Begriff der Mimetika auch als Analoga verstanden werden. Bevorzugt ist die Verwendung der erfindungsgemäßen Verbindungen zur Behandlung von Krankheiten, die mit einer Modifikation von intrazellulären Signaltransduktionsvorgängen assoziiert sind, die durch Poly-Prolin-Helix-Strukturen vermittelt werden, ausgewählt aus der Gruppe umfassend bakterielle und/oder virale Infektionskrankheiten, neurodegenerative Erkrankungen und/oder Tumorerkrankungen.

Weitere Krankheiten im Sinne der Erfindung, die mit den erfindungsgemäßen Mitteln behandelt werden können, sind ausgewählt aus der Gruppe umfassend Affenpocken, AIDS, Anthrax (Bacillus anthracis, Milzbrand), Aviäre Influenza (Geflügelpest, Vogelgrippe), Borreliose, Borrelia recurrentis (Läuserückfallfieber), Botulismus (Clostridium botulinum), Brucellose, Campylobacter-Infektionen, Chlamydiose, Cholera (Vibrio cholerae), Creutzfeldt-Jakob-Krankheit, Coxiella burnetii (Q-Fieber), Cryptosporidium parvuum (Kryptosporidiose), Dengue-Fieber, Diphtherie, Ebolavirus-Infektionen, Echinokokkose (Fuchsbandwurm, Hundebandwurm), EHEC-Infektionen (STEC-Infektionen, VTEC-Infektionen), Enteroviren, Fleckfieber (Rickettsia prowazeckii), Francisella tularensis (Tularämie), Frühsommer-Meningoenzephalitis (FSME), Gelbfieber, Giardiasis, Gonorrhoe, Grippe (Influenza), Haemophilis influenzae, Hantavirus, Helicobacter pylori, Hepatitis A, Hepatitis B, Hepatitis C, Hepatitis D, Hepatitis E, Herpes, HUS (hämolytisch urämisches Syndrom), Keratoconjunctivitis epidemica, Keuchhusten (Pertussis), Kinderlähmung (Poliomyelitis), Kopflausbefall, Krätzemilbenbefall, Krim-Kongo-Feber, Lassa-Fieber, lebensmittelbedingte Krankheiten, Legionellose, Leishmaniose, Lepra, Leptospirose, Listeriose, Lyme-Borreliose, Lymphogranuloma venereum, Malaria (Plasmodien-Infektionen), Marburgvirus-Infektionen, Masern, Meliodose, Meningokokken-Erkrankungen, MRSA (Staphylokokken), Mumps, Mykosen (Pilzinfektionen), neu und vermehrt auftretende Infektionskrankheiten, Noroviren, Ornithose (Papageienkrankheit), Papillomaviren, Paratyphus, Pest (Yersinia pestis), Pneumokokken-Infektionen (Streptococcus pneumoniae), Pocken, reiseassoziierte Infektionskrankheiten, Rinderbandwurm-Infektion beim Menschen, Rotaviren, Röteln, RSV-Infektionen, Salmonellose, Scharlach, Schweres Akutes Respiratorisches Syndrom (SARS), sexuell übertragbare Infektionen, Shigellose, Syphilis, Tetanus, Tollwut, Toxoplasmose, Thrichinellose, Tuberkulose, Typhus, Varizellen (Windpocken), Variante Creutzfeldt-Jakob-Krankheit, virale hämorrhagische Fieber, West-Nil-Fieber, Yersiniose und/oder Zecken-übertragene Erkrankungen.

Die Erfindung betrifft die Verwendung der erfindungsgemäßen Mittel als Arzneimittel. Im Sinne der Erfindung können die erfindungsgemäßen Mittel daher im gesamten Bereich der Medizin, insbesondere als pharmazeutische Mittel angewendet werden.

Bevorzugt handelt es sich bei den bakteriellen Erkrankungen um Erkrankungen, die durch folgende Bakterien assoziiert, insbesondere vermittelt werden: Legionellen, Streptokokken, Staphylokokken, Klebsiellen, Haemophilis influenzae, Rickettsien (Fleckfieber), Mykobakterien, Mykoplasmen, U-reaplasmen, Neisserien (Meningitis, Waterhoüse-Friedrichsen-Syndrom, Gonorrhoe), Pseudomonaden, Bordetellen (Pertussis), Corynobakterien (Diphtherie), Chlamydien, Campylobacter (Durchfall), Escherichia coli, Proteus, Salmonellen, Shigellen, Yersinien, Vibrionen, Enterokokken, Clostridien, Borrelien, Treponema pallidum, Brucellen, Francisellen und/oder Leptospira, insbesondere Listerien.

Besonders bevorzugte Erkrankungen sind die, die durch Listerien ausgewählt aus der Gruppe umfassend L. monocytogenes Sv1/2a, L. monocytogenes Sv4b F2365, L. monocytogenes Sv4b H7858,178 contigs, L. monocytogenes Sv1/2a F6854,133 contigs, L *monocytogenes* Sv4b, L *monocytogenes* Sv4a, L. innocua Sv6a, L. welshimeri Sv6b, L. seeligeri Sv1/2b und/oder L. ivanovii Sv5 ausgelöst werden oder auf die genannten bevorzugten Listerien im wesentlichen zurückgehen.

Die bevorzugten neurodegenerativen Erkrankungen sind ausgewählt aus der Gruppe umfassend Morbus Alzheimer, Morbus Parkinson, Morbus Huntington und/oder amyotrophische Lateralsklerose (ALS). Neurodegenerative Erkrankungen im Sinne der Erfindung sind außerdem Alexander-Krankheit, Alpers-Huttenlocher-Syndrom, Chorea Huntington, Creutzfeldt-Jakob-Krankheit, extrapyramidales Syndrom, Friedreich-Ataxie, kortikobasale Degeneration, Morbus Krabbe, Leukodystrophie, Lewy-Körperchen-Demenz, Maskengesicht, Morbus Pick, Multiple Sklerose, Multisystematrophie, neurodegenerative Eisenablagerungen im Gehim, progressive supranukleäre Blickparese, Shy-Drager-Syndrom, spinozerebelläre Ataxie, Synuleinopathie und/oder tropische spastische Paraparese.

Die bevorzugten Tumorerkrankungen sind ausgewählt aus der Gruppe umfassend Tumorerkrankungen des Hals-Nasen-Ohren-Bereichs, der Lunge, des Mediastinums, des Gastrointestinaltraktes, des Urogenital-Systems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppression-bezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs; das Melanom; das Hepatom; das Neuroblastom; das Papillom; das Apudo;, das Choristom; das Branchiom; das maligne Karzinoid-Syndrom; die Karzinoid-Herzerkrankung; das Karzinom (zum Beispiel Walker-Karcinom, Basalzellen-Karcinom, basosquamöses Karzinom, Brown-Pearce-Karzinom, duktales Karzinom, Ehrlich-Tumor, in-situ-Karzinom, Krebs-2-Karzinom, Merkel-Zetien-Karzinom, Schleimkrebs, nicht-kleinzelliges Bronchialkarzinom, Haferzellen-Karzinom, papilläres Karzinom, szirrhöses Karzinom, bronchiolo-alveoläres Karzinom, Bronchial-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukärmie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor, Reticuloendotheliose, Chondroblastom; Chondrom, Chondrosarkom; Fibrom; Fibrosarkom; Riesenzell-Tumore; Histiocy tom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom; Chordom; Craniopharyngiom; Dysgerminom; Hamartom; Mesenchymom; Mesonephrom; Myosarkom; Ameloblastom; Cementom; Odontom; Teratom; Thymom; Chorio-blastom; Adenokarzinom; Adenom; Cholangiom; Cholesteatom; Cylindrom; Cystadeno-cardnom; Cystadenom; Granulosa-zelltumor, Gynadroblastom; Hidradenom; Inselzelltumor; Leydig-Zelltumor, Papillom; Sertoli-Zell-Tumor; Thekazell-tumor; Leiomyom; Leiomyosarkom; Myoblastom; Myom; Myosarkom; Rhabdomyom; Rhabdomyosarkom; Ependynom; Ganglioneurom; Gliom; Medulloblastom; Meningiom; Neurilemmom; Neuroblastom; Neuroepitheliom; Neurofibrom; Neurom; Paragangliom; nicht-chromaffines Paragangliom; Angiokeratom; angiolymphoide Hyperplasie mit Eosinophilie; sderosierendes Angiom; Angiomatose; Glomangiom; Hemangioendotheliom; Hemangiom; Hemangiopericytom, Hemangiosarkom; Lymphangiom, Lymphangio-myom, Lymphangiosarkom; Pinealom; Cystosarkom phyllodes; Hemangiosarkom; Lymphangiosarkom; Myxosarkom; Ovarialkarzinom; Sarkom (zum Beispiel Ewing-Sarkom, experi-mentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Himanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des ürogenitaltrakts); Neurofibromatose und zervikale Plattenepitheldysplasie.

In einer weiter bevorzugten Ausführungsform ist die Krebserkrankung oder der Tumor ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Hamleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, EwingSarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoptastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehimmetastasen, Lungen-metastasen, Lebermetastasen, Knochenmetastasen, pleurale und perikardiale Metastasen und maligne Aszites.

In einer weiter bevorzugten Ausführungsform ist die Krebserkrankung oder der Tumor ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarm-krebs, der Ovariatkarzinome, der Zervikalkarzinome, Lungen-krebs, Prostatakrebs, Nierenzellkarzinome und/oder Lebermetastasen.

Virale Erkrankungen im Sinne der Erfindung sind ausgewählt aus der Gruppe umfassend Influenza, Schnupfen, Husten, Masern, Mumps, Röteln, Ringelröteln, Drei-Tage-Fieber, Windpocken, Pfeiffersches Drüsenfieber, SARS, Zytomegalie, Durchfall, Hepatitis, Kinderlähmung, Herpes labialis, War zen, Tollwut, Lassa-Fieber, Ebola, Marburg-Fieber, Hanta-Virus-Fieber, FSME, RSSE, Louping-ill-Enzephalitis, Powassan-Enzephalitis, Kyasanur-forest-Fieber, Omsk-hämorrhagisches-Fieber, Colora-do-tick-Fieber, Gelbfieber, Dengue-Fieber, Japanische Enzephalitis, West-Nil-Fieber, Chikungunya-Fieber, O'nyong-nyong-Fieber, Rift-Tal-Fieber, Sandmücken-Fieber, Ross-River-Fieber, Sindbis-Fieber, Mayaro-Fieber, Murray-Valley-Enzephalitis, St. Louis-Enzephalitis, Rocio-Enzephalitis, California-Encephalitis, Bunyamwera-Fieber, Oropouche-Fieber, AIDS, Herpes genitalis und/oder Herpes simplex, besonders bevorzugt sind die viralen Hepatitiserkrankungen ausgewählt aus der Gruppe umfassend Hepatitis A, Hepatitis B, Hepatitis C, Hepatitis D, Hepatitis E, Hepatitis F, Hepatitis G und/oder autoimmune Hepatitis und/oder asymptomatische oder symptomatische HIV-Infektionen.

Die HIV-Infektionen können zu der Krankheit AIDS führen Im Sinne der Erfindung ist AIDS charakterisiert bzw klassifiziert durch ein klinisches Bild, ausgewählt aus der Gruppe umfassend
(a) asymptomatische oder symptomatische HIV-Infektionen,
(b) bazilläre Angiomatosen, Entzündungen des kleinen Beckens, besonders bei Komplikationen eines Tuben- oder Ovarialabszesses, ausgedehnter oder rezidivierender Herpes zoster, thrombozytopene Purpura, lang anhaltendes Fieber oder Diarrhoen, die langer als einen Monat anhalten, Listeriose, orale Harfleukoplakie, oropharyngeale Candidiosen, chronische oder schwer zu therapierende Vaginale Candidosen, zervikale Dysplasien, Carcinoma in situ, penphere Neuropathie und/oder
(c) Candidosen der Atemwege oder der Speiseröhre, Cytomegalievirus-Infektionen, CMV-Retinitis, HIV-bedingte Enzephalopathie, Herpes simplex mit chronischen Ulzera (>1 Monat) oder durch Herpes simplex bedingte Bronchitis, Pneumonie oder Ösophagitis, chronische Histoplasmose, itestinale Isosporiasis, Kaposi-Sarkom, dissemnierte oder extrapulmonale Kokzidiomykose, extrapulmonale Kryptokokkose, chronisch intestinale Kryptospondiose, immunoblasfisches, primär zerebrales oder Burkitt Lymphom, extrapulmonale Mykobakterien, Pneumocystis-Pneumonie, rezidivierende bakterielle Pneumonie, progressive multifokale Leukenzephalopathie, rezidivierende Salmonellen-Septikämie, Tuberkulose, zerebrale Toxoplasmose, Wasting-Symdrom und/oder invasives Zervixkarzinom.

Behandlung im Sinne der Erfindung heißt sowohl Prophylaxe, Therapie, Verlaufskontrolle und/oder Nachbehandlung von Erkrankungen

Das erfindungsgemäße Mittel kann isoliert oder in Kombination mit anderen Mitteln insbesondere gegen behüllte, aber auch gegen unbehüllte Viren eingesetzt werden. Bei den behüllten Viren handelt es sich bevorzugt um

### Doppelsträngige DNA-Viren = dsDNA

- Familie Poxviridae
   o Unterfamilie *Chordopoxvirinae*
      ■ Gattung *Orthopoxvirus*
         ■ Orthopox-Variola-Virus =Pockenvirus - Pocken
         ■ Orthopox-Alastrim-Virus - Weiße Pocken
      ■ Gattung *Parapoxvirus*
         ■ Parapox-Ovis-Virus =Orf-Virus - Orf = Schafpocken, bei Tieren, auf den Mensch übertragbar
      ■ Gattung *Molluscipoxvirus*
         ■ Molluscum-Contagiosum-Virus - Dellwarze (*Molluscum contagio*sum)
- Familie Herpesviridae
   ○ Unterfamilie *Alphaherpesvirinae*
      ■ Gattung *Simplexvirus*
         ■ Herpes-simplex-Virus 1 (HSV 1) = Humanes-Herpes-Virus 1 (HHV 1) - Herpes simplex, Herpes labialis, Stomatitis aphtosa
         ■ Herpes-simplex-Virus 2 (HSV 2) = Humanes-Herpes-Virus 2 (HHV 2) - Herpes simplex, Herpes genitalis
         ■ Herpes-B-Virus = (Herpesvirus simiae)
      ■ Gattung Varicellovirus
         ■ Varizella-Zoster-Virus (VZV) = Humanes-Herpes-Virus 3 (HHV 3)-Windpocken = Varizellen (Herpes zoster), *Gürtelrose*
         ■ Pseudowut-Virus - Juckseuche = Tollkrätze, bei Tieren, auf den Menschen übertragbar!
   ○ Unterfamilie Betaherpesvirinae
      ■ Gattung *Cytomegalovirus*
         ■ Humanes-Zytomegalie-Virus = Humanes-Cytomegalie-Virus (HCMV) = Humanes-Herpes-Virus 5 (HHV 5) - Zytomegalie
      ■ Gattung *Reseolovirus*
         ■ Humanes-Herpes-Virus 6 (HHV 6) - Drei-Tage-Fieber
         ■ Humanes-Herpes-Virus 7 (HHV 7) - Drei-Tage-Fieber
   o Unterfamilie *Gammaherpesvirinae*
      ■ Gattung *Lymphocryptovirus*
         ■ Epstein-Barr-Virus (EBV) = Humanes-Herpes-Virus 4 (HHV 4) - Pfeiffersches Drüsenfieber, Burkitt Lynaphom
      ■ Gattung *Rhadinovirus*
         ■ Humanes-Herpes-Virus 8 (HHV 8) - Kaposi-Sarkom
- Familie *Hepadnaviridae*
   o Gattung *Orthohepadnavirus*
      ■ Hepatitis-B-Virus (HBV) - Hepatitis B

### Einzel(+)-Strang-RNA-Viren = ss(+)RNA

- Familie Togaviridae
   ○ Gattung *Alphaviren* - Erreger von Arbovirosen
      ■ Chikungunya-Virus (CHIKV) - Chikungunya-Fieber
      ■ O'nyong'nyong-Virus (ONNV) - O'nyong-nyong-Fieber
   o Gattung *Rubiviren*
      ■ Rubivirus = Rötelvirus = Rubellavirus - Röteln
- Familie Flaviviridae
   o Gattung *Hepacivirus*
      ■ Hepatitis-C-Virus (HCV) - Hepatitis C
      ■ GB-Virus-C (ohne Krankheitswert)
   o Gattung *Flavivirus*
      ■ West-Nil-Virus - West-Nil-Fieber
      ■ Dengue-Virus - Dengue-Fieber
      ■ Gelbfieber-Virus - Gelbfieber
      ■ Louping-ill-Virus - Louping-ill-Enzephalitis
      ■ St.-Louis-Enzephalitis-Virus - St.-Louis-Enzephalitis
      ■ Japan-B-Enzephalitis-Vrus - Japanische Enzephalitis
      ■ Powassan-Virus - Powassan-Enzephalitis
      ■ RSSE-Virus - RSSE = Russian-Spring-Summer-Enzephalitis
      ■ FSME-Virus - FSME = Frühsommer-Meningoenzephalitis
- Familie Coronaviridae - Magen-Darm-Entzündungen
   o Gattung *Coronavirus*
      ■ SARS-assoziiertes-Corona-Vrus (SARS-CoV)- SARS = atypische Lungenentzundung = (Pneumonie)
      ■ Humanes-Corona-Virus 229E (HCoV 229E) - Erkältung
      ■ Humanes-Corona-Virus OC43 (HCoV OC43) - Erkältung
   o Gattung *Torovirus* - Gaströenteritis
- Familie Retroviridae = Enzel(+)-Strang-RNA-Viren mit dsDNA-Zwischenstufe:
   o Unterfamilie *Orthoretrovirinae*
      ■ Gattung *Deltaretrovirus*
         ■ Humanes-T-Zell-lymphotropes-Virus Typ I (HTLV-I) - Leukämie
         ■ Humanes_T-Zell-lymphotrope_Virus Typ II (HTLV-III) - Leukämie
      ■ Gattung *Lentivirus*
         ■ Humanes-Immundefizienz-Virus Typ 1 (HIV-1) - AIDS
         ■ Humanes-Immundefizienz-Virus Typ 2 (HIV 2) - AIDS

### Einzel(-)-Strang-RNA-Viren = ss(-)RNA

- Familie *Arenaviridae*
   o Gattung *Arenavirus*
      ■ Lassa-Virus - Lassa-Fieber
      ■ lymphozytäre-Chorio-Meningitis-Virus (LCMU) - Choriomeningitis
      ■ Tacaribe-Virus
      ■ Junin-Virus - Junin-Fieber (argentinisches hämorrhagisches Fieber)
      ■ Machupo-Virus - Machupo-Fieber (bolivianisches hämorrhagisches Fieber)
- Familie Bornaviridae
   o Gattung *Bornavirus*
      ■ Virus der Borna schen Krankheit - beim Pferd, vielleicht auch auf den Menschen übertragbar - Affektive Störungen
- Familie Bunyaviridae - Erreger von Arbovirosen
   o Gattung *Orthobunyavirus*
      ■ Bunyamwera-Virus (Serogruppe)
      ■ California-Encephalitis-Virus (Serogruppe) - Encephalitis
   o Gattung *Phlebovirus*
      ■ Rift-Valley-Fieber-Virus - 3 Subtypen, Rift-Tal-Fieber
      ■ Sandmückenfieber-Virus *- Sandfly fever* = Sandmückenfieber
         ■ Subtyp Toscana-Virus - Pappataci-Fieber
   o Gattung *Nairovirus*
      ■ Krim-Kongo-Fieber-Virus (Serogruppe):
         ■ Subtyp Krim-Kongo-hämorrhagisches-Fieber-Virus - Krim-Kongo-Fieber
         ■ Subtyp Hazara-Virus
         ■ Subtyp Khasan-Virus
   o Gattung *Hantavirus*
      ■ Hantaan-Virus (4 Subtypen) hämorrhagisches Fieber, Nephritis
      ■ Seoul-Virus (Serogruppe) hämorrhagisches Fieber
      ■ Prospect-Hill-Virus (2 Subtypen) hämorrhagisches Fieber
      ■ Puumala-Virus (Serogruppe)- hämorrhagisches Fieber, Pneumonie, Nephritis
      ■ Dobrava-Belgrad-Virus-hämorrhagisches Fieber
      ■ Tula-Virus - hämorrhagisches Fieber
      ■ Sin-Nombre-Virus (Serogruppe) - hämorrhagisches Fieber mit schwerem Lungenödem
- Familie Filoviridae
   ○ Gattung Marburg-Virus
      ■ Lake-Victoria-Marburgvirus (Serogruppe)-Marburg-Fieber (hämorrhagisches Fieber)
   ○ Gattung Ebolavirus
      ■ Zaire-Ebolavirus (Serogruppe) - Ebola (hämorrhagisches Fieber)
      ■ Sudan-Ebolavirus - Ebola (hämorrhagisches Fieber)
      ■ Cöte d'Ivoire-Ebolavirus - Ebola (hämorrhagisches Fieber)
- Familie Orthomyxoviridae
   ○ Gattung *Influenzavirus A* - Influenza (Grippe)
      ■ Influenzavirus A-Variante (H1N1) - Influenza (Grippe)
      ■ Influenzavirus A-Variante (H3N2) - Influenza (Grippe)
      ■ (aviäres) Influenzavirus-A-Variante (H5N1), hoch pathogenes aviäres Influenzavirus (HPAIV) - Vogelgrippe, bei Tieren, auch auf den Mensch übertragbar, aber nicht von Mensch zu Mensch.
   o Gattung *Influenzavirus B -* Influenza (Grippe)
      ■ Influenzavirus B/Victoria-Linie - Influenza (Grippe)
      ■ Influenzavirus B/Yamagata-Linie - Influenza (Grippe)
   o Gattung *Influenzaviren C* - Influenza (Grippe)
- Familie Paramyxoviridae
   o Unterfamilie *Paramyxovirinae*
      ■ Gattung *Avulavirus*
         ■ Parainfluenzavirus (1, 3) - Parainfluenza
      ■ Gattung *Morbillivirus*
         ■ Masernvirus - Masern
      ■ Gattung *Rubulaviren*
         ■ Parainfluenzavirus (2, 4) - Parainfluenza
         ■ Mumpsvirus - Mumps
   o Unterfamilie *Pneumovirinae*
      ■ Gattung *Pneumovirus*
         ■ Respiratory-Syncytical-Virus (RSV) - Atemwegsinfektion
      ■ Gattung *Metapneumovirus*
         ■ Humanes Metapneumovirus (HMPV) - Atemwegsinfektion
- Familie Rhabdoviridae
   o Gattung *Vesiculovirus*
      ■ Vesicular-Stomatitis-Indiana-Virus (VSV) - Stomatitis vesicularis (Mundschleimhautentzündung mit Bläschenbildung) bei Tieren, auch auf den Mensch übertragbar
   ○ Gattung *Lyssavirus*
      ■ Rabiesvirus (RABV) (ehemals Genotyp 1) = Tollwutvirus - Tollwut, bei Tieren, auch auf den Mensch übertragbar
      ■ Mokola-Virus (MOKV) (ehemals Genotyp 3) - Tollwut, bei Tieren, auch auf den Mensch übertragbar
      ■ Duvenhage-Vrus (DUVV) (ehemals Genotyp 4) - Tollwut, bei Tieren, auch auf den Mensch übertragbar
      ■ Europäisches-Fledermaus-Lyssa-Virus 1 + 2 (EBLV 1, 2) (ehemals Genotypen 5 und 6) - Tollwut, bei Tieren, auch auf den Mensch übertragbar
      ■ Australisches-Fledermaus-Lyssa-Virus (ABLV) (ehemals Genotyp 7)-Tollwut, bei Tieren, auch auf den Mensch übertragbar

### Bei den unbehüllten Viren handelt es sich insbesondere um:

### Doppelsträngige DNA-Viren = dsDNA

- Adenoviridae
   ○ Humane Adenoviren A-F (51 Subtypen) - Schnupfen, Erkältungen, Durchfall
- Papovaviridae
   ○ Papovaviren
      ■ Humane-Papilloma-Viren
         ■ diverse Humane-Papilloma-Viren (HPV)-Warzen
         ■ Kondyloma-Virus 6 (HPV 6) - Feigwarzen
         ■ Kondyloma-Virus 11 (HPV 11) - Feigwarzen
         ■ Humanes-Papilloma-Virus, low-risk Typen (HPV 40,42,43,44,54,61,70,72,81 und CP6108; high-risk Typen (HPV 16, 18, 31 und 33, aber auch 35, 39, 45, 51, 52, 56, 58, 59, 68, 73 und 82)- Zervixkarzinom = Gebärmutterhalstumor/-Krebs
      ■ Polyomaviren

### Einzelsträngige DNA-Viren = ssDNA

- Parvoviridae
   ○ Parvovirinae
      ■ Dependovirus
         ■ Adenoassoziiertes-Virus 2 (AAV 2)
         ■ Adenoassoziiertes-Virus 3 (AAV 3)
         ■ Adenoassoziiertes-Virus 5 (AAV 5)
      ■ Erythrovirus
         ■ Parvovirus B19 - Ringelröteln

### Doppelsträngige RNA-Viren = dsRNA

- Reoviren
   ○ Rotaviren - Gastroenteritis = Durchfall
   ○ Orbiviren
      ■ Colorado- Tick-Virus - Colorado- Tick-Fieber
      ■ Epizootic hemorrhagic disease virus (EHDV) - Enzootische Hämorrhagie der Hirsche

### Einzel(+)-Strang-RNA-Viren = ss(+)RNA

- Picomaviridae
   ○ Rhinoviren
      ■ Humanes-Rhino-Virus (HRV), 1A, 1B-100 - Schnupfen, Erkältungen
   ○ Aphthoviren
      ■ Maul-und-Klauenseuche-Virus - Maul- und Klauenseuche beim Tier, auch in milder Form auf den Menschen übertragbar
   o Enteroviren
      ■ Poliovirus (1-3) - Kinderlähmung
      ■ Coxsackie-Virus-A 1-22,24 (CVA 1-22,24) Erkältungen, virale Meningitis, Myokarditis
      ■ Coxsackie-Virus-B 1-6 (CVB 1-6) - Erkältungen, virale Meningitis, Myokarditis
      ■ Echoviren - Erkältungen, Gastroenteritis = Durchfall, Meningoenzephalitis
      ■ Humane Enteroviren - Erkältungen, Gastroenteritis = Durchfall
      ■ SVD-Viren (Vesikuläre Schweinekrankheit)
   ○ Cardioviren
      ■ Enzephalo-Myocarditis-Virus (EMCV) - Enzephalomyocarditis
      ■ Mengo-Virus - Enzephalomyocarciitis
      ■ Theiler-Murines-Enzephalomyelitis-Virus (TMEV) - Enzephalomyelitis
      ■ Vilyuisk-Humanes-Enzephalomyelitis-Virus (VHEV) - Enzephalomyelitis
   ○ Hepatoviren
      ■ Hepatitis-A-Virus (HAV) - Hepatitis A
- Hepeviridae
   ○ Hepevirus
      ■ Hepatitis-E-Virus (HEV) Hepatitis E
- Caliciviridae
   o Caliciviren
      ■ SRSV = small rounded structured viruses
      ■ Norwalk-Virus - Gastroenteritis = Durchfall
      ■ Noroviren - Gastroenteritis = Durchfall
      ■ Sapoviren - Gastroenteritis = Durchfall
      ■ Vesiviren
      ■ Lagoviren
- Astroviridae
   o Astroviren
      ■ Humanes-Astro-Virus - Gastroenteritis = Durchfall

Das erfindungsgemäße Mittel kann in einer bevorzugten Ausführungsform ein weiteres Mittel umfassen ausgewählt aus der Gruppe umfassend Abacavir, Acyclovir, Adefovir, Amantadine, Amprenavir, Atazanavir, Codofovir, Darunavir, Delavirdine, Didanosine, Docosanol, Emtricitabine, Efavirenz, Enfuvirtide, Entecavir, Famciclovir, Foscamet, Fomivirsen, Fosamprenavir, Ganciclovir, Gardasil, Idoxuridine, Imiquimod, Indinavir, Interferon, Lamivudine, Lopinavir, Nevirapine, Nelfinavir, Oseltamivir, Penciclovir, Peramivir, Ribavirin, Rimantadine, Ritonavir, Saquinavir, Stavudine, Tenofovir, Tipranavir, Trifluridine, Tromantadine, Valaciclovir, Valganciclovir, Vidarabine, Viramidine, Zalcitabine, Zanamivir und/oder Zidovudine.

Die Erfindung betrifft auch einen Satz (einen Kit) bestehend aus getrennten Packungen
a) einer wirksamen Menge der erfindungsgemäßen Zusammensetzung und/oder ihren pharmazeutisch verwendbaren Derivaten, Solvaten und/oder Stereoisomeren, sowie deren Mischungen mit weiteren bzw. zusätzlichen Mitteln in allen Verhältnissen und
b) einer wirksamen Menge eines weiteren Arzneimittels. Der Satz beinhaltet geeignete Behältnisse wie Schachteln, einzelne Flaschen, Beutel oder Ampullen. Der Satz kann z.B. einzelne Ampullen umfassen, die jeweils eine wirksame Menge der erfindungsgemäßen Zusammensetzung und/oder ihren pharmazeutisch verwendbaren Derivaten, Solvaten und/oder Stereoisomeren, einschließlich deren Mischungen in allen Verhältnissen, sowie eine wirksame Menge eines weiteren Arzneimittelwirkstoffs in gelöster oder lyophilisierter Form enthalten. Weiterhin kann der Satz eine Information beispielsweise in Form eines Beipackzettels zum Kombinieren der Inhalte des Kits beinhalten. Diese Informationen kann aber auch die Darstellung eines Therapieschemas oder ähnliches umfassen.

In der obigen Beschreibung der pharmazeutischen Zusammensetzungen, die eine bevorzugte Verbindung enthalten, wurden die gleichwertigen Ausdrücke "Verabreichung", "Verabreichung von", "Verabreichen" und "ein (e)...verabreichen" in Bezug auf diese pharmazeutischen Zusammensetzungen verwendet. Diese Ausdrücke sollen im vorliegenden Zusammenhang bedeuten, dass einen behandlungsbedürftigen Patienten eine erfindungsgemäße pharmazeutische Zusammensetzung auf einem beliebigen der hier beschriebenen Verabreichungswege zur Verfügung gestellt wird, wobei es sich bei dem Wirkstoff um eine bevorzugte Verbindung oder ein Prodrug, ein Derivat oder einen Metaboliten hiervon handelt, das bzw. der sich zur Behandlung einer durch Virusinfektion vermittelten oder hiermit assoziierten Erkrankung, krankhaften Störung oder eines derartigen Leiden bei diesem Patienten eignet. Die vorliegende Erfindung erstreckt sich daher auf eine beliebige andere Verbindung, die bei Verabreichung an einen Patienten fähig ist, eine bevorzugte Verbindung direkt oder indirekt zur Verfügung zu stellen. Solche Verbindungen sind als Prodrugs bekannt, und es existieren viele etablierte Vorgehensweisen zur Herstellung solcher Prodrug-Formen der bevorzugten Verbindungen.

Bei der Behandlung der genannten Krankheiten ist es besonders bevorzugt, das pharmazeutische Mittel, welches die erfindungsgemäßen Verbindungen umfasst, als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zuzubereiten und/oder anzuwenden.

Bevorzugt liegt das pharmazeutische Mittel, welches die erfindungsgemäßen Verbindungen umfasst, in einer Konzentration von 0,1 bis 99,5, bevorzugt von 0,5 bis 95,0, besonders bevorzugt von 20,0 bis 80,0 Gew.-% in einer Zubereitung vor.

Bevorzugt wird diese Zubereitung oral, subkutan, intravenös, intramuskulär, intraperitoneal und/oder topisch gesetzt.

Bevorzugt wird das pharmazeutische Mittel, das die erfindungsgemäßen Verbindungen enthält, in Gesamtmengen von 0,05 bis 500 mg pro kg, bevorzugt von 5 bis 100 mg pro kg Körpergewicht, je 24 Stunden eingesetzt.

Bevorzugt erfolgt das In-Kontakt-Bringen oral, über Injektion, topisch, vaginal, rektal und/oder nasal.

Es besteht ein ständiger Bedarf an Verbindungen, die die Bindung von Strukturen, die die genannten Domänen (im Sinne der Erfindung sind hierbei insbesondere folgende Domänen erfasst: Src-Homologie-3-Domänen, WW-Domänen, Ena-VASP-Homologie-1-Domänen, GYF-Domänen, UEV-Domänen und/oder Profilin) enthalten und ihre Liganden beeinflussen. Es besteht daher das Bedürfnis, die neuen Verbindungen auch für das Testen in in vitro-Tests bereitzustellen, die beispielsweise für pharmakologische Durchmusterungstests auf bestimmte Leitverbindungen verwendet werden können.

Die erfindungsgemäßen Mittel werden in einer weiteren bevorzugten Ausführungsform auch als diagnostische Mittel bereitgestellt, die die erfindungsgemäßen Mittel umfassen und eine nachweisbare Markierung, die mit dem Mittel direkt, indirekt oder mittels Komplexbildung konjugiert ist. Die diagnostischen Mittel können verwendet werden, um die Anwesenheit von Src-Homologie-3-Domänen, WW-Domänen, Ena-VASP-Homologie-1-Domänen, GYF-Domänen, UEV-Domänen und/oder Profilin eines generischen oder spezifischen Typs in Zellen, Geweben oder Organen in vitro oder in vivo nachzuweisen. Für die in vivo-Anwendungen wird das diagnostische Mittel für die Verabreichung entweder enteral, parenteral oder mittels eines anderen Weges, der durch die Bedürfnisse der speziellen Anwendung vorgegeben ist, vorzugsweise mit einem pharmazeutisch verträglichen Träger gemischt

In einer speziellen Ausführungsform wird zum Beispiel ein Test auf Basis eines Fusionsproteins verwendet, der ein erfindungsgemäßes Mittel, z. B. eingebracht in ein Peptid umfasst, und ein Substrat für z. B. dereguliertes oder aktiviertes Src beinhaltet oder ein Protein mit einer anderen der genannten Domänen (WW-, Ena-VASP-Homologie-1-, GYF-, UEV-Domänen und/oder Profilin). Zum Beispiel kann eine Probe aus einem Patienten entnommen worden sein, die man daraufhin testen möchte, ob sie mit einem bestimmten Virus infiziert wird, wobei diese Probe mit einer wirksamen Menge des Fusionsproteins inkubiert wird. Durch anschließende Analyse bezüglich des Ausmaßes der Umwandlung des Substrats kann man potentiell die Infektion des Individuums mit dem Virus nachweisen. Die Anwesenheit eines Virus, der eine Expression von z. B. deregulierten oder aktivierten Src verursacht, kann somit durch unübliche hohe Niveaus an z. B. Src angezeigt werden, die durch hohe Mengen an umgewandelten Substrat detektiert werden.

Die Erfindung betrifft auch einen Kit, der mindestens eine der erfindungsgemäßen Verbindungen und/oder eines der erfindungsgemäßen pharmazeutischen Mittel, gegebenenfalls mit einer Information zum Kombinieren der Inhalte des Kits, umfasst - z. B. einen Beipackzettel oder eine Intemet-Adresse, die auf Homepages mit weiteren Informationen verweist etc. Die Information zur Handhabung des Kits kann beispielsweise ein Therapie-Schema für die o. g. Krankheiten, insbesondere für die bevorzugten Krankheiten umfassen. Die Information kann jedoch auch Angaben darüber umfassen, wie die erfindungsgemäßen Erzeugnisse innerhalb einer Diagnose der genannten Krankheiten einzusetzen sind. Der erfindungsgemäße Kit kann auch in der Grundlagenforschung verwendet werden.

Die Erfindung betrifft demgemäß auch die Verwendung des Kits zur Prophylaxe und/oder Therapie von neurodegenerativen Erkrankungen, bakteriellen Infektionserkrankungen oder Tumorerkrankungen.

Die Erfindung betrifft auch die Verwendung der erfindungsgemäßen Mittel zum Screening nach Peptidbindern. Dem Fachmann mit durchschnittlichem Können sind Screening-Methoden mit Peptid-Mimetika bekannt.

Die erfindungsgemäßen Mittel können auch als Bausteine in Peptide, insbesondere beispielsweise in immobilisierte Peptide eingebaut werden. Derartige immoblisierte Peptide sind mit Vorteil insbesondere in der Diagnostik einsetzbar. Die immobilisierten Peptide können beispielsweise auf Gold immobilisiert werden, beispielsweise indem die erfindungsgemäßen Mittel auf den Goldpartikeln immobilisiert werden. Die immobilisierten oder nicht immobilisierten Peptide oder andere Moleküle, die die erfindungsgemäßen Mittel als einen Baustein umfassen, können beispielsweise zum Screening von Substanzen verwendet werden, die in Protein-Protein-Wechselwirkungen bzw. Peptid-Peptid-Wechselwirkungen eingreifen. Eine bevorzugte Anwendung ist beispielsweise die Verwendung der Peptide, die die erfindungsgemäßen Mittel umfassen oder die Verwendung der erfindungsgemäßen Mittel in Kombination mit Kohlenhydraten oder Lipiden in Affinitätssäulen. Dem Fachmann sind aber auch weitere Immoblisierungsmöglichkeiten bekannt. Bei der Immobilisierung handelt es sich im Sinne der Erfindung um verschiedene Verfahren zum Fixieren der erfindungsgemäßen Mittel, die insbeondere durch biologische, chemische oder physikalische Einwirkung erfolgen kann. Beispielsweise ist es möglich, die erfindungsgemäßen Mittel trägergebunden zu fixieren oder aber durch Quervemetzungm, durch Einschluss oder durch das Einbringen in Mikrokapseln. Bei der Bindung an einen Träger wie z. B. in einer Mikrotiterplatte oder einer Chromatographiesäule erfolgt die Fixierung durch Adsorption, Ionenbindung oder kovalente Bindung. Beispielsweise können die erfindungsgemäßen Mittel durch Spacer oder Antikörper fixiert werden. Bei dem Einschluss in eine semipermeable Membran erfolgt die Fixierung in Form des Einschlusses in Gele, Mikrokapseln oder Fasern.

Mittels der genannten Affinitätssäulen können Interaktionspartner detektiert werden, indem Proteine ermittelt werden, die als spezifische Binder interagieren. Selbstverständlich ist es auch möglich, die erfindungsgemäße Mittel in nicht peptidische Strukturen wie Ester oder Sulfonamide, Zucker oder andere organische oder anorganische Verbindungen einzubauen. Hierdurch entstehen bevorzugt organische Module, die für das Screening nach spezifischen Bindern eingesetzt werden können.

Es kann beispielsweise bevorzugt sein, die erfindungsgemäßen Mittel mit anderen Substanzen zu kombinieren, wie sie beispielsweise in der WO 2007/021661 offenbart sind. Die Kombination aus den dort offenbarten Verbindungen mit den erfindungsgemäßen Mitteln führt zu neuen Kombinationsmolekülen, die überraschende Eigenschaften aufweisen. Überraschenderweise lassen sich die die neuen Kombinationsmoleküle sehr gut als Target für ein Screeningassay einsetzen. Derartige Verwendungen können beispielsweise für das Screening nach Komponenten genutzt werden, die beispielsweise mit der SH3-Domäne interagieren oder Proteininteraktionen modulieren, insbesondere inhibieren, wen mindestens eine der oben genannten Domänen involviert ist. Dem Fachmann ist bekannt, wie er die erfindungsgemäßen Mittel insbesondere mit Substanzen der WO 2007/021661 gemäß Anspruch 1 bis 11 oder aber gemäß 41 bis 44 oder anderen offenbarten Verbindungen kombinieren oder assoziieren kann, sodass insbesondere ein Einsatz in Screeningassays möglich ist. Beispielsweise ist in Anspruch 1 der WO 2007/021661 die Position einer Aminosäure oder eines analogen Peptiidomimetikum offenbart. Diese Position kann durch mindestens eines der erfindungsgemäßen Mittel eingenommen werden oder durch eine Verbindung, die die erfindungsgemäßen Verbindungen aufweisen. Bevorzugt können die erfindungsgemäßen Mittel als Scaffold verwendet werden, beispielsweise in Substanzen, die beispielsweise in einer Library enthalten sind, oder aber in strukturoptimierten Targets. Die neuen Kombinationsmoleküle können als Liganden für SH3- oder FYN-Domänen eingesetzt werden. Neben den erfindungsgemäßen Mitteln können auch Kombinationen aus diesen und den Verbindungen gemäß WO 2007/021661 verwendet werden, um die Aktivität von Proteinen zu beeinflussen, die eine SH3-Domäne umfassen oder aber eine EVH1-, GYF-, WW- oder aber UEF- und/oder Profilin-Domäne beinhalten. Bevorzugt ist es hierbei beispielsweise, dass die SH3-Domäne ein Teil einer Proteinkinase ist, wobei in einer weiteren bevorzugten Ausführungsform die Proteinkinase Mitglied der Src-Familie ist.

Src-Familienmitglieder sind beispielsweise FYN, Src, Fgr, Yes, Yrk, Lyn, Hck, Lck oder Blk. Die erfindungsgemäßen Mittel sowie Kombinationsmoleküle, die diese umfassen, können zur Behandlung von Krankheiten eingesetzt werden, in denen beispielsweise Src-Familienmitglieder assoziiert sind oder Proteine mit den oben genannten Domänen. Dazu gehören insbesondere kardiovaskuläre Erkrankungen, Entzündungskrankheiten, Immunerkrankungen, Knochenerkrankungen, Erkrankungen mit einem proliferativen Hintergrund wie insbesondere Krebserkrankungen, neurologische Erkrankungen, neurodegenerative Erkrankungen, Ischämie, insbesondere nach einem Schlaganfall, Störungen der Angiogenese, Allergien, Arthritis oder Infektionserkrankungen. Die Erfindung betrifft daher auch eine Bibliothek, insbesondere eine für kombinatorische Screeningmethoden einsetzbare Bibliothek, die die erfindungsgemäßen Mittel bzw. Moleküle, die diese umfassen, umfasst. Mithilfe dieser Bibliotheken können beispielsweise Kandidatenmoleküle detektiert werden, die an SH3-Domänen binden oder an andere der genannten Domänen.

SH3-Domänen können in einer Vielzahl von Proteinen detektiert werden, einschließlich aber nicht beschränkt auf Abl, Src, Grb2, PLCδ, PLCγ, Ras-GAP, Nck und p85-PI-3'-Kinase.

Die Erfindung betrifft auch die Verwendung der erfindungsgemäßen Mittel in isolierter Form oder mit organischen oder anorganischen Verbindungen kombinierten Mitteln als Sonde, die die Substanzen gezielt transportieren. In diesem Zusammenhang können sie beispielsweise nicht nur als Therapeutika sondern auch als Diagnostika eingesetzt werden. Die erfindungsgemäßen Mittel können auch als Sonden verwendet werden oder als Drug Delivery Systeme, um gewünschte Verbindungen bevorzugt zu den oben genannten Domänen zu transportieren oder diese verstärkt miteinander wechselwirken zu lassen.

Die erfindungsgemäßen Mittel sind Liganden bzw. Binder für die genannten Domänen. Überraschenderweise zeigen die erfindungsgemäßen Mittel ein weit höheres Ausmaß an Affinität für die genannten Domänen als bekannte Liganden oder Binder. Diese bindenden oder interagierenden erfindungsgemäßen Mittel können neben den oben genannten therapeutischen Anwendungen auch vielfach in der Forschung eingesetzt werden, beispielsweise in der Bereitstellung eines Verfahrens zur Modulierung von Signaltransduktionswegen, insbesondere auf der zellulären Ebene, zur Modellierung der Aktivität von onkogenen Proteinen oder anderen krankheitsassoziierten Strukturen oder der Bereitstellung von Leitverbindungen neben den oben genannten Arzneistoffen auch für die Entwicklung von Diagnostika im Labormaßstab. Vorteilhafterweise zeigen die erfindungsgemäßen Mittel die Fähigkeit, zahlreiche Proteine zu modulieren, insbesondere solche, die in der Signalübertragung involviert sind. Weiterhin weisen die erfindungsgemäßen Mittel den Vorteil auf, dass sie sowohl breite Klassen wie auch sehr spezifische Klassen von Proteinen modulieren können, wenn sie die oben genannten Domänen aufweisen. Es ist daher möglich, durch Routineversuche erfindungsgemäße Mittel auszuwählen, die für spezielle Domänen spezifisch sind und so die Modulation eines speziellen Proteins modulieren, während sie andere, die ähnliche oder identische Domänen tragen, unbeeinflusst lassen können. Selbstverständlich können die erfindungsgemäßen Mittel auch so ausgewählt werden, dass sie sowohl breite und spezifische Klassen von Domänen beeinflussen, indem sie die Aktivität der jeweiligen Proteine insgesamt modulieren. Die erfindungsgemäßen Mittel dienen daher auch als nützliche Leitklassen bei der Produktion weiterer beispielsweise peptidometrischer Wirkstoffe, die große Klassen von Proteinen mo-dufieren, die in Signaltransduktionswegen involviert sind oder beispielsweise bei der Onkogenese bzw. bei neurodegenerativen Erkrankungen oder aber viralen und bakteriellen Infektionskrankheiten. Somit werden durch die Erfindung neue Motive bereitgestellt, die Variationen bei den Selektivitäten oder Spezifitäten bei der Bindung der oben genannten Domänen reflektieren.

Die vorliegende Erfindung betrifft daher auch Konjugate der erfindungsgemäßen Mittel mit einem zweiten Molekül oder einer zweiten chemischen Gruppe, wobei es sich hierbei um Aminosäuren, Zucker, Kohlenhydrate oder anorganische Strukturen handeln kann. Dieses zweite Molekül kann insbesondere jede Substanz sein, deren Verabreichung an den Bereich der Domäne eines speziellen Proteins angestrebt wird. Es kann sich auch um Zellen handeln, die dieses spezielle Protein enthalten. Mögliche Zielzellen umfassen, sind aber nicht beschränkt auf neurale Zellen, Immunzellen (z. B. T-Zellen, B-Zellen, natürliche Killerzellen und dergleichen), Osteoclasten, Blutplättchen, epidermale Zellen und dergleichen, wobei die Zellen insbesondere die genannten Domänen oder verwandte Proteine exprimieren. Auf diese Weise kann die spezifische Modulation von Aktivitäten Proteinen, die die oben genannten Domänen aufweisen, erreicht werden.

Die erfindungsgemäßen Moleküle oder Strukturen, die diese umfassen, können in einem Verfahren zur Modulation der Aktivität beispielsweise von Src oder von mit Src verwandten Proteinen verwendet werden, welches die Verabreichung einer Zusammensetzung umfasst, die eine wirksame Menge der erfindungsgemäßen Mittel beispielsweise mit einem Träger umfasst. Die Modulation der Aktivität betrifft aber nicht nur Src, sondern alle Moleküle, die die oben genannten Domänen aufweisen (Src-Homologie-3-Domänen, WW-Domänen, Ena-VASP-Homologie-1-Domänen, GYF-Domänen, UEV-Domänen und/oder Profilin). In bevorzugten Ausführungsformen der Erfindung resultiert das in Betracht kommende Verfahren in der Inhibierung der Aktivität von Proteinen, die die genannten Domänen umfassen. In anderen bevorzugten Ausführungsformen ist das Verfahren bei der Aktivierung von Proteinen wirksam, die die genannten Domänen aufweisen.

Darüber hinaus können die Mittel gemäß der Erfindung auch in anderen Bereichen angewendet werden, wie beispielsweise Verfahren der Bildgebung von Zellen, Geweben und Organen, in denen Proteine mit den genannten Domänen exprimiert werden, welche insbesondere die Verabreichung einer wirksamen Menge der erfindungsgemäßen Zusammensetzung umfassen, die die erfindungsgemäßen Mittel aufweist, die mit einem nachweisbaren Marker oder einem bildgebenden Mittel konjugiert ist.

Somit werden durch die Erfindung auch Tests für die Identifizierung einer Verbindung bereitgestellt, die die Bindung zwischen einem ersten Molekül, das eine Domäne ausgewählt aus der Gruppe umfassend Src-Homologie-3-Domänen, WW-Domänen, Ena-VASP-Homologie-1-Domänen, GYF-Domänen, UEV-Domänen und/oder Profilin umfasst und einem zweiten Molekül, welches an diese Domänen bindet, beeinflusst, wobei das zweite Molekül bevorzugt ein Mittel gemäß der Erfindung ist, welches die Inkubation von einer oder mehrerer Kandidatenverbindungen, aus denen die Selektion einer solchen Verbindung gewünscht wird, mit dem ersten Molekül und dem zweiten Molekül unter Bedingungen umfasst, die Bindungen fördern, und den Nachweis der einen oder mehreren Verbindungen, die die Bindung des ersten Moleküls an das zweite Molekül beeinflussen.

Die Mittel der Erfindung können auch in Form von Kits für die Durchführung solcher Tests bereitgestellt werden, die ein erstes Molekül umfassen, das die genannten Domänen umfasst und ein zweites Molekül, das an diese Domänen bindet, wobei das besagte zweite Molekül ein Mittel gemäß der Erfindung ist

Die erfindungsgemäßen Moleküle zeigen einen weiten Bereich von biologischen Aktivitäten, der die Verstärkung oder Inhibition (in Abhängigkeit vom speziellen Peptid oder der Natur des Zielmoleküls) der natürlichen Funktion oder biologischen Funktion des Zielmoleküls der erfindungsgemäßen Mittel umfasst.

Es ist auch die Aufgabe der vorliegenden Erfindung, mit den neuen erfindungsgemäßen Substanzen Tests bereitzustellen, mit welchen bestimmt werden kann, ob eine Kandidatenverbindung in der Lage ist, die Bindung zwischen den genannten Domänen und einem Liganden für diese Domänen zu beeinflussen. Eine Verbindung, die in der Lage ist, diese Bindung zu modifizieren, wäre als Mittel zur Modulation der pharmakologischen Aktivität von Strukturen von Nutzen, die die genannten Domänen enthalten. Die vorliegende Erfindung stellt geeignete Liganden für diese Domänen für die Verwendung in solchen Tests bereit. Überraschenderweise hat sich gezeigt, dass die erfindungsgemäßen Mittel in diesen Tests überraschend gut eingesetzt werden können. Demgemäß werden durch die Erfindung auch Verfahren für die Identifizierung von Verbindungen bereitgestellt, die die Bindung eines Moleküls beeinflusst, welches eine der oben genannten Domänen umfasst und einen Liganden der Domäne, wobei der Ligand der Domäne das erfindungsgemäße Mittel ist Die Wirkung auf die Bindung kann insgesamt die Bindung oder die Affinität der Bindung verstärken oder schwächen. Vorzugsweise ist die Wirkung eine Inhibierung, beispielsweise in Form einer Reduktion oder des Verlustes der Bindung.

Die Erfindung betrifft daher auch ein Verfahren der Identifizierung eines Inhibitors der Bindung zwischen einem Molekül, welches eine Domäne ausgewählt aus der Gruppe umfassend Src-Homologie-3-Domänen, WW-Domänen, Ena-VASP-Homologie-1-Domänen, GYF-Domänen, UEV-Domänen und/oder Profilin umfasst und einem zweiten Molekül, das mindestens ein erfindungsgemäßes Mittel umfasst. Dieses Verfahren umfasst auch die Inkubation von mindestens einer oder aber mehreren Verbindungen, aus denen ein Inhibitor/Aktivator selektiert werden soll, mit dem ersten Molekül und einem Molekül gemäß der Erfindung und zwar unter Bedingungen, die für die Bindung förderlich sind. Das Verfahren umfasst auch den Nachweis der einen oder mehreren Verbindungen, die die Bindung des ersten Moleküls an das erfindungsgemäße Molekül inhibieren. Ein erfindungsgemäßer Test würde demgemäß mindestens aus einem Molekül bestehen, welches eine der oben genannten Domänen umfasst, mindestens einem der erfindungsgemäßen Mittel, beispielsweise in ein Peptid oder in eine andere Struktur eingebaut und einer Kandidatenverbindung, von der man annimmt, dass sie die Fähigkeit hat, die Bindung zwischen dem ersten Molekül und dem Liganden gemäß der Erfindung zu beeinflussen. Der Test kann weiterhin ein Mittel zum Nachweis der Bindung zwischen dem ersten Molekül und dem Liganden gemäß der Erfindung beinhalten. Solche Mittel können zum Beispiel nachweisbare Markierungen sein, die mit dem ersten Molekül, dem Liganden gemäß der Erfindung oder der Kandidatenverbindung verknüpft sind. Ein Verfahren, bei dem der Test im Sinne der Erfindung eingesetzt wird, umfasst in einer bevorzugten Ausführungsform folgende Schritte:
- In-Kontakt-Bringen der Domäne mit dem Mittel gemäß der Erfindung unter Bedingungen, die geeignet sind für die Bindung in Anwesenheit einer Kandidatenverbindung und Messen des Umfangs der Bindung zwischen der Domäne und dem Liganden;
- Vergleichen des im ersten Schritt gemessen Umfangs der Bindung mit dem Umfang der Bindung von der bekannt ist oder die bestimmt wurde, dass sie zwischen der Domäne und dem Mittel der Erfindung in Abwesenheit einer Kandidatenverbindung auftritt, wobei ein Unterschied zwischen dem im ersten Schritt gemessenen Umfang der Bindung und dem Umfang der Bindung, von der bekannt ist oder die bestimmt wurde, dass sie zwischen der Domäne und dem Mittel der Erfindung in Abwesenheit einer Kandidatenverbindung auftritt, darauf hinweist, dass die Kandidatenverbindung eine Verbindung ist, die die Bindung zwischen dem Molekül, das die Domäne umfasst, und dem Mittel gemäß der Erfindung beeinflusst.

Das Verfahren kann auch einen weiteren Schritt umfassen, der die Formulierung des detektieren Kandidatenmoleküls in einer pharmazeutisch akzeptablen Form umfasst. In allen genannten Verfahren und Tests können die erfindungsgemäßen Mittel als Bestandteil von Substanzen in einer Library eingesetzt werden, mit der beispielsweise definierte Targets detektiert werden sollen.

Es ist aber auch bevorzugt, mithilfe der erfindungsgemäßen Mittel optimierte Targets, insbesondere strukturoptimierte Targets zu erzeugen, die das erfindungsgemäße Mittel aufweisen und vorteilhafterweise eine Faltung aufweisen, die die Interaktion mit zu testenden Molekülen verbessert.

Selbstverständlich ist es auch möglich, die erfindungsgemäßen Mittel wiederum mit anderen Mimetika oder Peptidomimetika zu kombinieren; auch die Kombination mit Aminosäuren oder organischen Molekülen ist vorteilhaft.

Die anmeldungsgemäße Lehre zeichnet sich durch folgende Merkmale aus:
- Abkehr vom technisch Üblichen
- neue Aufgabenstellung
- Vorliegen eines seit langem ungelösten dringenden Bedürfnisses für die Lösung des mit der Erfindung gelösten Problems
- bisheriges vergebliches Bemühen der Fachwelt
- die Einfachheit der Lösung spricht für erfinderische Tätigkeit, insbesondere da sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteil)
- technischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren Mittels, Eröffnung eines zweiten Weges, Eröffnung eines neuen Gebietes, erstmalige Lösung einer Aufgabe, Reservemittel, Alternativen, Möglichkeit der Rationalisierung, Automatisierung oder Miniaturisierung oder Bereichung des Arzneimittelschatzes
- glücklicher Griff, da aus einer Vielzahl von Möglichkeiten eine bestimmte gewählt wurde, deren Ergebnis nicht vorausgesagt werden konnte, daher handelt es sich um ein patentwürdigen glücklichen Griff)
- Irrtümer in der Fachliteratur bzw. sehr widersprüchliche Darstellung zum Erfindungsgegenstand
- junges Gebiet der Technik
- Kombinationsefindung, d.h. mehrere bekannte Elemente werden zu einer Kombination zusammengeführt, die einen überraschenden Effekt aufweist
- Lizenzvergabe
- Lob der Fachwelt und
- wirtschaftlicher Erfolg.

Insbesondere die vorteilhaften Ausführungsformen der Erfindung weisen mindestens einen oder mehrere der genannten Vorteile auf.

Im Folgenden soll die Erfindung anhand der Synthese des Diprolin-Mimetikums 85 näher erläutert werden, ohne auf dieses beschränkt zu sein.

Motivation war die Suche nach Molekülen, die in der Lage sind, mit hoher Affinität an die EVH1-0omäne zu binden und so die nativen prolinreichen Sequenzen der Ligandenpeptide als Bindungspartner zu ersetzen. Auf Basis von Molecular-Modeling-Studien zur Interaktion von Ligandenpeptiden mit der VASP-EVH1-Domäne wurde Verbindung 85 entworfen, die als vielversprechendes Modul (Dipeptidmimetikum) in Testpeptide eingebaut werden könnte, um dort zwei benachbarte Prolin-Positionen des FPPPP-Kemmotivs zu ersetzen. Leitlinien des Struktur-Designs waren (1) eine geometrisch fixierte, helicale Struktur (Tricyclus), (2) eine größtmögliche Übereinstimmung der Bindungswinkel und-abstände im Vergleich zu denen des natürlichen Prolin-Prolin-Dipeptids (in der PPII-HelixKonformation), (3) eine zentrale Wasserstoffbrücken-Akzeptor-Funktion in Form einer Carbonylgruppe, und (4) eine Aminosäure-artige Gesamtstruktur mit einem Fmoc-geschütztem *N*-Terminus und einem freiem C-Terminus, um den geplanten Einbau in Peptide methodisch einfach zu gestalten. Diese Anforderungen werden von dem nachfolgend abgebildeten Molekül **85** erfüllt, das ein konformationell eingeschränktes Analogon zweier konsekutiver Proline in der PPII-Helix darstellt Durch den Einbau der Z-konfigurierten Olefinbrücke werden die Prolinringe in der vermuteten biologisch aktiven Konformation stabilisiert, der zentrale Siebenring gewährleistet eine perfekte Fixierung der *trans*-Amidbindung.

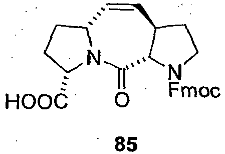

Zur Bereitstellung dieser Verbindung in substanziellen Mengen musste ein gangbarer, stereoselektiver Syntheseweg ausgearbeitet werden. Hierfür wurde eine Strategie gewählt, bei der das Zielmolekül in konvergenter Weise (retrosynthetische Zerlegung des zentralen Siebenringes, s. Schema 20) auf zwei Vinylproline vom Typ **100** und 101 zurückgeführt wird. Diese könnten zunächst durch eine Peptidkupplung miteinander verknüpft werden, bevor dann der Tricyclus durch Olefin-Metathese geschlossen würde. Ringschlussmetathesen zu Siebenringen sind in der Literatur hinreichend bekannt. Die beiden unterschiedlich substituierten Vinylprolin-Derivate **100** und **101** wiederum müssten jeweils stereoselektiv synthetisiert werden. Als Startmaterial könnte in beiden Fällen *L*-Pyroglutaminsäure dienen.

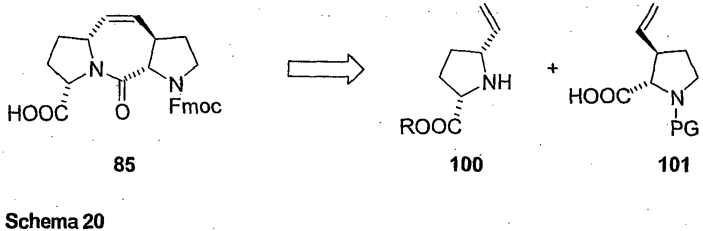

Als Baustein vom Typ **101** wurde das *trans*-3-Vinylprolin **166** mit einer N-Boc-Scutzgruppe avisiert, dessen Synthese mit der Umwandlung von *L*-Pyroglutaminsäure **133** in den Baustein 136 begann (Schema 31).

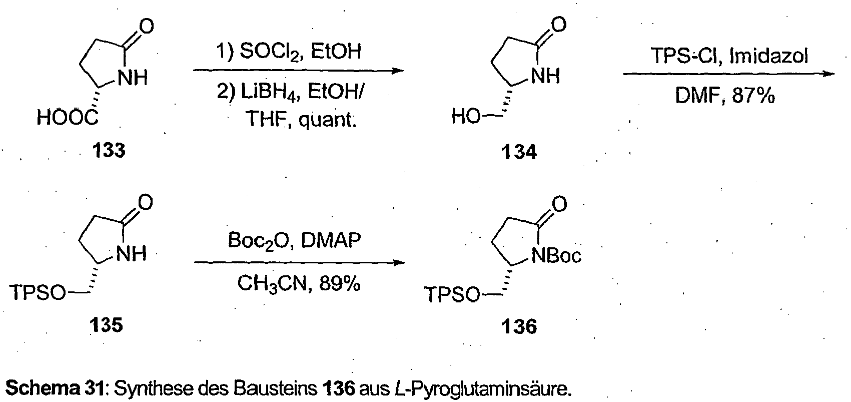

Hierzu wurde die Säure **133** zunächst in den Ethylester überführt, dessen Reduktion mit Lithiumborhydrid quantitativ den sehr polaren Alkohol 134 lieferte. Dieser wurde mit der stabilen und sterisch anspruchsvollen *tert*-Butyldiphenylsilyl-Gruppe (TPS) geschützt (87 %), die bei der späteren Einführung des neuen Substituenten die "Unterseite" des Moleküls möglichst abschirmen sollte, um die Bildung der gewünschten *trans*-Konfiguration zu begünstigen. Nachfolgend wurde das Stickstoffatom von 135 mit einer Boc-Schutzgruppe versehen (89 %), wobei nach einem modifizierten Verfahren nur eine katalytische Menge DMAP und kein weiterer stöchiometrischer Basenzusatz nötig war. Das so im Mulfigramm-Maßstab hergestellte, stabile und gut kristallisierbare Intermediat 136 diente als Ausgangsbasis für die weitere Synthese des *trans*-3-Vinylprolins **166,** wobei auf das Synthesekonzept von *Herdeis et al.* zurückgegriffen werden konnte. Nach Literaturvorschrift wurde **136** bei -78 °C mit der sterisch gehinderten Base LiHMDS in α-Position zur Carbonylgruppe deprotoniert und durch Umsetzen mit Phenylselenylchlorid in den Selenylether überführt, der durch Aufarbeitung des Rohproduktes bei -78 °C mit Wasserstoffperoxid (30%ig) oder mit Ozon zum Selenoxid oxidiert werden sollte, aus dem bei Erwärmen auf Raumtemperatur durch Eliminierung das Enon **18** entstehen sollte (Schema 41).

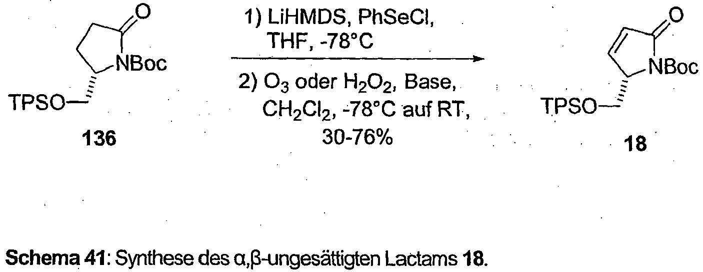

Trotz genauer Einhaltung der Literaturbedingungen ergaben sich allerdings Probleme bei der Reproduktion der Ergebnisse: Einerseits waren die Ausbeuten nicht konstant und teilweise unbefriedigend niedrig, andererseits wurde das Enon 18 nicht in sauberer Qualität erhalten (Auch in der Literatur variieren die erzielten Ausbeuten stark, d.h. von 54 bis 86 %, für das gleiche Substrat, was als Hinweis auf praktische Probleme gewertet werden kann).

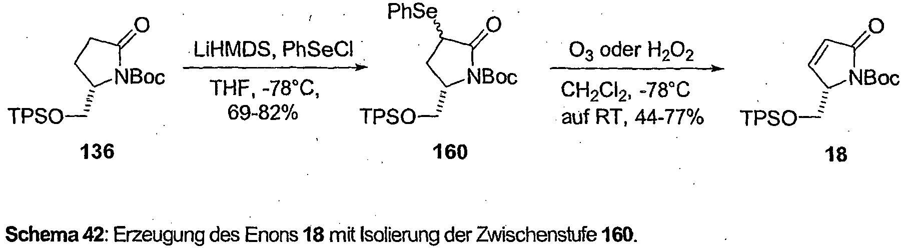

Zur näheren Untersuchung des Sachverhaltes wurden umfangreiche Testexperimente durchgeführt. Zunächst wurde dazu die selenierte Zwischenstufe **160** isoliert und chromatographisch aufgereinigt, um Reste des Edukts **136** (nach Eliminierung schlecht abtrennbar) als auch des Phenylselenylchlorids (verbraucht ebenfalls Oxidationsmittel) abzutrennen (Schema 42). Die Ausbeuten an erhaltener Zwischenstufe **160** waren mit den Ergebnissen in der Literatur vergleichbar (69-82%).

Problematischer gestaltete sich die saubere und quantitative Eliminierung zum Enon 18, weshalb verschiedene in Frage kommende Parameter (Lösungsmittel, Temperatur, Base, Oxidationsmittel) variiert wurden. Die Verwendung verschiedener Lösungsmittel (Ethylacetat, absolutes oder nicht-absolutes Dichlormethan) oder verschiedener Temperaturen (-78 °C, -10 °C, °C, unterschiedlich rasche Erwärmung auf Raumtemperatur oder auf 70°C) führte zu keiner erkennbaren Änderung. Vor allem wurde aber der Einfluss des Oxidationsmittels (H₂O₂ in Konzentrationen von 10 bis 30 %, Ozon, MCPBA, Natriumperiodat, jeweils verschiedene Äquivalentmengen) und der Base (Pyridin, Natriumacetat, verschiedene Äquivalentmengen oder auch kein Basenzusatz) überprüft. Die Ergebnisse können wie folgt zusammengefasst werden:
- Als Oxidationsmittel erwiesen sich Ozon oder H₂O₂ (25-30 %ig) gleichermaßen geeignet, auch MCPBA konnte verwendet werden, Natriumperiodat hingegen war nicht stark genug. Doch trotz anscheinend sauberer Reaktion auf dem Dünnschichtchromatogramm lagen die isolierten Ausbeuten z.T. erheblich niedriger, wobei der Verbleib der restlichen Substanz nicht abschließend geklärt werden konnte (Überoxidation? Zersetzung?).
- Es deutete sich an, dass ein (in der Literatur üblicher) Basenzusatz zur leichten Steigerung der Ausbeute von Vorteil sein kann, doch wurde hierdurch auch die Bildung eines Isomerisierungsproduktes in wechselnden Anteilen begünstigt, dessen Struktur vermutlich mit dem Pyrrolderivat **161** angeben werden kann (Schema 43, vgl. auch analytische Daten im Abschnitt 7.2.3.18). Auch eine basische Aufarbeitung der Reaktionsmischung führte verstärkt zur Bildung von **161.**

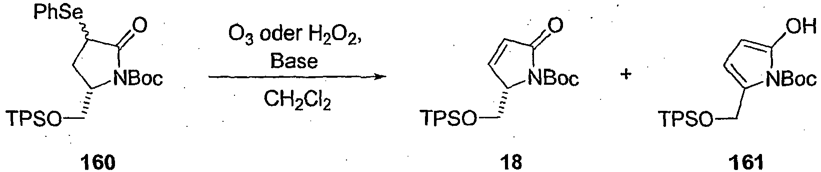

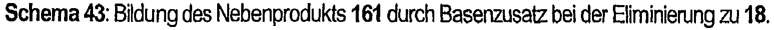

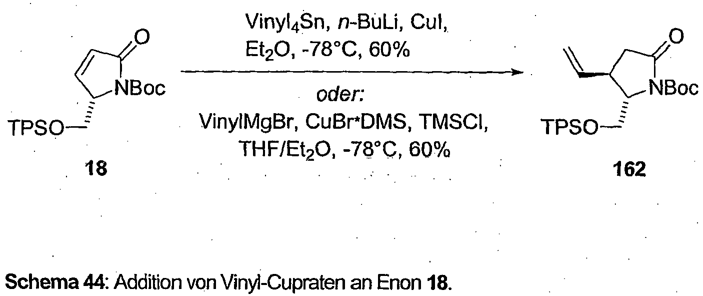

Mit dem erhaltenen Enon 18 wurde anschließend die Einführung der Vinylgruppe durch 1,4-Additionzunächst mit Gilman-Cupraten des Typs R₂CuLi - durchgeführt (Schema 44). Das hierfür benötigte Vinyllithium wurde aus Tetravinylzinn und *n*-Butyllithium durch Transmetallierung bei 0° C gewonnen. Ohne Isolierung erfolgte bei -20 °C durch Zugabe von Kupfer(I)iodid die Umwandlung in das Divinyl-Cuprat, zu erkennen an der Schwarzfärbung der Suspension, und nachfolgend bei-78 °C die Addition an das Enon durch Zugabe einer Lösung von 18 in Diethylether. Hierbei wurde das Additionsprodukt **162** in einer Ausbeute von 60 % gewonnen, was im Bereich der Literaturangaben liegt. Weiterhin konnte in Übereinstimmung mit der Literatur lediglich ein Diastereomer des Vinyl-Additionsprodukts gefunden werden. Teilweise kam es zur Bildung eines Nebenproduktes, das durch unvollständige Transmetallierung und daraus resultierende Addition eines *n*-Butylrests an **18** entstanden war.

Um den Einsatz des toxischen und teuren Tetravinylzinns zu umgehen, wurde im folgenden ein Nor-mant-Cuprat vom Typ R₂CuMgBr·DMS verwendet, das leicht durch Umsetzen einer Grignard-Verbindung mit CuBr·DMS erhältlich ist und in vergleichbarer Ausbeute (60 %), allerdings etwas geringerer Reinheit, zum 1,4-Additionsprodukt 162 führte.

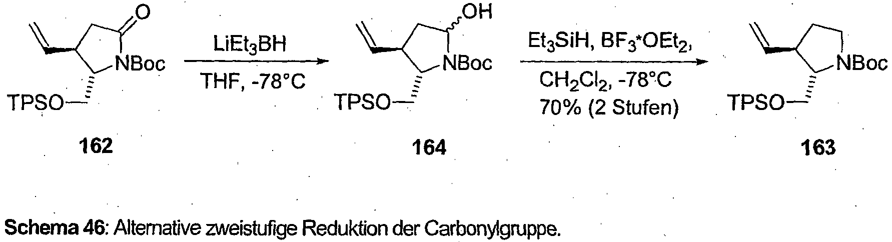

Zur Entfernung der LActam-Carbonylgruppe bot sich eine zweistufige Reduktionssequenz an (Schema 46): Zunächst wurde das *N*-Acyl-Lactam **162** mit Superhydrid zum α-Hydroxycarbamat 164 reduziert, das anschließend mit Triethylsilan (oder alternativ Triphenylsilan) und Bortrifluorid zum sekundären, weiterhin Boc-geschützten Amin **163** deoxygeniert wurde (70 % über zwei Stufen).

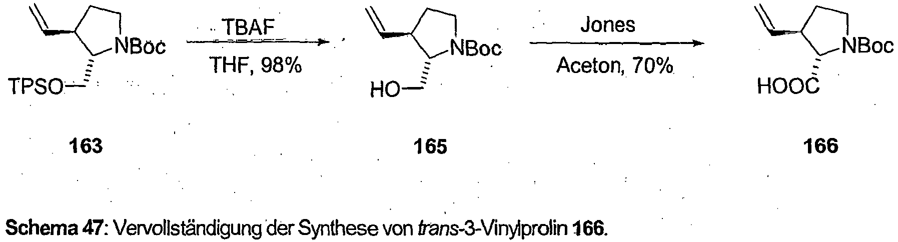

Es folgten die Spaltung des Silylethers mit TBAF in THF (98 %) und die Oxidation des resultierenden Alkohols **165** zur Carbonsäure (Schema 47). Hier konnte ebenfalls nicht auf die Bedingungen von *Herdeis* et al. zurückgegriffen werden, da die Vinylgruppe nicht mit den Bedingungen der Sharpless-Oxidation (NaIO₄/RuCl₃) kompatibel ist Unter Verwendung des Jones-Reagenz gelang die Oxidation zur Carbonsäure mit 70 % Ausbeute, wodurch die Synthese des *N*-Boc*-trans*-3-Vinyl-Prolins **166** ver-vollständigt werden konnte. Die korrekte Konstitution und die relative Konfiguration *(trans)* wurde durch eine Röntgen-Kristallstrukturanalyse bestätigt.

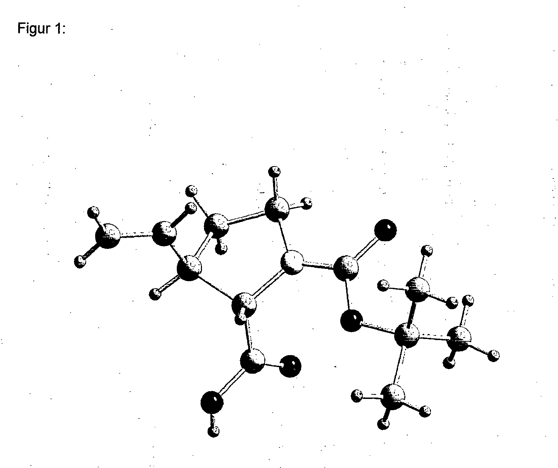

Figur 1 zeigt die Kristallstruktur des *N*-Boc-geschützten *trans*-3-Vinylprolins **166.**

Zur Synthese des zweiten Bausteins, einem *cis*-5-Vinylprolin-esters vom Typ **100** wurde auf die Route von *Mulzer* und *Schülzchen* zurückgegriffen, allerdings zunächst unter Verwendung des kommerziell erhältlichen *L*-Pyroglutaminsäure-ethylesters. Dieser wurde mit einer Boc-Schutzgruppe versehen und das geschützte Substrat **167** (Schema 48) bei tiefer Temperatur mit DIBAL-H selektiv zum α-Hydroxycarbamat reduziert, Bei langsamer Zugabe des Reduktionsmittels bei tiefer Temperatur tritt keine Reduktion des Esters auf, da die Carbonylgruppe des *N*-Acyl-Lactams bevorzugt reagiert. Nach neutraler Aufarbeitung mit Kalium-Natrium-Tartrat-Lösung wurde das erhaltene Rohprodukt mit einer katalytischen Menge PPTS in Methanol in das α-Methoxycarbamat 168 überführt (Epimerengemisch, 90 % über zwei Stufen).

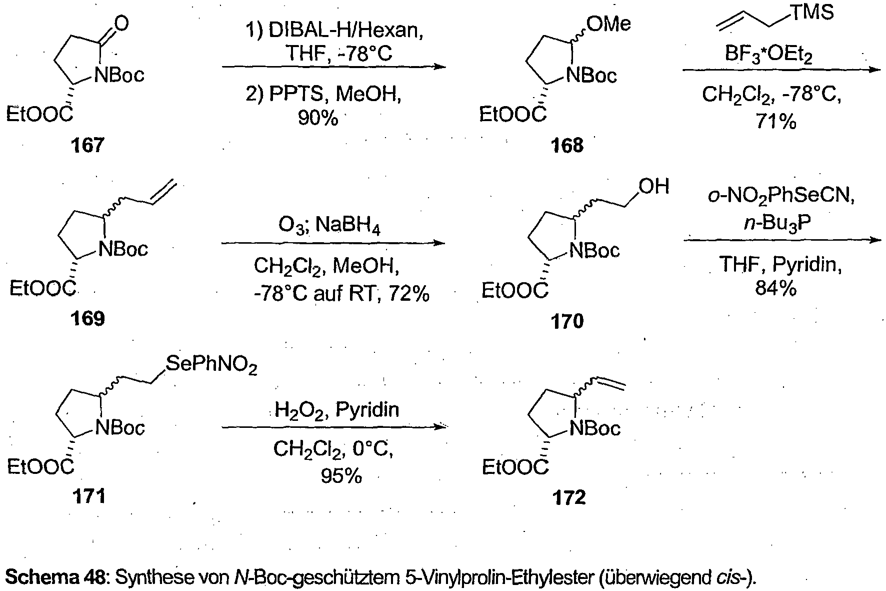

Zur Einführung des zweiten Substituenten wurde **168** mit Bortrifluorid und Allyltrimethylsilan umgesetzt, worauf man das Allyl-Substitutionsprodukt **169** als untrennbares Diastereomerengemisch erhielt *(cis*/*trans* = 4:1, zusammen 71 %), Die Doppelbindung von **169** wurde anschließend durch Ozonolyse gespalten und das entstehende Ozonid mit Natriumborhydrid zum Alkohol **170** reduziert, wobei eine Reduktion des Ethylesters nicht bzw. nur in Spuren beobachtet wurde (72 %). Zur Überführung in das Olefin 172 wurde der Alkohol 170 nach *Grieco et al*. unter Verwendung von *ortho-*Nitrophenylselenocyanat und *n*-Tributylphosphin umgesetzt, wodurch zunächst der Selenylether 171 erhalten wurde (84 %). Durch Oxidation bei 0 °C mit H₂O₂ (30%ig) und nachfolgende Eliminierung konnte das Vinylprolin 172 als Diastereomerengemisch erhalten werden (95 %).

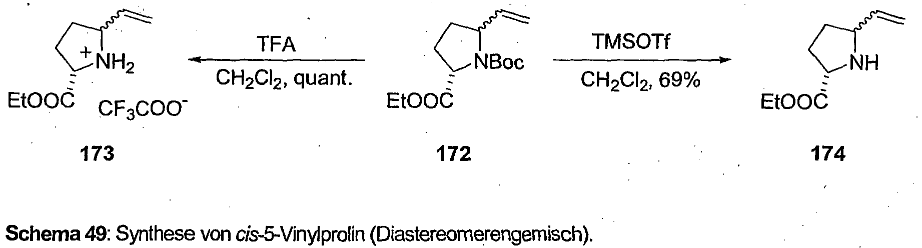

Die Abspaltung der Boc-Schutzgruppe (Schema 49) gelang durch Behandlung mit Trifluoressigsäure (quantitativ) oder mit TMSOTf (69 %). Letzteres führte nach Hydrolyse zum chromatographierbaren freien Amin **174,** das so in reiner Form, jedoch nach wie vor als untrennbares Diastereomerengemisch erhalten wurde (Anteil *cis*-Produkt: 80 %).

Mit dem so erhaltenen Material wurde die weitere Synthese der tricyclischen Diprolinmimetika ausgelotet (Peptidkupplung und nachfolgende Ringschlussmetathese). Trotz sterischer Hinderung durch die beiden Vinylgruppen gelang die Knüpfung der Peptidbindung unter Standardbedingungen in guter Ausbeute (Schema 52). Dabei wurde die Carbonsäure 166 mit EDAC/HOBt präaktiviert, bevor die Zugabe des Amins 173 und Triethylamin als Base erfolgte. Die Identität des Kupplungsproduktes konnte im ¹H-NMR-Spektrum trotz Vorliegen eines Diastereomeren- und Rotamerengemisches bestätigt werden.

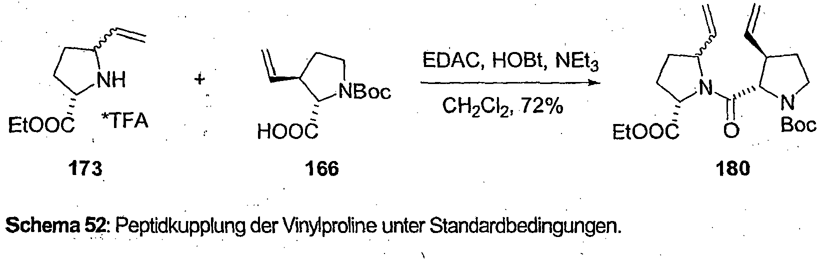

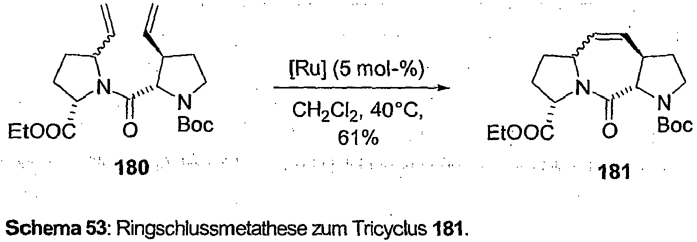

Um den Tricyclus **181** durch Ringschlussmetathese zu erzeugen, wurde Dipeptid 180 (Schema 53) zunächst mit dem Grubbs-II-Katalysator (5 mol-%) versetzt, jedoch blieb der Umsatz unvollständig, weshalb auf den aktiven und luftstabilen Metathese-Katalysator 82 von *Blechert et al.* zurückgegriffen wurde (für dessen einfache Synthese aus dem Grubbs-II-Katalysator s. Schema 54). Hiermit verlief die Reaktion it. Dünnschichtchromatogramm schließlich vollständig ab (isolierte Ausbeute 61 % im 10 mg-Maßstab). Der Erfolg der Ringschlussmetathese ließ sich dabei im ¹H-NMR-Spektrum eindeutig anhand der Änderung der Olefinsignale belegen.

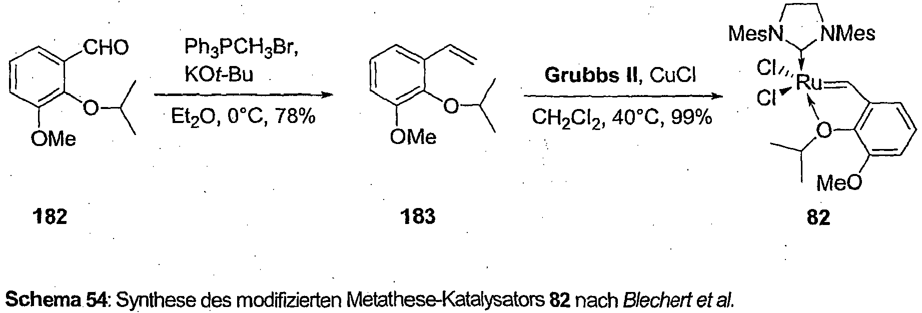

Nach der gelungenen Erzeugung des Tricyclus **181** erschien es angebracht die Synthese des Peptidmimetikums 85 mit diastereomerenreinen Komponenten vorzunehmen. Die Auftrennung der *cis*/*trans-*Isomere des Allyl-Substitutionsproduktes ist nach *Mulzer*/*Schülzchen* unter Verwendung präparativer HPLC im Falle des *tert-*Butylesters **46** möglich, was im vorliegenden Fall aber aus praktischen Gründen ausschied. Jedoch ist die Trennung von **46** nach Entfernung der Boc-Schutzgruppe auch durch Säulenchromatographie beschrieben worden.

Daher wurde die Synthese des tert-Butylester-Bausteins **185** durchgeführt. Hierzu wurde Pyroglutaminsäure **133** mit *tert*-Butylacetat durch Umesterung unter Katalyse von Phosphorsäure (60%ig) in den *tert-*Butylester umgewandelt (58 %), der mit einer Boc-Schutzgruppe versehen wurde (84%, Schema 55). Wie zuvor folgten Reduktion von **44** zum α-Hydroxycarbamat mit DIBAL-H und anschließende Überführung in das korrespondierende α-Ethoxycarbamat **45** (89 % über 2 Stufen).

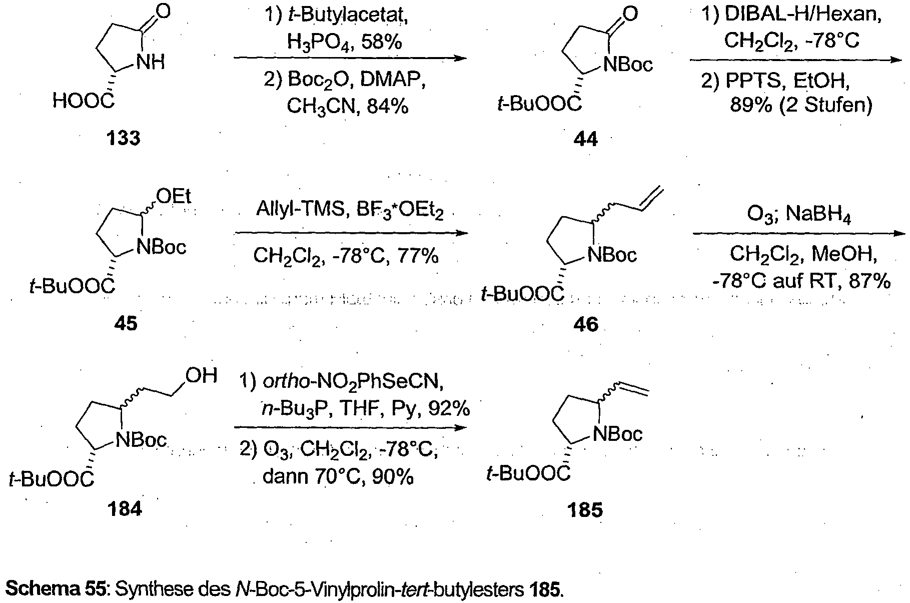

Substitution an **45** mit Allyltrimethylsilan/Bortrifluorid führte erwartungsgemäß zum Diastereomerengemisch des Allyl-Substitutionsproduktes **46** (77%, *cis*/*trans* 75:25), das säulenchromatographisch nicht aufgetrennt werden konnte. Eine Abspaltung der *N*-Schutzgruppe an dieser Stelle wurde nicht angestrebt, da die Auftrennung im Multigramm-Maßstab aufwändig gewesen wäre und die Sequenz aus Entschützung-Diastereomerentrennung-Schützung einen Umweg bedeutet hätte. Stattdessen wurde eine Trennung der Diastereomere auf der Stufe des Vinylprolinesters **185** favorisiert, da hier ohnehin die *N*-schutzgruppe für die Peptidkupplung entfernt werden musste.

Nach Ozonolyse von **46** und Reduktion zum Alkohol **184** mit NaBH₄ (87%) folgte die Umsetzung zum entsprechenden Selenylether, dessen Oxidation mit Ozon bei-78°C und nachfolgende Selenoxid-Eliminierung bei 70°C zum 5-Vinylprolin-*tert-*butylester **185** führte (92%). Hierbei war festzustellen, dass die intensiv-gelb gefärbten, selenhaltigen Neben-produkte recht schwierig abzutrennen waren. Trotz mehrfacher Chromatographie mit unterschiedlichen Laufmittelkombinationen gelang dieses nicht vollständig, und es wurde kein farbloses Produkt erhalten. Doch wies das ¹H-NMR-Spektrum von 185 keine erkennbaren Verunreinigungen von Nitrophenylselen-Spezies mehr auf (d.h. Reinheit > 95%). Die rest-lichen, intensiv-gelben Verunreinigungen konnten aber auf der nächsten Stufe vollständig abgetrennt werden.

Zur Abspaltung der Boc-Schutzgruppe unter Erhalt des ebenfalls säurelabilen *tert*-Butylesters war eine Differenzierung der beiden Schutzgruppen notwendig, wofür z.B. TMSOTf, TBSOTf oder TMSClO₄ in der Literatur beschrieben werden. (Umgekehrt ist auch die Verseifung von Estern unter Erhalt der Boc-Gruppe möglich, wofür man z.B. KOSiMe₃ einsetzen kann.)

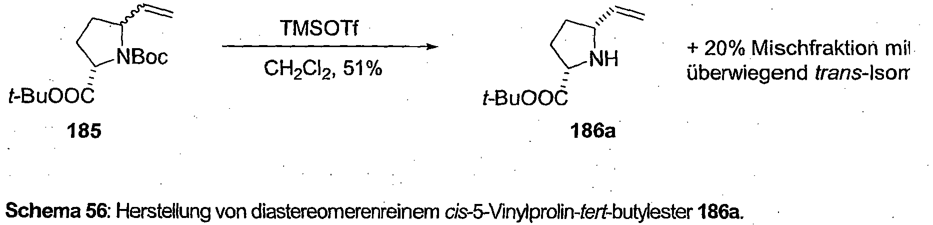

Das langsame Versetzen einer Lösung von **185** mit einer äquimolaren Menge TMSOTf (1.01 Äquivalente) führte zur gewünschten Entschützung des Stickstoffs unter Erhalt der Esterfunktion (Schema 56). In Folge waren die beiden Diastereomere hinreichend verschieden, so dass ihre Trennung durch sorg-fältige Chromatographie gelang und man den *cis*-5-Vinylprolinester **186a** als leicht gelbliches Öl erhielt (51 %). Die relative Stereochemie der beiden Substituenten konnte durch NOE-Experimente bestätigt werden. Da eine allmähliche Epimerisierung der Verbindung eintrat, wurde das Produkt unmittelbar weiter umgesetzt oder in einer Benzolmatrix eingefroren gelagert.

Zur Synthese des konformationell fixierten Diprolin-Peptidmimetikums 85 wurde eine Peptidkupplung der diastereomerenreinen Vinylproline **166** und **186a** unter EDAC/HOBt-Bedingungen versucht, die aber nur zu einer geringen Ausbeute an gewünschtem Produkt 187 (38%) und zu einer erheblichen Menge Nebenprodukt (24%) führte, dessen NMR-Spektren einer epimerisierten Verbindung zugeordnet werden konnten. Da in einem anderen Zusammenhang beschrieben wurde, dass die Verwendung von Triethylamin als Base zu einer Epimerisierung bestimmter Stereozentren führen kann, wurde es im vorliegenden Fall durch DIPEA ersetzt. Außerdem kam als Kupplungsreagenz das reaktivere PyBOP zum Einsatz, wodurch die Präaktivierung der Carbonsäure entfallen konnte. Auf diese Weise ließen sich Ausbeuten an Dipeptid **187** von 81 % realisieren, und es wurde ein Zurückdrängen des (allerdings gut abtrennbaren) Epimerisierungsproduktes erreicht (Schema 57).

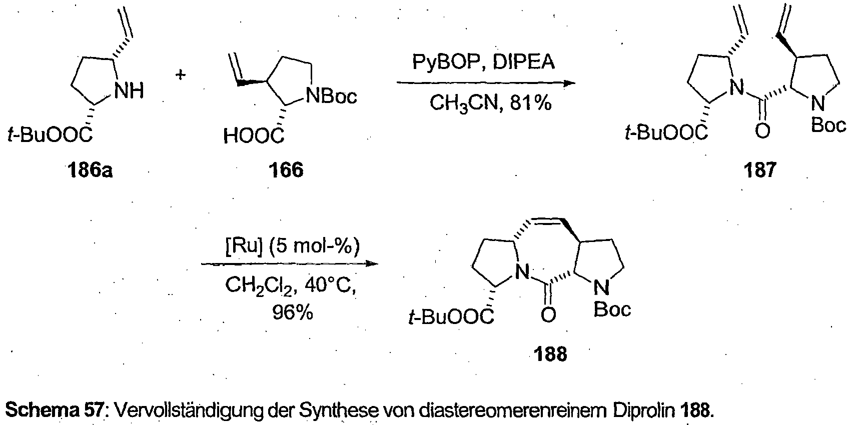

Für die Ringschlussmetathese zum Tricyclus **188** wurden zwei verschiedene Katalysatoren getestet der luftstabile Katalysator 82 nach *Blechert et al.* und der Grubbs II-Katalysator 81. Mit beiden wurden vergleichbare Ergebnisse erzielt: Für einen vollständigen Umsatz des Eduktes 187 benötigte man ca. 5 mol-% des entsprechenden Katalysators bei Reaktion über Nacht (40°C). Nach Aufarbeitung der Reaktion, bei der darauf geachtet wurde, alle Katalysatorrückstände möglichst vollständig zu entfernen, wurden hohe Ausbeuten bis zu 96% realisiert.

Um den synthetisierten Tricyclus **188** für die Festphasen-Peptidsynthese nutzbar zu machen, mussten schließlich beide Schutzgruppen abgespalten und die Aminfunktion mit einer Fmoc-Schutzgruppe versehen werden. Diese ist die einzige *N*-Schutzgruppe von praktischer Bedeutung, die unter milden basischen Bedingungen abgespalten werden kann, wofür in der Regel sekundäre Amine wie Piperidin, Morpholin u.a. verwendet werden. Dadurch wird die Generierung der ungeschützten Aminofunktion bei der Festphasensynthese ohne zusätzliche Produktion von Salzen ermöglicht. Die erhöhte Basenlabilität beeinträchtigt den Einsatz der Fmoc-Gruppe in der organischen Synthese und erfordert deshalb den späten Wechsel der Schutzgruppe.

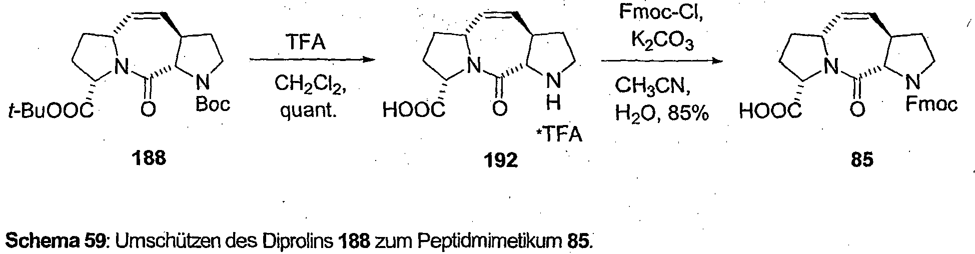

Zur gemeinsamen sauren Abspaltung der Boc-Schutzgruppe und des *tert*-Butylesters wurde **188** mit einem Überschuss Trifluoressigsäure (99%ig) in Dichlormethan versetzt und bei Raumtemperatur gerührt (Schema 59). Nach Einengen erhielt man die freie Aminosäure **192** als gelblichen Feststoff (Kontroll-NMR-Spektrum in D₂O), der in einem Acetonitril/Wasser-Gemisch aufgenommen und unter Schotten-Baumann-Bedingungen in das Fmoc-geschützte Endprodukt **85** überführt wurde. Dieses erwies sich als ausgesprochen polar, aber konnte dennoch mit Chloroform aus der wäßrigen Phase bei pH ≈ 4 extrahiert und anschließend säulenchromatographisch an Kieselgel aufgereinigt werden. Man erhielt 85 als amorphen altweißen Feststoff (85 %), dessen NMR-Spektren sich mit den Erwartungen deckten (siehe Figur 2 für 500 MHz ¹H-NMR-Spektrum (MeOH-d⁴), 60:40 Rotamerengemisch).

Figur 2 zeigt das ¹H-NMR-Spektrum des Diprolin-Bausteins 85 (500 MHz, MeOH-d⁴ RT).

### Biologische Ergebnisse

Insgesamt konnten etwa 300 mg des konformationell fixierten Diprolins 85 synthetisiert und zwecks Einbau in Test-Ligandenpeptide und biologische Bindungsstudien an das FMP in Berlin-Buch übergeben werden. Basierend auf der Sequenz ₃₃₂SFEFPPPPTEDEL₃₄₄ des ActA-Peptids von *Listeria Monocytogenes*, das als stark bindendes, natives Ligandenpeptid der EVH1-Domäne bekannt ist, wurden bis zum Abschluss dieser Arbeit zwei Testpeptide synthetisiert und auf ihre Bindungsaffinität mit dem VASP-EVH1-Rezeptor hin untersucht : ₃₃₂SFEFP*ₚₚ*PTEDEL₃₄₄ **(I)** und ₃₃₂SFEWP*ₚₚ*PTEDEL₃₄₄ **(II),** wobei "*pp*" den synthetischen Diprolin-Baustein 85 repräsentiert. Bereits in früheren Substitutionsexperimenten hatte sich gezeigt, dass der Austausch von Phenylalanin (P) gegen Tryptophan (W) zu einer deutlich höheren Bindungsaffinität mit der VASP-EVH1-Domäne führt K_{D} =13.7 µM für das Ligandenpeptid mit WPPPP-Kemmotiv im Vergleich zu K_{D} = 55.1 µM für den Liganden mit FPPPP-Kemmotiv.

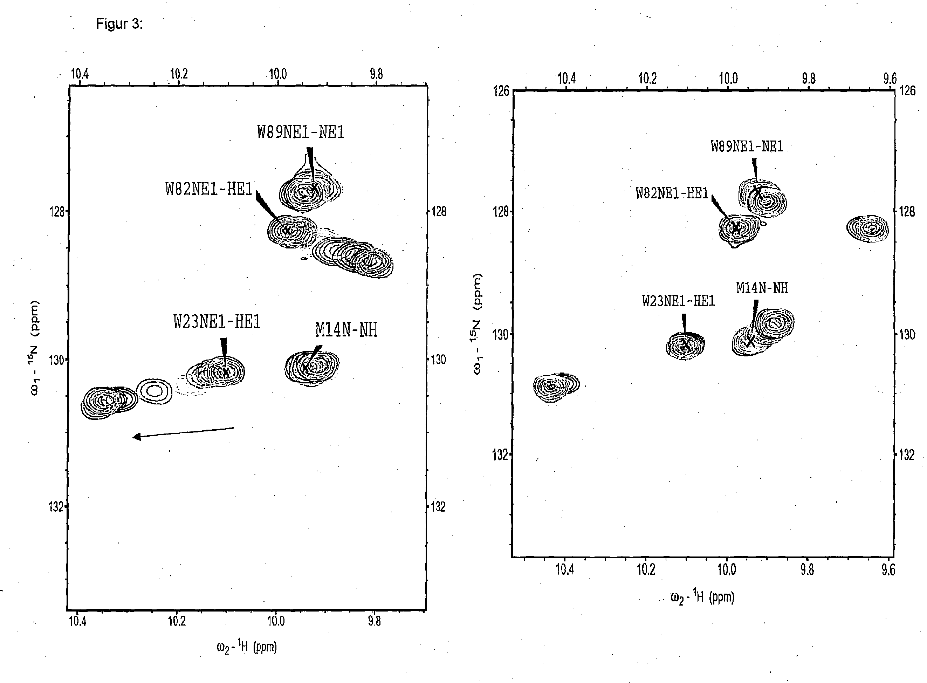

Figur 3 zeigt Überlagerte Serien zweidimensionaler ¹⁵N-¹H-HSOC-NMR-Spektren der VASP-EVH1-Domäne (600 MHz) unter dem Einfluss verschiedener Konzentrationen der Ligandenpeptide **I** (links) und **II** (rechts) sowie eine ausschnittsweise Darstellung der Cross-Peaks zwischen Protonen und Stickstoffen einiger aromatischer Seitenketten, wobei der Verschiebungshift von W23NE1-HE1 mit steigender Konzentration des Ligandenpeptids **I** (in Pfeilrichtung) besonders anschaulich ist.

Die Wechselwirkungen zwischen der EVH1-Domäne eines vollständig ¹⁵N-markierten VASP-Proteins und den Ligandenpeptiden **I** und **II** wurden unter Verwendung mehrdimensionaler NMR-spektroskopischer Methoden aufgeklärt. Hierzu wurde die EVH1-Domäne in Lösung mit unterschiedlichen Mengen an Ligandenpeptid titriert und durch Aufnahme von ¹⁵N-¹H-HSQC-Spektren jeweils untersucht. Die schrittweise Erhöhung der Ligandenkonzentration (10, 20, 50,100, 200, 500 und 1000 µM) führt im Fall einer positiven Bindungsinteraktion zu einer deutlich erkennbaren Verschiebung einiger Domänensignale, woraus sich später Werte für die Bindungskonstante errechnen lassen. Das Ergebnis der Untersuchungen mit den Ligandenpeptiden **I** und **II** ist den übereinandergelegten Ausschnitten der jeweiligen HSQC-Spektren in Figur 3 zu entnehmen. Da besonders aromatische Aminosäurereste der Domäne an der Ausbildung der hydrophoben Bindungswechselwirkungen beteiligt sind ("aromatische Wiege", vgl. Abschnitte 2.4/2.5), ist hier die Verschiebung der Signale deutlich zu erkennen. Dies gilt aber auch insbesondere für das NH-Signal des Tryptophans 23 (W23NE1-HE1), da dieses eine Wasserstoff-Brücke zu einer Carbonylgruppe des FPPPP-Ligandenmotivs ausbildet.

Der Vergleich der beiden NMR-Spektren in Figur 3 zeigt allerdings, dass der oben beschriebene Sachverhalt nur für Ligand **I** beobachtet wird (links). Da in Lösung ein ständiges Gleichgewicht [EVH1]-Ligand (gebunden) ↔ [EVH1] + Ligand (ungebunden) vorliegt, ergeben sich abhängig von der Austauschgeschwindigkeit des Liganden verschiedene NMR-Spektren (analog den Signalen für -OH und - NH im eindimensionalen Bereich). Für Ligand **I** werden zeitlich gemittelte Signale aus beiden Zuständen beobachtet, während für Ligand **II** beide Zustände separate Signalsätze auf der NMR-Zeitskala erzeugen. Dies ist besonders deutlich durch Vergleich der beiden NMR-Spektren anhand des W23NE1-HE1-Signals zu erkennen: Für Ligand **I** (links) ergibt sich bei der Titration eine allmähliche Verschiebung des Cross-Peaks, für Ligand **II** (rechts) beobachtet man zwei separate Signale, die sich nur wenig ändern, und keine "Zwischenzustände".

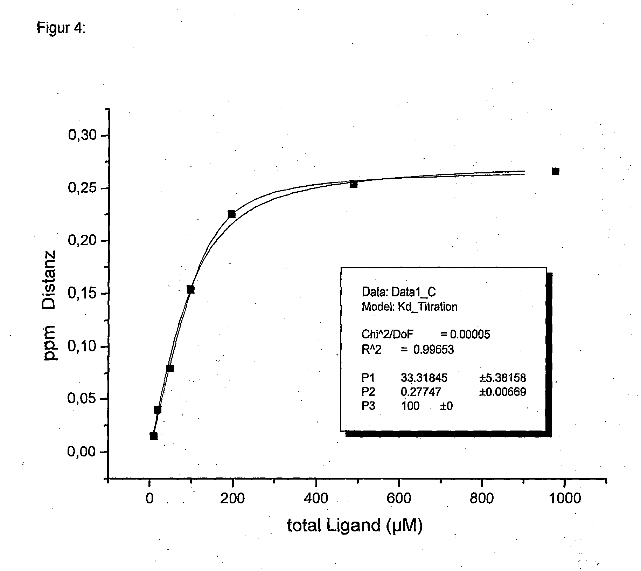

Korreliert man die Änderung der chemischen Verschiebung der Cross-Peaks mit der Bindungsstärke zwischen Domäne und Ligandenpeptid, lassen sich durch Auftragung der Verschiebungsveränderung Δδ gegen die Konzentration des Ligandenpeptids Werte für die Bindungskonstante errechnen. Dies wurde für **I** durchgeführt (Figur 4) und ergab eine etwas höhere Bindungsaffinität **(K_{D} = 20-35 µM)** als die native Peptidsequenz von ActA **(K**_{D} **= 45 µM): Für II** war dies aufgrund des oben besprochenen Sachverhaltes nicht möglich.

Figur 4 zeigt die Auftragung der Veränderung der chemischen Verschiebung Δδ gegen Konzentration des Ligandenpeptids **I** zur Ermittlung der Bindungskonstanten K_{D}.

In Ergänzung zur NMR-basierten Aufklärung von Wechselwirkungen zwischen Peptiden und Proteinen wird heutzutage als Standard auch die *Biacore*-Methode eingesetzt. Hierbei wird das Domänenprotein auf der Oberfläche eines Sensorchips immobilisiert, während eine Lösung des Ligandenpeptids über die Oberfläche strömt. Anhand der Messung der Oberflächenplasmonenresonanz, die zu einer Änderung des Brechungsindex führt, kann in Echtzeit die Bindungskinetik verfolgt werden, wodurch man ebenfalls Konstanten der Assoziation und Dissoziation bestimmen kann. Besonders für Fälle, in denen die NMR-Spektroskopie keine Ermittlung der Bindungskonstanten zulässt wie für das Ligandenpeptid **II,** ist dies die Methode der Wahl. Erste, noch zu verifizierende Messungen ergaben folgende Bindungskonstanten: **K_{D} = 29,3 - 33,3 µM für** Ligandenpeptid **I** und K_{D} = **3,7 -12,5 µM** für Ligändenpeptid **II.** (entsprechende native Peptide: K_{D} = 55.1 **µM** bzw. K_{D} = **13.7 µM)**.

Diese Ergebnisse sind insofern besonders bemerkenswert, da es sich hierbei um bisher für die EVH1-Domäne nicht beschriebene Experimente handelt und diese zu einer Reihe wichtiger Erkenntnisse führen, die wie folgt zusammengefasst werden können und eine Grundlage für zukünftige Forschungsarbeiten bilden:
1) Der Einbau des synthetisch hergestellten Tricyclus 85 in verschiedene Testpeptide unter den Standard-Kupplungsbedingungen (DIC/HOBt) der Festphasen-Peptidsynthese verlief ohne Probleme.
2) Die bisher getroffenen Voraussagen, dass sich die mittleren beiden Prolin-Positionen des F/WPPPP-Kemmotivs ersetzen lassen sollten, konnten bestätigt werden.
3) Die Vorhersage der Reaktivkonformation bei Bindung der PPII-Helix an den EVH1-Rezeptor wird durch die gute Bindungsaffinität des Ligandenpeptids **I** bestätigt, bzw. es stimmen die biologisch benötigte Konformation und die tatsächliche Konformation des Tricyclus 85 gut überein.
4) Der erwartete günstige Entropie-Effekt durch lokale, konformationelle Vorfixierung des Liganden scheint sich durch die festgestellte höhere Bindungsaffinität im Vergleich zur nativen Peptidsequenz zu bestätigen. Andererseits verhindert die geometrische Fixierung aber auch nicht die Annäherung von Ligand und Domänenprotein und damit die Bindungsbildung

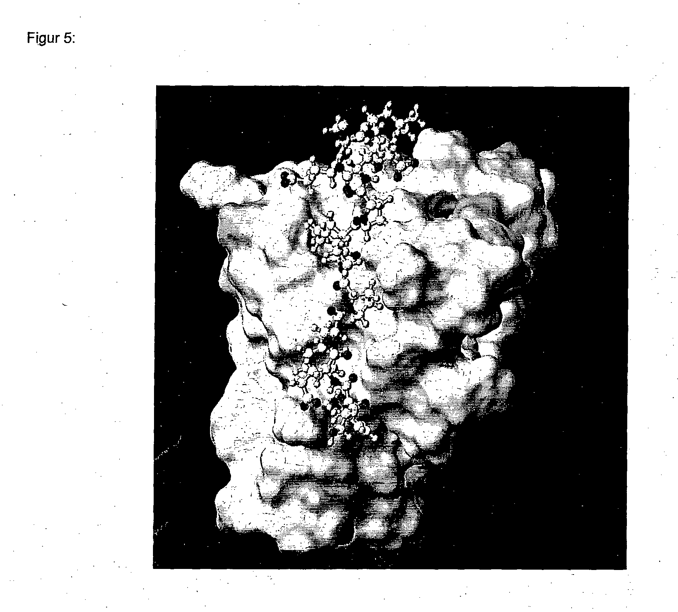

Figur 5 zeigt eine Abbildung der VASP-EVH1-Domäne mit ₃₃₂SFEFP*pp*PTEDEL₃₄₄Peptidligand (Molecular-Modeling-Abbildung) Gut zu erkennen ist die EVHI-Domäne als "Graben" auf der Oberfläche des VASP-Proteins, der das Ligandenpeptid aufnimmt.

### Biologische Ergebnisse und Ausblick

Mit Hilfe der synthetischen Verbindung **85** wurden beispielhaft Ligandenpeptide mit teilweise oder vollständig ersetzten Prolinsequenzen synthetisiert und auf ihre Wechselwirkung mit der EVH1-Domäne untersucht (*pp* = Baustein **85).** Dabei zeigte sich, dass durch den Einbau des Mimetikums **85** die Bin-dungsaffinität der Ligandenpeptide zur Domäne gesteigert werden konnte. Der Austausch aller Proline durch 2 miteinander verknüpfte Bausteine **85** führte zu Ligandenpeptiden mit einer Affinität vergleichbar zu der des nativen Peptides. Dies ist in der nachfolgenden Tabelle 1 zusammengefasst, die die Bindungsaffinitäten der Ligandenpeptide sowie der zu Grunde liegenden nativen Peptidliganden auflistet. Die Bindungsaffinitäten wurden mittels NMR-Titration, der kinetischen *Biacore*-Methode beziehungsweise mittels Fluoreszenztitration bestimmt. Die Ergebnisse sind in Tabelle 1 zusammengefaßt.

**Tabelle 1: Vergleich der gemessenen Bindungsaffinitäten (Wechselwirkung der Ligandenpeptide mit der VASP-EVH1-Domäne in Lösung).**

| **Ligandenpeptid** | **Bindungsaffinität (NMR)** | **Bindungsaffinität (*Biacore*)** | **Floreszenz-Titration** |
|---|---|---|---|
| SFEFPPPPTEDEL | 45 µM | 551 µM | 37 ± 10 µM |
| SFEFP*pp*PTEDEL(I) | 20-35 µM | 29.3-33 µM | 47 ± 14 µM |
| SFEF*pp*PPTEDEL | - | - | 82 ± 30 µM |
| SFEFPP*pp*TEDEL | - | - | 26 ± 5 µM |
| SFEF*pppp*TEDEL | - | - | 45 ± 15 µM |
| SFE**W**PPPPTEDEL | *nicht bestimmbar* | 13.7 µM | - |
| SFE**W**P*pp*PTEDEL(**II**) | *nicht bestimmbar* | 3.7-12.5 µM | - |

Der Austausch aller Proline durch 2 miteinander verknüpfte Bausteine **85** führte zu Ligandenpeptiden mit einer Affinität vergleichbar zu der des nativen Peptides Damit ist es erstmals gelungen, Liganden-peptide ohne Prolin-reiche Sequenzen, die durch PRM-bindende Domänen gebunden werden, zu entwickeln. Da das generelle Konzept in Hinblick auf die Synthese und den biologischen Einsatz der Zielstruktur **85** erfolgreich umgesetzt werden konnte, eröffnen sich auf dieser vielversprechenden Grundlage zahlreiche weitere Möglichkeiten, mit Hilfe kleiner synthetischer Moleküle gezielt relevante Proteindomänen zu adressieren, die polyprolinhaltige Liganden binden. Neben der Variation bestehender Molekülstrukturen wäre auch eine Ausweitung des Konzepts auf die Synthese von Liganden für andere Domänen (Mena-EVH1, WW, SH3 u.a.), der Ersatz anderer als der zentralen beiden Prolineinheiten des FPPPP-Motivs oder der Ersatz von PPP- oder sogar PPPP-Motiven durch geeignete, organischsynthetisch erzeugte Strukturen denkbar.

### Experimenteller Teil

Alle Reaktionen mit luft- oder wasserempfindlichen Komponenten wurden unter absoluten Bedingungen durchgeführt. Dazu wurden die verwendeten Glasgeräte im Ölpumpenvakuum (Enddruck von 0.1-0.5 mbar) mit einer Bunsenbrennerflamme ausgeheizt und nach dem Abkühlen mit Argon geflutet, wobei eine Vakuum-Argon-Doppelhahnglasapparatur zum Einsatz kam. Spritzen und Kanülen zum Transfer von Reagenzien und Lösungsmitteln wurden in einem Ofen bei 85 °C getrocknet und vor Gebrauch mehrfach mit Argon gespült. Feste Reagenzien wurden im Argon-Gegenstrom in den Reaktionskolben eingefüllt. Lösungsmittel wurden an einem Rotationsverdampfer bei einer Wasserbadtemperatur von 40 °C und einem Druck von 10-1013 mbar entfernt. Die weitere Trocknung der Substanzen erfolgte im Ölpumpenvakuum (0.1-0.5 mbar) i.d.R. bei Raumtemperatur. Die verwendeten Chemikalien wurden von gängigen Firmen wie *Merck, Sigma-Aldrich, Fluka, Acros, Lancaster* und *Strem* kommerziell bezogen und meist ohne weitere Reinigung eingesetzt. Die Konzentration metallorganischer Reagenzien wurde nach *Paquette et al.* durch Titration gegen Menthol mit Phenanthrolin als Indikator bestimmt Alle Lösungsmittel, die für Extraktions- und Reinigungsvorgänge zum Einsatz kamen, wurden vor Gebrauch destilliert. Wasserfreie Lösungsmittel wurden nach einschlägigen Verfahren erhalten.

Die Aufreinigung der Substanzen erfolgte durch Flashsäulenchromatographie an Kieselgel 60 (0.040-0.063 mm) der Firma Merck und wurde unter leichtem Überdruck (0.3-0.5 bar) durchgeführt. Analytische Dünnschichtchromatographie (DC) zur Reaktionskontrolle wurde mit Kieselgelplatten der Firma Merck durchgeführt (Kieselgel 60F₂₅₄). Die Auswertung der Chromatogramme erfolgte im UV-Licht (λ = 254 nm) und durch Anfärben mit einer KMnO₄-Lösung (3 g KMnO₄, 20 g K₂CO₃, 5 ml 5%ige NaOH, 300 ml H₂O) und anschließendes Erhitzen mit einem Heißluftfön. Analytische HochdruckFlüssigkeitschromatographie (HPLC) wurde mit einem *Knauer*-System (HPLC Pump K-1001, DAD K-2700 Wellchrom, Lamp K-2701 Wellchrom, Solvent Organizer K-1500), mit einem *Merck-Hitachi-*System (L-4000 A UV-Detektor, D-6200A Intelligent Pump, Differential Refractometer Ri 71), einem *Merck-Hitachi*-System (L-7250A Intelligent Pump, L-7455 UV-Detektor) oder einem *Agilent* 1100 HPLC-MS-System durchgeführt. Weitere Angaben befinden sich bei den aufgeführten Messungen.

¹H- und ¹³C-NMR-Spektren wurden an den Geräten AC 250, DPX 300 und DRX 500 der Firma *Bruker* bei Raumtemperatur aufgenommen. Die chemischen Verschiebungen sind relativ zum Restprotonen-gehalt bzw. zur Resonanz des Lösungsmittels als internem Standard angegeben. Die Zuordnung der Signale erfolgte durch die Aufnahme geeigneter 2D-Experimente (DEPT, APT, H-H-COSY, HMQC, HMBC, NOE) und durch Vergleich mit analogen Verbindungen. Zur Auswertung der NMR-Spektren wurde das Programm *Mestre-C* mit den dort enthaltenen Analytikfunktionen verwendet. Die angegebene Nummerierung der Moleküle zur Zuordnung der Signale ist weitgehend identisch mit IUPAC, kann aber aus Gründen der Übersicht teilweise davon abweichen. Signal-Multiplizitäten werden wie folgt angegeben: (s = Singulett, d = Dublett, t = Triplett, q = Quartett, quint = Quintett, br = breites Signal, m = Multiplett). Wegen der gehinderten Rotation der Carbamat-Schutzgruppen bei Raumtemperatur wurden viele Verbindungen als Rotamerengemisch beobachtet und werden als solche beschrieben (breite Peaks bzw. doppelter Signalsatz im ¹H- und ¹³C-Spektrum).

IR-Spektren wurden an einem FT-IR Paragon 1000 der Firma *Perkin-Elmer* bei Raumtemperatur als ATR (Attenuated Total Reflectance) auf einem ZnSe-Kristall aufgenommen, auf den die Proben als Lösung aufgebracht wurden. Die Absorptionsbanden werden in Wellenzahlen (*ṽ* [cm⁻¹] angegeben und folgendermaßen nach ihrer relativen Intensität charakterisiert: vs (sehr stark), s (stark), m (mittel), w (schwach), br (breites Signal).

Massen-Bestimmungen wurden an den Geräten MAT Incos 50 Galaxy System (EI) und MAT 900 (ESI, HRMS) der Firma Finnigan durchgeführt. Die Einlassmethode (Direkteinlass (DIP) oder GC-MS), die Ionisierungsart (EI oder ESI) sowie die Ionisierungsenergie in [eV] sind jeweils in Klammern angegeben. Die aufgeführten Signale beziehen sich auf das Verhältnis m/z und sind in ihrer Intensität relativ zum Basispeak (100%) angegeben.

Drehwerte wurden an einem Polarimeter 343 plus der Firma *Perkin Elmer* gemessen. Die Konzentration von [α] ist in der Einheit [g/100 ml] angegeben, die Messtemperatur, die Wellenlänge (i. d. R. 589 nm, z.T. zusätzlich 546, 405, 365 und 334 nm) und das Lösungsmittel sind jeweils vermerkt.

Elementaranalysen wurden mit einem Elementar Vario EL durchgeführt, wobei die massenprozentuale Zusammensetzung nach den Elementen C, H und N aufgeschlüsselt werden konnte. Schmelzpunkte wurden mit Hilfe eines B-545 der Firma Büchi bestimmt und sind nicht korrigiert.

Die Benennung der Verbindungen erfolgte unter Zuhilfenahme des Autonom-Programms der elektronischen Beilstein-Datenbank nach den Richtlinien der IUPAC. Geringfügige Abweichungen von dieser Nomenklatur ergeben sich durch Anlehnung der Benennung an literaturbekannte Substrate oder die stärkere Berücksichtigung von funktionellen Gruppen als Einheit (z.B. Schutzgruppen) und daraus folgend veränderte Prioritäten. Die Bestimmung der Stereochemie erfolgte nach den Regeln von Cahn-Ingold-Prelog. (Lit: R. S. Cahn, C. K. Ingold, V. Prelog, Angew. Chemie 1966, 78,413)

### (2S)-5-Oxo-pyrrolidin-2-carbonsäure-ethylester (203)

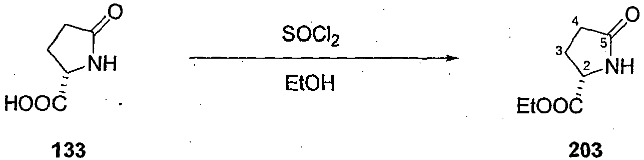

Eine Suspension von 30.0 g *L*-Pyraglutaminsäure **133** (232 mmol) in 100 ml Ethanol (abs.) wurde bei 0 °C langsam mit 20 ml SOCl₂ (274 mmol, 1.2 Äq.) versetzt und anschließend 15 h lang gerührt, wobei der Ansatz auf Raumtemperatur auftaute und eine klare Lösung entstand. Zur Aufarbeitung wurden alle flüchtigen Bestandteile im Vakuum vollständig entfernt, dann der verbleibende Rückstand in 500 ml Essigester aufgenommen und nacheinander über K₂CO₃ und MgSO₄ gerührt, wobei jeweils vom Trockenmittel abgesaugt wurde. Nach Filtration über wenig Silicagel und Entfernen des Lösungsmittels im Vakuum erhielt man 36.0 g (230 mmol, 99 %) des Esters **203** als leicht gelbliches, viskoses Öl.
**M(C₇H₁₁NO₃) = 157.1672.**
R_{f} = 0.47 (SiO₂, CH₂Cl₂/MeOH 9:1).
**¹H-NMR** (300 MHz, CDCl₃): δ (ppm) = 7.04 (br, 1H, NH), 4.10-4.22 (m, 3H, H-2, CH_{2,Ester}), 2.22-2.48 (m, 3H, H-3_{α}, H-4), 2.07-2.22 (m, 1H, H-3_{β}), 1.22 (t, 3H, J = 7.1 Hz, CH_{3,Ester}).
**¹³C-NMR** (75 MHz, CDCl₃): δ (ppm) = 178.37 (C=O_{Lactam}), 172.10 (C=O_{Ester}), 61.58 (CH_{2,Ester}), 55.59 (CH), 29.33 (CH₂, C-4), 24.77 (CH₂, C-3), 14.1 (CH_{3,Ester}).

### (5S)-5-Hydroxymethyl-pyrrolidin-2-on (134)

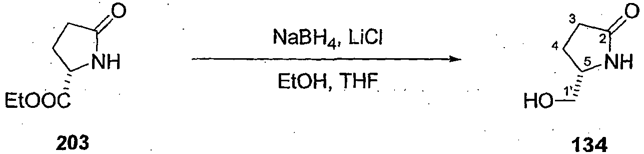

Eine Lösung von 36.0 g (230 mmol) des Esters 203 in 400 ml THF wurde mit 16.0 g NaBH₄ (460 mmol, 2 Äq.) und 19.5 g LiCl (460 mmol, 2 Äq.) versetzt. Anschließend wurden 100 ml EtOH hinzugefügt und die Mischung über Nacht gerührt Die Vollständigkeit der Reaktion wurde per DC (CH₂Cl₂/MeOH 9:1) überprüft (ggf. wurden einige Milliliter Wasser zugegeben, wodurch sich die Reaktion deutlich beschleunigte). Nach beendeter Reaktion wurden 500 ml 5%ige Zitronensäure zugegeben (Wasserstoffentwick-lung!), worauf eine klare Lösung entstand. Nach vollständigem Einengen wurde der weiße, schleimige Rückstand in einem Gemisch aus Essigester/Methanol 3:1 aufgenommen und über Celite filtriert. Ein ggf. verbleibender Niederschlag wurde durch Einengen, Aufnehmen des Rückstandes in 500 ml CH₂Cl₂/MeOH (9:1) und anschließende Filtration über Kieselgel entfernt.

Nach Einengen im Vakuum wurde das Rohprodukt bei 100 °C einige Stunden im ÖPV getrocknet, wodurch man 25.6 g Pyroglutaminol **134** (169 mmol, 73 %) als farblosen glasigen Feststoff erhielt.
**M (C₅H₉NO₂) = 115.1305.**
**R_{f}** = 0.18 (SiO₂, CH₂Cl₂/MeOH 9:1).
**¹H-NMR** (250 MHz, DMSO-d⁶): δ (ppm) = 7.66 (br, 1 H, NH), 5.00 (t, 1H, J = 5.4 Hz, OH), 3.50 (m, 1H, H-5), 3.28 (t, 2H, J = 5.1 Hz, H-1'), 1.90-2.18 (m, 3H, CH₂), 1.69 (m, 1 H, CH₂).
**¹³C-NMR** (62.5 MHz, DMSO-d⁶): δ (ppm) = 176.64 (C=O), 64.40 (CH₂, C-1'), 55.16 (CH, C-5), 29.54 (CH₂, C-4), 22.73 (CH₂, C-3).

### (5S)-5-(tert-Butyl-diphenyl-silanyloxymethyl)-pyrrolidin-2-on (135)

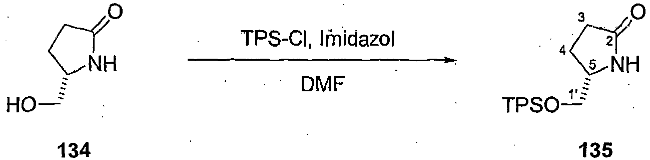

8.40 g Pyroglutaminol 134 (73 mmol) und 9.80 g Imidazol (144 mmol, 2 Äq.) wurden in 100 ml DMF (abs.) gelöst und im Eisbad gekühlt. Dann wurden 18.8 ml TPS-Cl (73 mmol) hinzugefügt und die Mischung über Nacht bei RT gerührt. Zur Aufarbeitung wurde der Ansatz in einen Scheidetrichter über-führt, mit 500 ml MTBE und 100 ml Wasser verdünnt die Phasen getrennt und die organische Phase über MgSO₄ getrocknet. Nach Säulenchromatographie (EE/CH 2:1) erhielt man 22.5 g Silylether 135 (64 mmol, 87 %) als farbloses Öl, das allmählich zu feinen weißen Kristallen erstarrte.
**M (C₂₁H₂₇NO₂Si)** = **353.5301.**
**R_{f} =** 0.19 (SiO₂, EE/CH 2:1).
**Smp.:** 75 °C, Lit: 77 - 78 °C.
**¹H-NMR** (300 MHz, CDCl₃): δ (ppm) = 7.61-7.64 (m, 4H, PhH), 7.37-7.42 (m, 6H, PhH), 6.19 (br, 1 H, NH), 3.78 (ddd, 1H, J = 14.8 Hz, J = 7.6 Hz, J = 4.8 Hz, H-5), 3.60 (dd, 1H, J = 10.3 Hz, J = 4.2 Hz, H-1'_{α}), 3.51 (dd, 1H, J = 10.3 Hz, J = 7.2 Hz, H-1'_{β}), 2.27-2.33 (m, 2H, H-3), 2.04-2.20 (dt 1H, J = 12.9 Hz, J = 7.8 Hz, H-4_{α}), 1.72 (dddd, 1H, J = 13.0 Hz, J = 9.1 Hz, J = 7.4 Hz, J = 5.3 Hz, H-4_{β}), 1.03 (s, 9H, Si*t*Bu).
**¹³C-NMR** (75 MHz, CDCl₃): δ (ppm) = 177.96 (C=O), 135.44, 135.40 (CHₐᵣ), 132.90, 132.87 (C_{ar,q}), 129.81 (CHₐᵣ), 127.75 (CHₐᵣ), 67.29 (CH₂, C-1'), 55.57 (CH, C-5), 29.70 (CH₂, C-3), 26.71 (CH₃, Si*t*Bu), 22.73 (CH₂, C-4), 19.09 (Cq, SitBu).
**IR** (ATR) *ṽ* (cm⁻¹) = 3211 (br), 3069 (w), 2928 (m), 2855 (m), 1697 (vs), 1587 (w), 1471 (m), 1461 (m), 1426 (s), 1261 (w), 1111 (vs), 1029 (s), 867 (m), 822 (s), 740 (s), 701 (vs).
**MS** (DIP-EI, 70 eV): m/z (%) = 352 ([M⁺-H], <1), 296 (100), 252 (5), 218 (72),199 (19),181 (17),162 (11), 135 (12),105 (14), 84 (12), 77(7), 55 (7).
**[α]_{D}²⁰ =** +15.0° (c =1.245, CHCl₃). Lit: +15.4° (c = 0.825, CHCl₃).

### (5S)-1-(tert-Butoxycarbonyl)-5-(tert-butyl-diphenyl-silanyloxymethyl)-pyrrolidin-2-on (136)

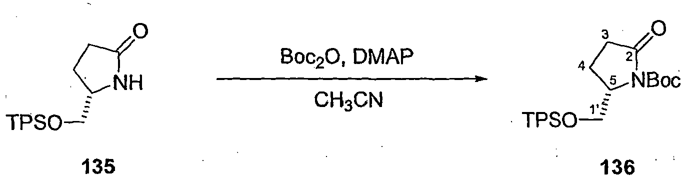

7.47 g **135** (21.1 mmol) wurden in 100 ml absolutem Acetonitril gelöst, auf 0°C gekühlt und mit 100 mg DMAP (0.81 mmol, 3.8 mol-%) und 5.0 ml Boc₂O (23.4 mmol, 1.1 Äq.) versetzt. Nach 15 h Rühren bei RT wurde die gelbe-orange Lösung vollständig eingeengt, in einer Mischung aus Cyclohexan/Ethylacetat (3:1) aufgenommen und über eine kurze Silicagelsäule filtriert. Nach erneutem Einengen erhielt man einen gelblichen, festen Rückstand, der aus Et₂O/Pentan umkristallisiert wurde, wobei 8.57 g 136 (18.9 mmol, 89%) als farblose Kristalle entstanden.
**M (C₂₆H₃₅NO₄Si) = 453.6459**
**R_{f}** = 0.30 (SiO₂, EE/CH 1:3).
**Smp.**: 106 - 108 °C. Lit: 110 °C.
**¹H-NMR** (500 MHz, CDCl₃): (Rotamerengemisch) δ (ppm) = 7.58-7.63 (m, 4H, Hₐᵣ), 7.34-7.42 (m, 6H, Hₐᵣ), 4.19 (tdd, 1 H, J = 8.2 Hz, J = 4.1 Hz, J = 2.2 Hz, H-5), 3.87 (dd, 1H, J = 10.5 Hz, J = 4.2 Hz, H-1'_{α}), 3.68 (dd, 1H, J = 10.5 Hz, J = 2.4 Hz, H-1'_{β}), 2.77 (td, 1 H, J = 17.6 Hz, J = 10.4 Hz, K-3_{α}), 241 (ddd, 1H, J = 17.6 Hz, J = 9.2 Hz, J = 2.8 Hz, H-3_{β}), 2.05-2.18 (m, 2H, H-4), 1.41 (s, 9H, O*t*Bu), 1.02 (s, 9H, Si*t*Bu).
**¹³C-NMR** (125 MHz, CDCl₃): (Rotamerengemisch) δ (ppm) = 174.93 (C=O_{Lactam}), 149.72 (C=O_{Boc}), 135.48,135.44 (CHₐᵣ), 132.99, 132.58 (C_{q,ar}), 129.82, 129.81 (CHₐᵣ), 127.81, 127.77 (CHₐᵣ), 82.62 (Cq, O*t*Bu), 64.90 (CH₂, C-1'), 58.74 (CH, C-5), 32.26 (CH₂, C-3), 27.94 (CH₃, O*t*Bu), 26.74 (CH₃, Si*t*Bu), 21.05 (CH₂ C-4), 19.12 (C_{q}, Si*t*Bu).
**IR** (ATR) *ṽ* (cm⁻¹) = 3069 (w), 2957 (m), 2929 (m), 2856 (m), 1785 (s), 1747 (s), 1708 (s), 1588 (w), 1471 (m), 1426 (m), 1365 (s), 1308 (vs), 1286 (s), 1254 (s), 1150 (vs), 1106 (vs), 1076 (s), 1031 (s), 997 (m), 898 (m), 860 (m), 846 (m), 821 (s), 742 (vs), 700 (vs), 615 (s).
**MS** (DIP-EI, 70 eV): m/z (%) = 438 (<1, [M⁺-CH₃]), 401 (<1), 380 (1), 340 (15), 296 (33), 218 (100),199 (12),181 (14), 162 (8), 135 (15), 105 (12), 84 (11), 57 (25).
**[α]_{D}²⁰** = -37.2° (c = 0.995, CHCl₃). Lit.: -38.4° (c = 1.30, CHCl₃).

### (2R/S,5S)-1-(tert-Butoxycarbonyl)-5-(tert-butyl-diphenyl-silanyloxymethyl)-2-hydroxy-pyrrolidin (204)

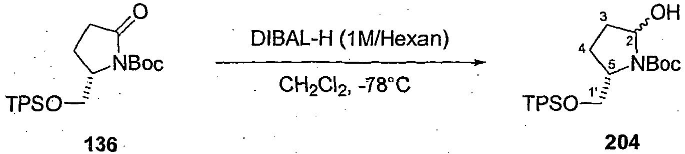

In einem ausgeheizten Dreihalskolben mit Tropftrichter wurde eine Lösung von 3.00 g 136 (6.61 mmol) in 15 ml CH₂Cl₂ (abs.) auf -78 °C gekühlt und tropfenweise mit 12.5 ml DIBAL-H (12.5 mmol, 2.2 Äq., 1 M/Hexan) versetzt. Nach zwei Stunden Rühren bei -78 °C wurde die Reaktion durch Zugabe von 1 ml Isopropanol abgebrochen, mit 20 ml K-Na-Tartrat-Lösung (1.77M) versetzt und langsam auf Raumtemperatur aufgetaut. Nach Trennen der beiden Phasen wurde die wässrige Phase zusätzlich mit 3x 50 ml MTBE extrahiert und die vereinigten organischen Phasen über MgSO₄ getrocknet. Nach Filtration über eine kurze Kieselgelsäule (mit EE/CH 1:3 eluiert) wurde das Filtrat eingeengt, worauf man 204 als leicht gelbliches Öl erhielt, das ohne Aufreinigung weiter umgesetzt wurde. Ausbeute: 2.92 g (6.41 mmol, 97 %).
**M (C₂₆H₃₇NO₄Si) = 455.6618.**
**R_{f} =** 0.37 (SiO₂, EE/CH 1:3).
**¹H-NMR** (250 MHz, CDCl₃): (Diastereomeren-/Rotamerengemisch) δ (ppm) = 7.57-7.70 (m, 4H, Hₐᵣ), 7.30-7.45 (m, 6H, Hₐᵣ), 5.46 (br, 1H, H-2), 3.20-4.05 (m, 4H, H-5, H-1', OH), 1.40-2.50 (m, 4H, H-3, H-4), 1.49, 1.32 (2s, 9H, O*t*Bu), 1.04 (s, 9H, Si*t*Bu).
**¹³C-NMR** (62.5 MHz, CDCl₃): (Diastereomeren-/Rotamerengemisch) δ (ppm) = 154.85, 154.55, 153.17 (C=O_{Boc}), 135.43 (CHₐᵣ), 133.30, 133.16, 132.94 (C_{q,ar}), 129.62 (CHₐᵣ), 127.64 (CHₐᵣ), 82.78, 82.44, 82.18 (CH, C-2), 80.19, 80.05 (Cq, O*t*Bu), 64.51, 64.22, 64.07, 63.31 (CH₂ C-1'), 58.71, 58.40 (CH, C-5), 33.00, 31.19 (CH₂, C-3/C-4), 28.24 (CH₃, O*t*Bu), 26.78 (CH₃, Si*t*Bu), 25.67, 25.31, 24.03 (CH₂, C-3/C-4), 19.17, 18.81 (C_{q}, Si*t*Bu).
**IR** (ATR) *ṽ* (cm⁻¹) = 3442 (br), 3064 (w), 2955 (m), 2928 (m), 2854 (m), 1679 (vs), 1587 (w), 1471 (m), 1425 (s), 1389 (vs), 1364 (vs), 1255 (m), 1163 (vs), 1104 (vs), 1024 (s), 996 (s), 965 (s), 902 (s), 851 (s), 822 (s), 806 (s), 775 (s), 735 (vs), 698 (vs).

### (2R/S,5S)-1-(tert-Butoxycarbonyl)-5-(tert-butyl-diphenyl-silanyloxymethyl)-2-methoxy-pyrrolidin (59)

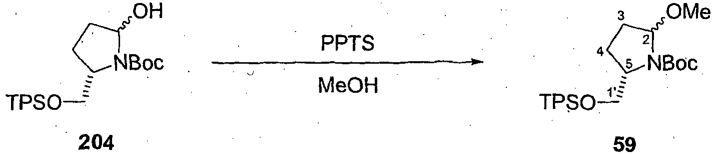

Eine Lösung von 2.92 g **204** (6.41 mmol) in 10 ml MeOH (abs.) wurde mit 100 mg PPTS (0.40 mmol, 6.2 mol-%) versetzt und über Nacht bei RT gerührt. Nachdem die Vollständigkeit der Reaktion per DC (EE/CH 1:7) festgestellt war, wurde die Lösung eingeengt, der verbleibende Rückstand in 25 ml Essigester aufgenommen und über wenig Kieselgel filtriert. Das Filtrat wurde eingeengt und säulenchromatographisch an Kieselgel (EE/CH 1:7) weiter aufgereinigt, worauf man 2.98 g **59** (6.34 mmol, 99 %) als farbloses Öl erhielt.
**M (C₂₇H₃₉NO₄Si) = 469.6884.**
**R_{f} =** 0.29/0.33 (SiO₂, EE/CH 1:7, Diastereomere).
**¹H-NMR** (300 MHz, CDCl₃): (Diastereomeren-/Rotamerengemisch) δ (ppm) = 7.58-7.70 (m, 4H, Hₐᵣ), 7.29-7.45 (m, 6H, Hₐᵣ), 5.21 (br, 0.7H, H-2), 5.05 (d, 0.2H, J = 4.3 Hz, H-2), 4.93 (d, 0.1 H, J = 4.4 Hz, H-2), 3.42-4.04 (m, 3H, H-5 und H-1'), 3.37 (s, 0.4H, OCH₃), 3.32 (s, 0.3H, OCH₃), 3.25 (s, 2.3H, OCH₃), 2.00-2.30 (m, 2H, H-3), 1.65-2.00 (m, 2H, H-4), 1.46, 1.32, 1.28 (s/br/s, zus. 9H, O*t*Bu), 1.04 (s, 9H, Si*t*Bu).
**¹³C-NMR** (75 MHz, CDCl₃): (Diastereomeren-/Rotamerengemisch) δ (ppm) = 155.14,154.15,153.73 (C=O), 135.50 (CHₐᵣ), 133.79, 133.67, 133.48, 133.31 (C_{q,ar}), 129.64, 129.50, 127.57 (CHₐᵣ), 90.17, 89.77,89.61 (CH, C-2), 79.85, 79.59 (C_{q,Boc}), 66.54 (br, CH₂, C-1'), 63.88, 63.76 (CH₂, C-1'), 59.15, 58.18 (CH, C-5), 56.35, 55.82, 55.11 (CH₃, OCH₃), 31.59 (br, CH₂, C-3), 28.29, 27.38 (CH₃, O*t*Bu), 26.80 (CH₃, Si*t*Bu), 25.68, 25.43 (CH₂, C-4),19.29 (C_{q}, Si*t*Bu).
**IR** (ATR) *ṽ* (cm⁻¹) = 3069 (w), 3044 (w), 2955 (m), 2927 (m), 2855 (m), 2342 (w), 1959 (w), 1891 (w), 1809 (w), 1696 (vs), 1588 (w), 1471 (m), 1452 (m), 1426 (s), 1364 (vs), 1320 (m), 1255 (m), 1164 (s), 1104 (vs), 1028 (m), 996 (m), 9191 (m), 885 (m), 849 (m), 822 (s), 805 (m), 774 (m), 738 (s), 700 (vs).
**MS** (DIP-EI, 70 eV): m/z (%) = 454 ([M⁺-CH₃], <1), 410 (<1), 396 (<1), 324 (21), 280 (100), 260 (6), 248 (13), 234 (19), 199 (19), 184 (13), 135 (24), 114 (21), 100 (20), 68 (35), 57 (85).

### (5S)-1-(tert-Butoxycarbonyl)-5-(tert-butyl-diphenyl-silanyloxymethyl)-2-oxo-3,4-dihydro-pyrrol (18)

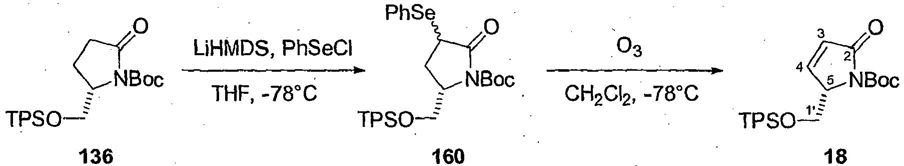

Eine Lösung von 10.0 g **136** (22.0 mmol) in 50 ml THF wurde auf -78 °C gekühlt, tropfenweise mit 24.0 ml LiHMDS (24.0 mmol, 1M/THF, 1.1 Äq.) versetzt und 1 h gerührt. Anschließend wurde eine Lösung von 7.37 g PhSeCl (38.5 mmol, 1.75 Äq.) in 30 ml THF zugetropft und 2h gerührt. Nach Zugabe von 100 ml ges. NH₄Cl-Lsg. wurde der Ansatz auf RT gebracht, mit 300 ml MTBE verdünnt und mit 50 ml ges. NH₄Cl-Lsg und 50 ml ges. NaCl-Lsg. gewaschen. Die organische Phase wurde über MgSO₄ getrocknet, vom Trockenmittel abfiltriert und eingeengt. Säulenchromatographie an Kieselgel (EE/CH 1:8) lieferte 10.5 g Selenylether **160** (17.3 mmol, 78 %) als gelbliches Öl.

Dieser wurde in 250 ml CH₂Cl₂ aufgenommen, auf -78 °C gekühlt und mit Ozon versetzt. Bei beginnender Blaufärbung wurde die Reaktion abgebrochen, das überschüssige Ozon durch Spülen mit Sauerstoff vertrieben und die Reaktion über Nacht auf RT aufgetaut. Nach vollständigem Einengen wurde der Rückstand säulenchromatographisch an Kieselgel (EE/CH 1:3) aufgereinigt. Man erhielt 5.24 g (11.6 mmol, 67 %) des Enons 18 als gelbliches Öl, das allmählich zum Feststoff erstarrte.
**M (C₂₆H₃₃NO₄Si) = 451.6301.**
**R_{f} =** 0.23 (EE/CH 1:3).
**Smp.:** 94 - 95°C. Lit.: 96 °C.
**¹H-NMR** (250 MHz, CDCl₃): (Rotamerengemisch) δ (ppm) = 7.54-7.65 (m, 4H, Hₐᵣ), 7.30-7.47 (m, 6H, Hₐᵣ), 7.25 (dd, 1H, J = 6.1 Hz, 2.1 Hz, H-3), 6.15 (dd, J = 6.1 Hz, 1.7 Hz, 1H, H-4), 4.63 (ddd, 1H, J = 8.0 Hz, J = 3.5 Hz, J = 1.8 Hz, H-5), 4.10 (dd, 1H, J = 9.7 Hz, 3.5 Hz, H-1'_{α}), 3.80 (dd, 1H, J = 9.8 Hz, 6.5 Hz, H-1'_{β}), 1.41 (s, 9H, O-*t*Bu), 1.01 (s, 9H, Si-*t*Bu).
**¹³C-NMR** (62.5 MHz, CDCl₃): (Rotamerengemisch) δ (ppm) = 169.46 (C=O, C-2), 1929, 149.21 (C=O, Boc), 135.45 (CHₐᵣ), 132.90 (C_{q,ar}), 132.64 (CHₐᵣ), 129.60 (CH_{olef}, C-4), 127.81 (CHₐᵣ), 127.34 (CH_{olef}, C-3), 82.88 (C_{q,Boc}), 63.44 (CH, C-2), 62.98 (CH₂, C-1'), 28.11 (CH₃, O*t*Bu), 26.70 (CH₃, Si*t*Bu), 19.24 (Cq, Si*t*Bu).
**IR** (ATR) *ṽ* (cm⁻¹) = 3069 (w), 2930 (m), 2856 (m), 1780 (vs), 1742 (vs), 1709 (vs), 1588 (w), 1472 (w), 1427 (w), 1353 (s), 1319 (vs), 1255 (m), 1160 (s), 1112 (vs), 1044 (m), 981 (w), 821 (m), 740 (m), 702 (vs), 616 (m), 609 (m).
**MS** (DIP-EI, 70 eV): m/z (%) = 378 ([M⁺-O*t*Bu], 2), 338 (30), 294 (14), 263 (5), 216 (100),199 (14),181 (11), 135 (28),105 (10), 91 (5), 77 (4), 57 (29).
**[α]_{D}²⁰** = -121.1° (c = 0.63, CHCl₃). Lit.: -124° (c = 0.23, CHCl₃).

### 1-(tert-Butoxycarbonyl)-5-(tert-butyl-diphenyl-silanyloxymethyl)-2-hydroxy-pyrrol (161) (Nebenprodukt der Selenoxid-Eiminierung)

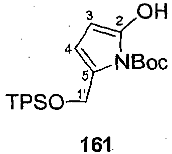

**R_{f} =** 0.21 (SiO₂, EE/CH 1:3).
**¹H-NMR** (250 MHz, CDCl₃): 7.56-7.62 (m, 4H, Hₐᵣ), 7.31-7.44 (m, 6H, Hₐᵣ), 6.88 (d, 1 H, J = 6.1 Hz), 6.11 (d, 1H, J = 6.1 Hz), 4.54 (br, 1H, OH), 4.21 (d, 1H, J = 10.2 Hz, H-1'_{α}), 3.84 (d, 1H, J = 10.2 Hz, H-1'_{β}), 1.51 (s, 9H, O*t*Bu), 1.00 (s, 9H, Si*t*Bu).
¹**³C-NMR** (62.5 MHz, CDCl₃):166.96 (C_{q}), 149.93 (C_{q}),149.30 (CH_{olef.}), 135.22 (CHₐᵣ), 135.16 (CHₐᵣ), 132.35 (C_{q,ar}), 129.69 (CHₐᵣ), 129.65 (CHₐᵣ), 127.53 (CHₐᵣ), 127.24 (CHₐᵣ), 91.97 (Cq), 83.30 (Cq), 64.62 (CH₂, C-1'), 27.82 (CH₃, O*t*Bu), 26.35 (CH₃, Si*t*Bu), 18.88 (C_{q}, Si*t*Bu).
IR (ATR) *ṽ* (cm⁻¹) = 3433 (br), 2955 (w), 2928 (w), 2853 (w), 1772 (m), 1753 (m), 1721 (m), 1694 (m), 1469(w), 1426(m), 1367(m), 1329(s), 1252(m), 1164 (s), 1112 (vs), 1021 (m), 919 (w), 820 (s), 741 (m), 700 (vs).

### (4R,5S)-1-(tert-Butoxycarbonyl)-5-(tert-butyl-diphenyl-silanyloxymethyl)-2-oxo-4-vinyl-pyrrolidin (162)

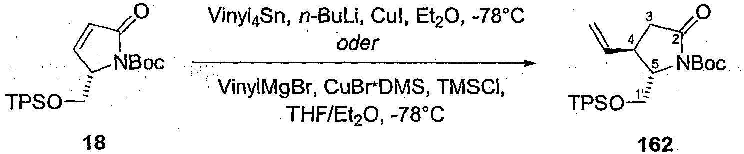

### Methode A:

Eine Lösung von 0.14 ml Tetravinylzinn (0.77 mmol, 3.5 Äq.) in 2 ml Et₂O (abs.) wurde bei 0 °C mit 1.99 ml *n*-BuLi (3.08 mmol, 1.54 M/Hexan, 14 Äq.) versetzt und eine Stunde gerührt. Dann wurden bei-20 °C 293 mg wasserfreies Cul (1.54 mmol, 7 Äq.) hinzugefügt, wobei sich eine schwarze Suspension bildete. Diese wurde auf-78 °C gekühlt; und eine Lösung von 100 mg Enon **18** (0.22 mmol) in 2 ml Et₂O (abs.) wurde zugetropft. Nach 2 Stunden wurden 2 ml ges. NH₄Cl-Lsg. zugetropft und die Reakfionsmischung auf RT gebracht Nach Zugabe von 15 ml MTBE und 5 ml Wasser wurde über Celite in einen Scheidetrichter filtriert, die organische Phase mit 2x 3 ml ges. NH₄Cl-Lsg und 3 ml ges. NaCl-Lsg. gewaschen, über MgSO₄ getrocknet und eingeengt. Chromatographie an Kieselgel (EE/CH 1:7) lieferte 64 mg **162** (0.13 mmol, 60 %) des Additionsproduktes als farbloses Öl.

### Methode B:

Eine Lösung von 81.90 ml Vinylmagnesiumbromid (81.90 mmol, 1M/THF, 10 Äq.) in 80 ml Et₂O (abs.) wurde bei -20 °C mit 8.41 g wasserfreiem CuBr·DMS (40.95 mmol, 5 Äq.) versetzt, worauf sich eine schwarze Suspension bildete. Nach 15 min. wurde auf-78 °C gekühlt, und man gab eine Lösung von 3.70 g Enon **18** (8.19 mmol) in 30 ml Et₂O (abs.) und 2.07 ml TMSCI (16.38 mmol, 2 Äq.) zu. Nach 2 Stunden wurden 70 ml ges. NH₄Cl-Lsg. zugegeben und die Reaktionsmischung auf RT erwärmt. Nach Zugabe von 300 ml MTBE und 20 ml Wasser wurde der Ansatz über Celite in einen Scheidetrichter filtriert, die organische Phase mit 3x 50 ml ges. NH₄Cl-Lsg. und 10 ml ges. NaCl-Lsg. gewaschen, so dass die blaue Farbe vollständig verschwand, über MgSO₄ getrocknet und eingeengt. Säulenchromatographie an Kieselgel (EE/CH 1:7) lieferte 2.30 g (4.79 mmol, 59 %) des Vinyl-Addionsproduktes **162** als gelbliches Öl.
**M (C₂₈H₃₇NO₄Si) = 479.6832.**
**R**_{f} = 0.32 (EE/CH 1:5).
**¹H-NMR** (300 MHz, CDCl₃): (Rotamerengemisch) δ (ppm) = 7.60 (m, 4H, PhH), 7.40 (m, 6H, PhH), 5.85 (ddd, 1H, J =17.4 Hz, J = 8.8 Hz, J = 4.6 Hz, CH_{olef}.), 5.06 (dd, 2H, J =14.2 Hz, J = 3.0 Hz, CH_{2,olef}.), 3.91 (m, 2H, H-5, H-1'_{α}), 3,72 (dd, 1H, J=11.9 Hz, 3.7 Hz, H-1'_{β}), 2.97 (m, 2H, H-3_{□}, H-4), 2.31 (td, 2H, J = 13.6 Hz, J = 6.2 Hz, H-3_{□}), 1.41 (s, 9H, O*t*Bu), 1.03 (s, 9H, Si*t*Bu).
**¹³C-NMR** (75 MHz, CDCl₃): (Rotamerengemisch) δ (ppm) =173.73 (C=O_{Lactam}), 149.72 (C_{q},_{Boc}), 139.12 (CH_{def.}), 135.50 (CHₐᵣ), 132.93, 132.56 (C_{q,ar}), 129.82, 129.67 (CHₐᵣ), 127.75, 127.62 (CHₐᵣ), 115.16 (CH_{2,def.}), 82.77 (C_{q,Boc}), 64.25 (CH, C-5). 63.84 (CH₂, C-1'), 37.84 (CH₂, C-3), 36.94 (CH, C-4), 28.32, 27.88 (CH₃, O*t*Bu), 26.71 (CH₃, Si*t*Bu), 19.09 (C_{q}, Si*t*Bu).
**IR** (ATR) *ṽ* (cm⁻¹) = 3070 (w), 2955 (m), 2929 (m), 2856 (m), 1787 (s), 1750 (vs), 1711 (vs), 1640 (w), 1588 (w), 1471 (m), 1426(m), 1391(m), 1366(s), 1307(vs), 1256(s), 1152 (vs), 1111(vs), 1062(m), 1026 (m), 997 (w), 970 (m), 920 (m), 872 (m), 847 (m), 821 (m), 778 (w), 740 (s), 701 (vs), 615 (m).
**MS** (DIP-EI, 70 eV): m/z (%) = 420 ([M⁺-*t*Bu-2H], <1), 401 (2), 394 (3), 366 (40), 322 (11), 272 (4), 244 (100), 224(4), 199(8), 181 (8),162 (6),135 (13),105 (7), 57 (43).
[α]_{D}²⁰=-21.9° (c=0.71, CHCl₃). Lit.:-23°(c=0.21, CHCl₃).

### (2S,3R)-1-(tert-Butoxycarbonyl)-2-(tert-butyl-diphenyl-silanyloxymethyl)-3-vinyl-pyrrolidin (163)

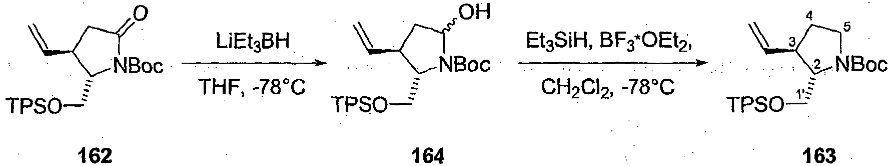

4.90 g **162** (10.21 mmol) wurden in 30 ml THF (abs.) gelöst und auf-78 °C gekühlt. Dann wurden 12.20 ml LiEt₃BH (1220 mmol, 1.2 Äq., 1M/THF) zugetropft. Nach einer Stunde wurden 10 ml K-Na-Tartrat-Lsg. (1.77 M) zugegeben und der Ansatz auf RT gebracht Zur Aufarbeitung wurden 50 ml MTBE zugegeben, die wäßrige Phase mit 2x 10 ml MTBE extrahiert und die vereinigten organischen Phasen über MgSO₄ getrocknet Nach Entfernen des Lösungsmittels im Vakuum erhielt man das Rohprodukt **164** als farbloses Öl, das im ÖPV getrocknet wurde.

Das Rohprodukt **164** wurde in 30 ml CH₂Cl₂ (abs.) aufgenommen, auf -78°C gekühlt und mit 3.20 ml Et₃SiH (20.14 mmol, 2 Äq.) und 2.80 ml BF₃·OEt₂ (22.10 mmol, 2.2 Äq.) versetzt und zwei Stunden lang gerührt. Nach Zugabe von 10 ml ges. NaHCO₃-Lsg. wurde die Reaktionsmischung auf RT erwärmt Der Ansatz wurde mit 100 ml MTBE verdünnt, die organische Phase mit 10 ml ges. NH₄Cl-Lsg. gewaschen und über MgSO₄ getrocknet. Nach Einengen wurde der Rückstand säulenchromatographisch an Kieselgel aufgereinigt (EE/CH 1:15), wodurch man 3.10 g 163 (6.66 mmol, 65 %) als farbloses Öl erhielt.
**M (C₂₈H₃₉NO₃Si) = 465.6997.**
**R_{f}** = 0.23 (SiO₂, EE/CH 1:15).
**¹H-NMR** (500 MHz, CDCl₃): (Rotamerengemisch) δ (ppm) = 7.61-7.71 (m, 4H, Hₐᵣ), 7.30-7-45 (m, 6H, Hₐᵣ), 5.79 (qd, 1H, J =17.6 Hz, J = 9.6 Hz, CH_{olef}.), 5.07 (m, 2H, CH_{2,olef.}), 4.09 (d, 0.4H, J = 6.4 Hz, CH₂, H-1'_{α}), 3.50-3.56 (m, 3.6H, H-2, H-5_{α}, H-1'_{β}), 3.35 (td, 1H, J =11.0 Hz, J = 6.9 Hz, H-5_{β}), 3.11 (m, 1H, H-3), 2.08 (qt, 1H, J=13.0 Hz, J = 6.5 Hz, H-4_{α}), 1.72 (td, 1H, J =13.3 Hz, J = 7.0 Hz, H-4_{β}), 1.45, 1.31 (2s, 9H, O*t*Bu), 1.04, 1.03 (2s, 9H, Si*tB*u).
**¹³C-NMR** (125 MHz, CDCl₃): (Rotamerengemisch) δ (ppm) = 154.13 (C=O), 139.69, 139.54 (CH_{olef.}), 135.40 (CHₐᵣ), 133.61, 133.38, 133.24 (C_{q,ar}), 129.52 (CHₐᵣ), 127.53 (CHₐᵣ), 114.80, 114.56 (CH_{2,olef.}), 79.05, 78.75 (C_{q,Boc}), 63.40, 63.29 (CH, C-2), 63.14, 61.84 (CH₂, C-1'), 46.58, 45.87 (CH₂, C-5), 44.74, 43.98 (CH, C-3), 30.46, 29.51 (CH₂, C-4), 28.30, 28.15 (CH₃, O*t*Bu), 26.73 (CH₃, Si*t*Bu), 19.16 (Cq, Si*t*Bu).
**IR** (ATR) *ṽ* (cm⁻¹) = 3070 (w), 3047 (w), 2960 (m), 2928 (m), 2856 (m), 1693 (vs), 1640 (w), 1588 (w), 1471 (m), 1426 (m), 1391 (s), 1364(s), 1344(w), 1255 (w), 1171 (s), 1111 (vs), 1029 (w), 1006 (w), 989 (w), 914 (m), 899 (m), 859 (w), 822 (m), 772 (m), 740 (m), 700 (s), 604 (m).
**MS** (DIP-EI, 70 eV): m/z(%) = 408 ([M⁺-*t*Bu], 1), 392 (5), 380 (3), 368 (4), 352 (100), 306 (8), 253 (7), 230 (57), 211 (5),199 (60), 181 (24),168 (6),140 (81),121 (7),105 (6), 96 (24), 77 (2), 57 (2).
**HRMS** (ESI, C₂₈H₃₉NNaO₃Si): ber.: 488.2597, gef.: 488.260.
[α]_{λ}²⁰= - 34.0° (589 nm), - 40.4° (546 nm), - 83.5° (405 nm), -110.2° (365 nm), -142.2° (334 nm) (c = 0.725, CHCl₃).

| | | | |
|---|---|---|---|
| **CHN-Analyse:** | ber.: C: 72.21 %, | H: 8.44 %, | N: 3.01 %; |
| | gef.: C: 71.78%, | H: 8.50 %, | N: 2.88%. |

### (2S,3R)-1-(tert-Butoxycarbonyl)-2-(hydroxymethyl)-3-vinyl-pyrrolidin (165)

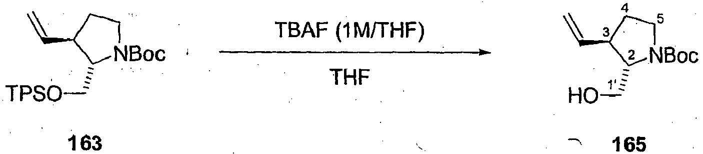

2.67 g 163 (5.73 mmol) wurden in 35 ml THF (abs.) gelöst, auf 0°C gekühlt und mit 8.60 ml TBAF-Lösung (8.60 mmol, 1.5 Äq., 1M/THF) versetzt. Nach Reaktion über Nacht bei RT wurden 0.5 ml Wasser zugegeben, und man ließ 30 min. rühren. Anschließend wurde der Ansatz mit etwas MTBE über wenig Kieselgel filtriert und eingeengt. Säulenchromatographie an Kieselgel (EE/CH 1:3) lieferte 1.28 g Alkohol 165 (5.63 mmol, 98 %) als farbloses Öl.
**M (C₁₂H₂₁NO₃) = 227.3001.**
**R_{f}** = 0.18 (SiO₂, EE/CH 1:3).
**¹H-NMR** (300 MHz, CDCl₃): δ (ppm) = 5.72 (m, 1H, CH_{def.}), 5.06 (ddd, 2H, J = 8.7 Hz, J = 4.9 Hz, J = 3.8 Hz, CH_{2,olef}), 3.71 (t, 1H, J = 8.9 Hz, H-1'_{α}). 3.54 (m, 3H, H-2, H-5_{α}, H-1'_{β}), 3.20 (ddd, 1H, J =10.9 Hz, J = 9.8 Hz, J = 6.5 Hz, H-5_{β}), 2.31 (br, 1H, H-3),1.91 (br, 1H, H-4_{α}), 1.62 (m, 1H, H-4_{β}), 1.42 (s, 9H, O*t*Bu).
¹**³C-NMR** (75 MHz, CDCl₃): δ (ppm) =156.56 (Cq, C=O), 137.93 (CH_{olef.}), 116.32 (CH_{2olef.}), 80.09 (C_{q,Boc}), 65.54 (CH₂, C-1'), 64.94 (CH, C-2), 46.52 (CH₂, C-5), 45.87 (CH, C-3), 30.40 (CH₂, C-4), 28.25 (CH₃, O*t*Bu).
**IR** (ATR) *ṽ* (cm⁻¹) = 3407 (br), 3078 (w), 2973 (s), 2930 (m), 2879 (m), 1692 (vs), 1669 (vs), 1477 (s), 1453(s), 1404(vs), 1365(vs), 1344(m), 1253(m), 1169(vs), 1117(s), 1086(m), 1054(m), 992(m), 916 (m), 894 (w), 861 (w), 772 (m), 668 (w).
**MS** (GCMS-EI, 70 eV): m/z (%) = 227 ([M⁺], <1), 207 (<1), 196 (13),154 (10),140 (52), 96 (71), 81 (4), 67 (13), 57 (100).
**HRMS** (ESI, C₁₂H₂₁NNaO₃): ber.: 250.1419, gef.: 250.142.
**[α]_{λ}²⁰** =-52.5° (589 nm), -123.7° (405 nm), -159.2° (365 nm), -199.2° (334 nm), -62.0° (546 nm), (c= 1.08, CHCl₃).

| | | | |
|---|---|---|---|
| **CHN-Analyse:** | C: 63.41 %, | H: 9.31 %, | N: 6.16 %; |
| | C: 63.22 %, | H: 9.48 %, | N: 6.28 %. |

### (2S,3R)-1-(tert-Butoxycarbonyl)-3-vinyl-pyrrolidin-2-carbonsäure(166)

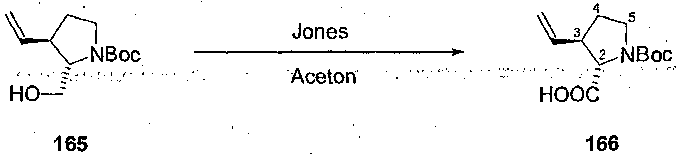

600 mg Alkohol **165** (2.64 mmol) wurden in 15 ml Aceton gelöst, auf 0°C gekühlt und tropfenweise mit 5 ml Jones-Reagenz (7.40 mmol, 2.8 Äq.) (frisch zubereitet aus: 2 g CrO₃, 2.7 g konz. H₂SO₄, 12 ml H₂O) versetzt und 2 h bei RT gerührt, wobei der Verlauf der Reaktion per DC (EE/CH 1:1) kontrolliert wurde. Anschließend wurden 4 ml Isopropanol zugegeben, wobei die orange Farbe der Reaktionsmischung allmählich nach grün-braun umschlug. Nach Filtration über Celite wurde mit 5 ml einer Aceton/Eisessig-Mischung (20:1) gewaschen und vollständig eingeengt. Der verbleibende grünliche Rückstand wurde säulenchromatographisch an Kieselgel aufgereinigt (CH₂Cl₂/MeOH 20:1), wodurch man 470 mg Vinylprolin 166 (1.95 mmol, 74 %) in Form weißer Kristalle erhielt, die für die Röntgenstrukturanalyse geeignet waren.
**M(C₁₂H₁₉NO₄) = 2412836.**
**R_{f} =** 0.26 (SiO₂, CH₂Cl₂/MeOH 19:1).
**Smp.:** 116 -118 °C.
**¹H-NMR** (300 MHz, CDCl₃): (Rotamerengemisch) δ (ppm) =10.70 (br, 1H, COOH), 5,81 (ddd, 1H, J = 17.3 Hz, J =10.3 Hz, J = 7.3 Hz, CH_{olef.}), 5.12 (m, 2H, CH_{2,olef.}), 4.09/3.95 (2d, 1H, J = 5.6 Hz, H-2), 3.55 (m, 2H, H-5), 2.98 (m, 1H, H-3), 2.08 (m, 1H, H-4_{α}), 1.77 (m, 1 H, H-4_{β}), 1.45, 1.39 (2s, 9H, O*t*Bu).
¹**³C-NMR** (75 MHz, CDCl₃): (Rotamerengemisch) δ (ppm) =177.89, 176.37 (C=O_{Carbonsäure}),155.05, 153.73 (C=O_{Boc}), 137.09, 136.84 (CH_{olef.}), 116.35, 116.01 (CH_{2,olef.}), 80.65, 80.55 (C_{q,Boc}), 64.06, 63.73 (CH, C-2), 48.19, 45.76 (CH, C-3), 45.93, 45.64 (CH₂, C-5), 30.53, 3024 (CH₂, C-4), 2829,28.15 (CH₃, O*t*Bu).
**IR** (ATR) *ṽ* (cm⁻¹) = 3085 (br), 2975 (s), 2873 (m), 2559 (br), 2245 (w), 1744 (vs), 1696 (br, vs), 1477 (s), 1391 (vs), 1365(vs), 1242(s), 1161 (vs), 1119(vs), 989(m), 913(vs), 858(m), 772(m), 730(s), 681 (m), 646(w).
**MS** (DIP-EI, 70 eV): m/z (%) = 241 ([M⁺], <1), 207 (1),196 (5),168 (3),140 (100), 126 (5),110 (3), 96 (77), 87 (3), 79 (4), 68 (16), 57 (88).
**ESI-MS** (C₁₂H₁₉NNaO₄): ber.: 264.12, gef.: 264.30.
[α]_{λ}²⁰ = -38.5° (589 nm), -45.6° (546 nm), -98.7° (405 nm), -133.4° (365 nm), absorbiert bei 334 nm (c = 0.765, CHCl₃).

| | | | |
|---|---|---|---|
| **CHN-Analyse:** | ber.: C: 59.73 %, | H: 7.94 %, | N: 5.81 %; |
| | gef.: C: 59.68 %, | H: 7.89 %, | N: 5.63 %. |

### Kristalldaten:

| | | |
|---|---|---|
| Identification code: | jz101 | |
| Empirical formula: | C₁₂H1₉NO₄ | |
| Formula weight: | 241.28 | |
| Temperature: | 100(2) K | |
| Wavelength: | 0.71973Å | |
| Crystal system, space group: | orthorhombic, P212121 | |
| Unit cell dimensions: | a = 9.2926(2) Å | α = 90° |
| | b =11.8100(3) Å | β = 90° |
| | c =12.1256(2) Å | γ = 90° |
| Volume: | 1330.73(5) Å³ | |
| Z, Calculated density: | 4, 1.204 g/cm³ | |
| Absorption coefficient: | 0.090 mm | |
| F(000): | 520 | |
| Crystal size: | 0.1 x 0.1 x 0.2 mm | |
| Theta range for data collection: | 2.44 to 27.00° | |
| Limiting indices: | -11<=h<=11, -14<=k<=14, -15<=l<=15 | |
| Reflections collected/unique: | 11042/2797 [R(int) = 0.0266] | |
| Reflection observed [I>2sigma(I)] | 2494 | |
| Completeness to theta = 25.00 | 100 % | |
| Absorption correction: | none | |
| Refinement method: | Full-matrix least-squares on F² | |
| Data/restraints/parameters: | 2797/0/230 | |
| Goodness-of-fit on F²: | 1.043 | |
| Final R indices [I>2sigma(I)]: | R₁ = 0.0306, wR₂ = 0.0751 | |
| R indices (all data): | R₁ = 0.0368, wR₂ = 0.0784 | |
| Absolute structure parameter. | -0.2(8) | |
| Largest diff.peak hole: | 0.155 and -0.158 e.A⁻³ | |

### (2S)-1-(tert-Butoxycarbonyl)-5-oxo-pyrrolidin-2-carbonsäure-ethylester (167)

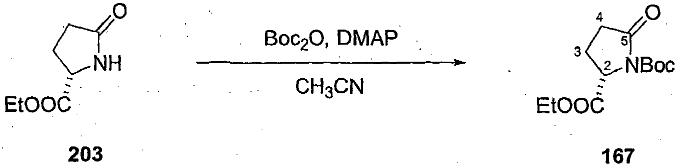

Eine Lösung von 7.00 g Pyroglutaminsäure-ethylester 203 (44.5 mmol) in 60 ml Acetonitril (abs.) wurde auf 0 °C gekühlt und mit 6.3 ml NEt₃ (45.4 mmol, 1.02 Äq.), einer Spatelspitze DMAP und 10.5 ml Boc₂O (49.1 mmol, 1.1Äq.) versetzt. Nach 15 h Rühren bei RT wurde das Lösungsmittel entfernt und der Rückstand chromatographisch an Kieselgel (EE/CH 1:1) aufgereinigt. Das gelbliche, feste Produkt wurde durch Umkristallisation aus Et₂O/Pentan weiter aufgereinigt, worauf man 11.0 g 167 (42.9 mmol, 96 %) in Form weißer Kristalle erhielt.
**M (C₁₂H₁₉NO₅)** = **257.2830.**
**R_{f} =** 0.31 (SiO₂, EE/CH 1:1).
**Smp.:** 53 °C. Lit.:50-51°C.
**¹H-NMR** (500 MHz, CDCl₃): δ (ppm) = 4.57 (dd, 1H, J = 9.5 Hz, J = 2.9 Hz, H-2), 4.21 (q, 2H, J = 7.1 Hz, CH_{2,Ester}), 2.60 (td, 1H, J =17.5 Hz, J = 9.9 Hz, H-4_{α}), 2.46 (ddd, 1H, J =17.5 Hz, J = 9.4 Hz, J = 3.5 Hz, H-4_{β}), 2.29 (qd, 1H, J =13.3 Hz, J = 9.7 Hz, H-3_{α}), 2.00 (tdd, 1H, J =13.0 Hz, J = 9.6 Hz, J = 3.2 Hz, H-3_{β}), 1.51 (s, 9H, O*t*Bu), 1.27 (t, 1H, J = 7.1 Hz, CH_{3,Ester}).
**¹³C-NMR** (125 MHz, CDCl₃): δ (ppm) =173.31 (C=O), 171.30 (C=O), 83.54 (C_{q,Boc}), 61.64 (CH_{2,Ester}), 58.93 (CH, C-2), 31.14 (CH₂, C-4), 27.86 (CH₃, O*t*Bu), 21.53 (CH₂, C-3), 14.16 (CH_{3,Ester}).
IR (ATR) *ṽ* (cm⁻¹) = 2977 (m), 2932 (w), 1788 (vs), 1741 (vs), 1713 (vs), 1475 (m), 1457 (m), 1393 (m), 1367 (s), 1306 (vs), 1284 (vs), 1254 (vs), 1189 (vs), 1146 (vs), 1095 (m), 1042 (s), 1019 (vs), 958 (w), 914 (w), 883 (w), 842 (m), 819 (m), 777 (m), 746 (m), 639 (w).
MS (DIP-EI, 70 eV): m/z (%) = 242 ([M+-15], <1), 202 (1), 184 (10),158 (9),156 (8), 149 (1), 128 (2), 110 (8), 84 (83), 75 (3), 69 (4), 57 (100).
[α_{]D}²⁰ = -34.9° (c = 0.810, CHCl₃). Lit.: -32.3° (c = 2.1, CHCl₃).

### (2S,5R/S)-1-(tert-Butoxycarbonyl)-5-hydroxy-pyrrolidin-2-carbonsäure-ethylester (205)

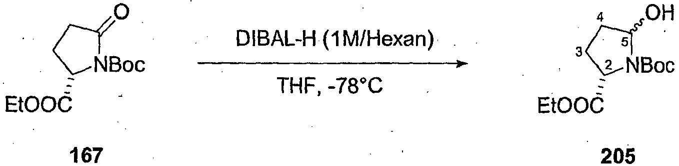

Eine Lösung von 10.0 g 167 (38.9 mmol) in 100 ml THF (abs.) wurde auf-78 °C gekühlt und sehr lang-sam mit 85.5 ml DIBAL-H (85.5 mmol, 2.2 Äq., 1M/THF) versetzt. Nach einer Stunde wurde die Reaktion durch Zugabe von 10 ml Isopropanol in der Kälte abgebrochen, dann wurden 240 ml K-Na-Tartrat Lsg. (1.77 M) zugegeben und die Mischung langsam aufgetaut, zunächst auf 0 °C und anschließend auf RT. Die entstehenden Phasen wurden getrennt, die organische Phase mit 3x 200 ml MTBE extrahiert und die vereinten organischen Phasen mit 100 ml ges. NaCl-Lsg. gewaschen. Nach Trocknen über MgSO₄ wurde das Lösungsmittel entfernt, und man erhielt 9.69 g **205** (37.4 mmol, 96 %) als gelblichen öligen Rückstand, der ohne weitere Reinigung umgesetzt wurde. Für analytische Zwecke wurde eine kleine Probe säulenchromatographisch an Kieselgel (EE/CH 1:1) aufgereinigt.
**M (C₁₂H₂₁NO₅)** = **259.2989.**
**R_{f} =** 0.27 (SiO₂, EE/CH 1:1).
**¹H-NMR** (300 MHz, CDCl₃): (Diastereomeren-/Rotamerengemisch) δ (ppm) = 5.40-5.70 (m, 1H, H-5), 4.06-4.40 (m, 3H, H-2 und CH_{2,Ester}), 3.40-3.75 (m, 1H, OH), 1.77-2.60 (m, 4H, H-3, H-4), 1.35-1.52 (m, 9H, *t*Bu), 1.17-1.30 (m, 2H, CH_{3,Ester}).
¹**³C-NMR** (75 MHz, CDCl₃): (Diastereomeren-/Rotamerengemisch) δ (ppm) = 173.01, 172.75, 172.45, 172.18 (C=O_{Ester}), 154.03, 153.82, 153.15 (C=O_{Boc}), 82.16,81.91, 81.67 (CH, C-5), 80.96, 80.87, 80.71 (C_{q}, O*t*Bu), 61.10, 60.90, 60.83 (CH_{2,Ester}), 59.43, 59.15, 59.00 (CH, C-2), 32.31, 31.77, 31.05 (CH_{2,} C-4), 28.27,28.08 (CH₃, O*t*Bu), 27.80, 27.69, 26.83, 26.77 (CH₂, C-3),14.14,13.99 (CH_{3,Ester}).
**IR** (ATR) *ṽ* (cm⁻¹) = 3480 (br), 2976 (m), 1741 (s), 1696 (vs), 1476 (m), 1452(m), 1364(vs), 1323 (m), 1256 (m), 1156 (vs), 1123 (s), 1061 (m), 1025 (vs), 973 (m), 914 (m), 848 (m), 775 (m).
**MS** (DIP-EI, 70 eV): m/z(%) = 258 [(M⁺-H], 1), 242 (10), 208 (3), 202 (4),186 (3),158 (18),142 (100), 130 (6),114 (3), 86 (14), 68 (28), 57 (37).

### (2S,5R/S)-1-(tert-Butoxycarbonyl)-5-methoxy-pyrrolidin-2-carbonsäure-ethylester (168)

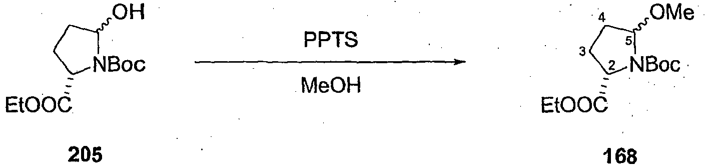

Eine Lösung von 9.69 g 205 (37.4 mmol) in 100 ml MeOH (abs.) wurde mit 200 mg PPTS (0.80 mmol, 2.1 mol-%) versetzt und über Nacht bei RT gerührt. Dann wurde das Lösungsmittel vollständig entfernt und der verbleibende Rückstand säulenchromatographisch an Kieselgel (EE/CH 1:3) aufgereinigt. Man erhielt 9.56 g α-Methoxycarbamat **168** (35.0 mmol, 94%) als farbloses Öl (Diastereomerengemisch).
**M (C₁₃H₂₃NO₅)** = **273.3255.**
R_{f} = 0.26 (SiO₂, EE/CH 1:3).
**¹H-NMR** (500 MHz, CDCl₃): (Diastereomeren-/Rotamerengemisch) δ (ppm) = 5.11-5.29 (m, 1H, H-5), 4.05-4.38 (m, 3H, CH_{2,Ester}, H-2), 3.37 (m, 3H, OCH₃), 1.65-2.50 (m, 4H, H-3, H-4), 1.39, 1.41, 1.46, 1.48 (4s, 9H, Boc), 125 (m, 3H, CH_{3,Ester}).
**¹³C-NMR** (125 MHz, CDCl₃): (Diastereomeren-/Rotamerengemisch) δ (ppm) =172.60, 172.28 (C=O_{Ester}), 154.15, 153.95, 153.81, 153.74 (C=O_{Boc}), 89.16, 89.11, 88.34, 88.16 (CH, C-5), 80.52, 80.37, 80.30 (C_{q,Boc}), 60.73 (CH_{2,Ester}), 59.50, 59.21, 58.88, 58.81 (CH, C-2), 56.00, 55.72, 55.11, 54.78 (CH₃, OCH₃), 32.78, 32.10, 30.92, 29.90 (CH₂, C-4), 28.15, 27.99 (CH_{3,Boc}), 27.64, 26.93, 26.83 (CH₂, C-3), 14.10,13.96 (CH_{3,Ester}).
**IR** (ATR) *ṽ* (cm⁻¹) = 2976(m), 2935 (m), 1746(s), 1707(vs), 1476(m), 1456(m), 1442(m), 1375(vs), 1329(m), 1255(m), 1185(vs), 1116(m), 1085(vs), 1035(m), 939(m), 912(m), 844(m), 773(m).
**MS** (GCMS-EI, 70 eV): m/z (%) = 241 ([M⁺-MeOH], 2), 200 (12),172 (8),142 (24),100 (48), 68 (100), 57 (88), 41 (63).

### (2S,5R/S)-5-Allyl-1-(tert-butoxycarbonyl)-pyrrolidin-2-carbonsäure-ethylester (169)

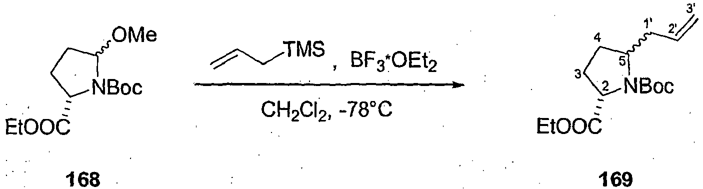

Eine Lösung von 9.25 g α-Methoxycarbamat 168 (33.8 mmol) in 100 ml CH₂Cl₂ (abs.) wurde auf-78 °C gekühlt, zuerst mit 13.50 ml Allyltrimethylsilan (84.6 mmol, 2.5 Äq.) und dann tropfenweise mit 8.58 ml BF₃·OEt₂ (67.7 mmol, 2 Äq.) versetzt und eine Stunde gerührt. Dann wurden in der Kälte 10 ml H₂O und 30 ml ges. NaHCO₃-Lsg. zugegeben, man brachte den Ansatz auf 0 °C, ließ 30 min. rühren und taute auf RT auf. Nach Trennen der Phasen wurde die wäßrige Phase mit 3x 50 ml CH₂Cl₂ extrahiert, die vereinten organischen Phasen mit 30 ml ges. NaCl-Lsg. gewaschen und über MgSO₄ getrocknet. Nach Entfernen des Lösungsmittels wurde der Rückstand säulenchromatographisch an Kieselgel (EE/CH 1:6) gereinigt. Man erhielt 6.83 g Allyl-Substitutionsprodukt 169 (24.1 mmol, 71 %) als farbloses Öl (Epimerengemisch, *cis*/*trans*4:1)*.*
**M (C₁₅H₂₅NO₄)** = **283.3633.**
**R_{f}**=0.39 (SiO₂, EE/CH 1:3).
**¹H-NMR** (500 MHz, CDCl₃): (Diastereomeren-/Rotamerengemisch) δ (ppm) = 5.70 (m, 1H, H-2'), 4.95 (m, 2H, H-3'), 4.00-4.28 (m, 3H, H-2, CH_{2,Ester}), 3.76, 3.87 (2br, 1H, H-5), 2.32-2.70 (m, 1H, H-1'_{α}), 1.62-2.23 (m, 5H, H-1'_{β}, H-3, H-4), 1.35, 1.36, 1.41, 1.42 (4s, 9H, O*t*Bu), 1.21 (m, 3H, CH_{3,Ester}).
**¹³C-NMR** (125 MHz, CDCl₃): (Diastereomeren-/Rotamerengemisch) δ (ppm) = 173.31, 173.11, 172.72 (C=O_{Ester}), 153.49 (C=O_{Boc}), 135.34,135.06,134.99 (CH_{2,olef.}, C-3'), 11720, 117.12, 116.77 (CH_{olef}, C-2'), 79.78 (C_{q,Boc}), 60.77, 60.74 (CH_{2,Ester}), 60.15, 59.91, 59.84, 59.59 (CH, C-2), 58.00, 57.89, 57.46, 57.41 (CH, C-5), 39.01, 38.94, 38.10, 38.05 (CH₂, C-1'), 29.45, 28.72, 28.40 (CH₂, C-3/C-4), 28.32, 28.20 (CH₃, O*t*Bu), 27.96, 27.75, 27.40, 26.75 (CH₂, C-3/C-4), 14.21, 14.17 (CH_{3,Ester}).
**IR** (ATR) *ṽ* (cm⁻¹) = 3074 (w), 2974 (s), 2931 (m), 1746 (s), 1696 (vs), 1639 (w), 1477 (m), 1451 (m), 1388(vs), 1365(vs), 1329(m), 1273(m), 1256(m), 1184(vs), 1165(vs), 1123(s), 1105(s), 1031 (m), 995 (m), 948 (m), 912 (m), 859 (m), 770 (m).
**MS** (GCMS-EI, 70 eV): m/z(%) = 242 ([M⁺-C₃H₅], 6), 210 (3), 182 (3),154 (6),142 (100),114 (5), 110 (8), 96 (3), 68 (20), 57 (40).
[α]_{D}²⁰ = -26.4° (c = 1.025, CHCl₃).

### (2S,5R/S)-1-tert-(Butoxycarbonyl)-5-(2-hydroxyethyl)-pyrrolidin-2-carbonsäure-ethylester (170)

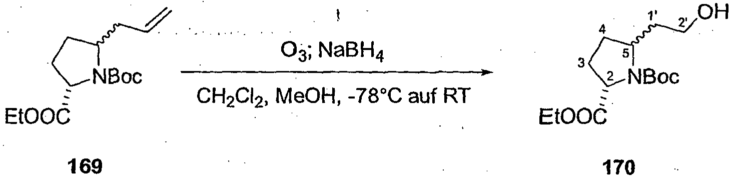

Durch eine Lösung von 1.00 g 169 (3.53 mmol) in einem Gemisch aus 12 ml MeOH und 6 ml CH₂Cl₂ wurde bei -78 °C Ozon geleitet, bis sich die Lösung blau färbte. Dann wurde die Reaktion abgebrochen und das überschüssige Ozon durch Spülen mit Sauerstoff vertrieben. Anschließend wurden 270 mg NaBH₄ (7.06 mmol, 2 Äq.) zugegeben und die Mischung über Nacht gerührt, wobei sie sich langsam auf RT erwärmte. Nachdem die Vollständigkeit der Reaktion per DC (EE/CH 1:1) überprüft worden war, wurde der Ansatz eingeengt und der verbleibende Rückstand in 60 ml CH₂Cl₂ und 40 ml halbkonz NaCl-Lsg. aufgenommen. Die wäßrige Phase wurde mit 3x 20 ml CH₂Cl₂ extrahiert, die vereinigten organischen Phasen über MgSO₄ getrocknet und eingeengt. Der Rückstand wurde säulenchromatographisch an Kieselgel (EE/CH 1:1) aufgereinigt, worauf man 691 mg (2.40 mmol, 68 %) des Alkohols **170** als Epimerengemisch erhielt (farbloses Öl).
**M (C₁₄H₂₅NO₅) = 287.3520.**
**R_{f}** = 0.20 (SiO₂, EE/CH 1:1).
**¹H-NMR** (300 MHz, CDCl₃): (Diastereomeren-/Rotamerengemisch) δ (ppm) = 3.95-4.33 (m, 5H, H-2, H-5, CH_{2,Ester}, OH), 3.42-3.78 (m, 2H, CH₂), 1.45-2.33 (m, 6H, 3x CH₂), 1.43,1.37 (2s, 9H, O*t*Bu), 1.22 (t, 3H, J = 6Hz, CH_{3,Ester}).
**¹³C-NMR** (75 MHz, CDCl₃): (Diastereomeren-/Rotamerengemisch) δ (ppm) =173.24 (C=O_{Ester}), 155.54, 155.32 (C=O_{Boc}), 80.75, 80.60 (C_{q,Boc}). 60.90 (CH_{2,Ester}), 59.99 (CH, C-2), 59.01, 58.74 (CH₂), 54.47, 54.30 (CH, C-5), 38.78, 37.57 (CH₂), 30.52 (CH₂), 29.10, 28.77, 28.62 (CH₂), 28.11 (CH₃, *t*Bu),14.14 (CH_{3,Ester}).
**IR** (ATR) *ṽ* (cm⁻¹) = 3444 (br), 2975 (m), 2873 (m), 1743 (s), 1674 (vs), 1476 (m), 1451 (m), 1390 (vs), 1364 (vs), 1299 (m), 1256 (m), 1156 (vs), 1116 (vs), 1069 (s), 1034 (s), 991 (m), 926 (w), 854 (m), 773 (m).
**MS** (DIP-EI, 70 eV): m/z = 287 ([M⁺], 1), 231 (2),214 (8), 186 (4), 158 (8),142 (23),128 (3),114 (100), 96 (4), 82 (3), 68 (16), 57 (54).
**[α]_{D}²⁰** = -48.9° (c = 0.885, CHCl₃).

### (2S,5R/S)-1-(tert-Butoxycarbonyl)-5-[2-(2-nitro-phenylselanyl)ethyl]-pyrrolidin-2-carbonsäure-ethylester (171)

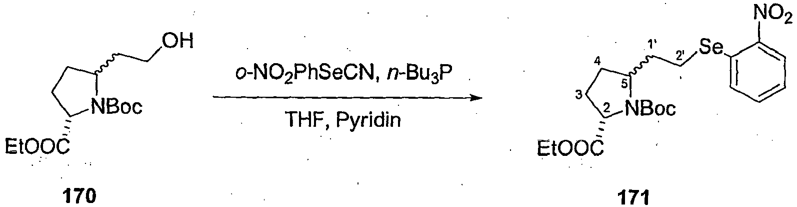

In einem Lösemittelgemisch, bestehend aus 10 ml THF (abs.) und 3 ml Pyridin (abs.) wurden 497 mg Alkohol 170 (1.73 mmol) gelöst und 432 mg *ortho*-Nitrophenylselenocyanat (1.90 mmol, 1.1 Äq.) suspendiert. Anschließend wurden bei 0 °C vorsichtig 0.56 ml *n*-Tributylphosphin (2.25 mmol, 1.3 Äq.) zugetropft, wobei man eine dunkelrote Lösung erhielt. Nach einer Stunde wurde der Ansatz mit 30 ml MTBE verdünnt, über wenig Kieselgel filtriert und eingeengt. Der braune Rückstand wurde säulenchro-matographisch an Kieselgel (EE/CH 1:3) aufgereinigt, wobei man 685 mg Selenylether 171 (1.45 mmol, 84 %) als intensiv gelb-gefärbtes Öl erhielt.
**M (C₂₀H₂₈N₂O₆Se) = 471.4062.**
**R_{f}** =0.26 (SiO₂, EE/CH 1:3).
**¹H-NMR** (250 MHz, CDCl₃): (Diastereomeren-/Rotamerengemisch) δ (ppm) = 8.10-8.35 (m, 1H, Hₐᵣ), 7.20-7.70 (m, 3H, Hₐᵣ), 3.95-4.40 (m, 4H, C-2, C-5, CH_{2,Ester}), 2.75-3.12 (m, 2H, CH₂), 1.62-2.35 (m, 6H, 3x CH₂), 1.37-1.45 (m, 9H, O*t*Bu), 1.19-1.28 (m, 3H, CH_{3,Ester}).

### (2S,5R/S)-1-(tert-Butoxycarbonyl)-5-vinyl-pyrrolidin-2-carbonsäure-ethylester (172)

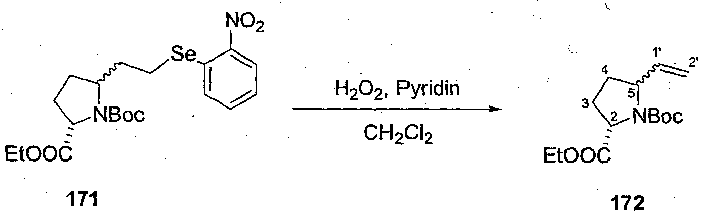

Eine Lösung von 576 mg Selenylether 171 (1.22 mmol) in 15 ml CH₂Cl₂ (abs.) wurde auf 0 °C gekühlt, mit 0.4 ml Pyridin und 0.8 ml H₂O₂ (30%) versetzt und über Nacht gerührt. Anschließend wurde die stark orangefarbene Lösung eingeengt und säulenchromatographisch an Kieselgel gereinigt (CH₂Cl₂). Man erhielt 312 mg Vinylprolin **172** (1.16 mmol, 95 %) als gelbliches Öl.
**M (C₁₄H₂₃NO₄) = 269.3368.**
**R_{f}** = 0.10 (SiO₂, CH₂Cl₂).
**¹H-NMR** (300 MHz, CDCl₃): (Diastereomeren-/Rotamerengemisch) δ (ppm) = 5.60-5.90 (m, 1H, CH_{olef.}, H-1'), 4.92-5.38 (m, 2H, CH_{2,olef.}, H-2'), 4.02-4.55 (m, 4H, H-2, H-5, CH_{2,Ester}), 1.66-2.25 (m, 4H, H-3, H-4), 1.35,1.34 (2s, 9H, O*t*Bu), 1.22 (t, 3H, J = 9 Hz, CH_{3,Ester}).
**¹³C-NMR** (75 MHz, CDCl₃): (Diastereomeren-/Rotamerengemisch) δ (ppm) = 172.98, 172.91, 172.79 (C=O_{Ester}), 153.47, 153.28, 153.27 (C=O_{Boc}), 138.90, 138.32, 138.12, 137.79 (CH_{olef.}, C-1'), 114.90, 114.64, 113.90, 113.69 (CH_{2,olef.}, C-2'), 79.86, 79.81, 79.70 (C_{q,Boc}), 60.74 (CH_{2,Ester}), 60.52, 60.22, 59.91, 59.61, 59.53, 59.33, 59.18 (2x CH, C-2, C-5), 31.47, 31.01, 30.84, 30.69, 30.46, 30.11, 29.83, 29.03, 28.79, 2721 (2x CH₂, C-3, C-4), 28.18 (CH₃, O*t*Bu), 14.17, 14.07 (CH_{3,Ester}).
**IR** (ATR) *ṽ* (cm⁻¹) = 3078 (w), 2975 (m), 2928 (m), 1744 (vs), 1692 (vs), 1643 (w), 1477 (m), 1454 (m), 1383 (vs), 1364 (vs), 1284 (s), 1254 (s), 1157 (vs), 1110 (vs), 1068 (m), 1031 (s), 991 (m), 913 (s), 859 (s), 769 (s), 680 (w).
**MS** (GCMS-EI, 70 eV): m/z (%) = 269 ([M⁺], <1), 213 (4),196 (11), 168 (8),140 (60), 96 (100), 79 (8), 67 (8), 57 (77).
**[α]_{D}²⁰** = -56.2° (c = 1.13, CHCl₃).

### (2S,5R/S)-5-Vinyl-pyrrolidin-2-carbonsäure-ethylester (174)

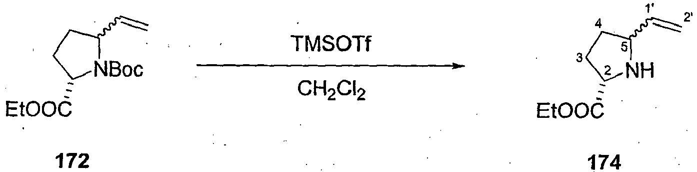

130 mg 172 (0.46 mmol) wurden in 3 ml CH₂Cl₂ (abs.) gelöst, auf 0 °C gekühlt und tropfenweise mit 90 µl TMSOTf (0.466 mmol, 1.01 Äq.) versetzt. Nach 15 min. wurden 0.5 ml ges. NaHCO₃-Lsg., 0.5 ml H₂O und 10 ml Et₂O zugegeben und der Ansatz auf RT gebracht. Nach Phasentrennung wurde die organische Phase über MgSO₄ getrocknet, filtriert und eingeengt. Das erhaltene Rohprodukt wurde säulenchromatographisch an Kieselgel (DCM/MeOH 25:1) gereinigt, worauf man 54 mg Amin 174 (0.32 mmol, 70%) als leicht gelbliches Öl isolierte (Diastereomerengemisch, *cis:trans* = 4:1).
**M (C₉H₁₅NO₂) = 169.2209.**
**R_{f}** = 0.19 (SiO₂, CH₂Cl₂/MeOH 25:1).
**¹H-NMR** (300 MHz, CDCl₃): (Diastereomerengemisch) δ (ppm) = 5.67-5.88 (m, 1H, H-1'), 5.07-5.18 (m, 1 H, H-2'_{α}), 4.93-5.02 (m, 1H, H-2'_{β}), 4.08-4.17 (m, 2H, CH_{2,Ester}), 3.77-3.84 (m, 0.2H, H-2), 3.65-3.76 (m, 1 H, H-2, H-5), 3.50-3.61 (m, 0.8H, H-5), 2.22 (br, 1H, NH), 1.98-2.18 (m, 1H, H-3_{α}), 1.75-1.96 (m, 2H, H-3_{β}, H-4_{α}), 1.36-1.56 (m, 1H, H-4_{β}), 1.22 (t, 3H, J = 7.1 Hz, CH_{3,Ester}).
**¹³C-NMR** (75 MHz, CDCl₃): (Diastereomerengemisch) δ (ppm) = 174.98 (C=O_{Ester}), 140.83, 140.07 (CH_{olef.}, C-1'), 115.04,114.39 (CH_{2,olef.}, C-2'), 62.11, 60.77 (CH, C-5), 60.88, (CH_{2,Ester}), 59.93, 59.15 (CH, C-2), 31.79 (CH₂, C-4), 30.00, 29.37 (CH₂, C-3), 14.12 (CH_{3,Ester}).
**IR** (ATR) *ṽ* (cm⁻¹) = 3344 (br), 3078 (w), 2977 (m), 2866 (w), 1730 (vs), 1639 (w), 1446 (m), 1369 (m), 1276 (m), 1208 (s), 1100 (m), 1035 (m), 993 (m), 918 (m), 861 (w), 759 (w).
**MS** (GCMS-EI, 70 eV): m/z (%) = 169 ([M⁺], 1), 142 (1), 96 (100), 79 (12), 68 (11), 54 (3).
**HRMS** (EI, C₉H₁₅NO₂) ber.: 169.1103 gef.: 169.109.
**[α]_{D}²⁰** =-39.8° (c =1.065, CHCl₃).

### (2S,2'S,3'R,5R/S)-1-[1'-((tert-Butoxycarbonyl)-3'-vinyl-pyrrolidin-2'-yl)-carbonyl]-5-vinyl-pyrrolidin-2-carbonsäure-ethylester (180)

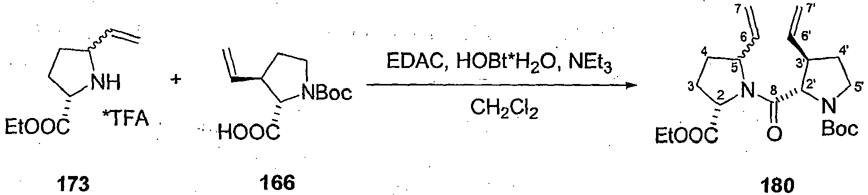

Zu einer Lösung von 34 mg Carbonsäure **166** (141 µmol) in 2 ml CH₂Cl₂ (abs.) wurden bei 0 °C 23 mg HOBt·H₂O (170 µmol, 1.2 Äq.) und 33 mg EDAC (170 µmol, 1.2 Äq.) gegeben und die Mischung 15 min. gerührt, wobei das HOBt in Lösung ging. Anschließend wurden 50 mg Ammoniumsalz 173 (177 µmol, 1.25 Äq.) und 25 µl NEt₃ (170 µmol, 1.2 Äq.) hinzugefügt und der Ansatz über Nacht gerührt. Zur Aufarbeitung wurde die Reaktionsmischung mit 10 ml MTBE verdünnt, mit wenig halbgesättigter NH₄Cl-Lsg. gewaschen und die organische Phase über MgSO₄ getrocknet. Nach Einengen wurde der Rück-stand säulenchromatographisch an Kieselgel gereinigt (EE/CH 1:1), worauf man 40 mg (101 µmol, 72 %) des Dipeptids 180 als farbloses Öl erhielt
**M (C₂₁H₃₂N₂O₅) = 392.4893.**
**R_{f}** = 0.35 (SiO₂, EE/CH 1:1).
**¹H-NMR** (250 MHz, CDCl₃): (Diastereomeren-/Rotamerengemisch) δ (ppm) = 5.82-6.03 (m, 1H, CH_{olef.}), 5.62-5.82 (m, 1H, CH_{olef.}), 5.37-5.49 (m, 1H, CH_{2,olef.}), 4.89-5.20 (m, 3H, CH_{2,olef.}), 4.42-4.63 (m, 1H), 4.10-4.28 (m, 3H), 3.32-3.73 (m, 2H), 2.76-2.93 (br, 1H), 1.60-2.50 (m, 7H), 1.40, 1.39 (2s, 9H, O*t*Bu), 1.24 (dt, 3H, J = 7.1 Hz, J = 3.1 Hz, CH_{3,Ester}).

### (3aS,5S,7aR/S,9aR)-3-(tert-Butoxycarbonyl)-4-oxo-1,3a,4,5,6,7,7a,9a-octahydro-2H-3,4a-diaza-cyclopenta[f]azulene-5-carbonsäure-ethylester (181)

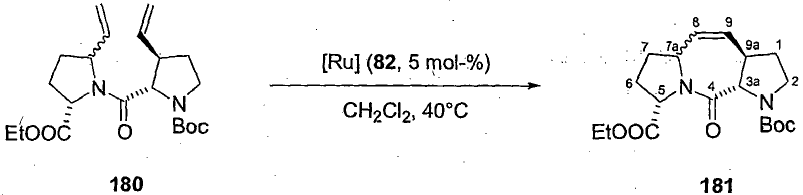

18 mg Dipeptid 180 (45.8 µmol) wurden in 2 ml CH₂Cl₂ (abs.) gelöst, mit 2.0 mg [Ru]_{gr} (3.0 µmol, ca. 6 mol-%) versetzt und über Nacht bei 40 °C am Rückfluss erhitzt. Nachdem die Vollständigkeit der Umsetzung auf dem Dünnschichtchromatogramm (EE/CH 2:1) überprüft worden war, wurde die Lösung auf RT gebracht, eingeengt und der verbleibende braune Rückstand säulenchromatographisch (EE/CH 2:1) gereinigt. Man erhielt 11 mg Metathese-Produkt 181 (30.2 µmol, 66%) als leicht bräunliches Öl.
M **(C₁₉H₂₈N₂O₅)** = 364.4361.
**R_{f}** = 0.17 (SiO₂, EE/CH 2:1).
**¹H-NMR** (500 MHz, CDCl₃): (Diastereomeren-/Rotamerengemisch) δ (ppm) = 5.80 (dd, 1H, J = 11.2 Hz, J = 1.7 Hz, H-8), 5.53 (dd, 1 H, J = 11.2 Hz, J = 1.6 Hz, H-7), 4.77 (dd, 0.6H, J = 7.2 Hz, J = 3.1 Hz, H-5), 4.67 (br, 1.4 H, H-5, H-7a), 4.34 (d, 0.7H, J =10.8 Hz, H-3a), 4.28 (d, 0.3H, J = 10.7 Hz, H-3a), 4.10 (m, 2H, CH_{2,Ester}), 3.73 (dd, 0.4H, J = 10.6 Hz, J = 8.2 Hz, H-2_{α}), 3.65 (dd, 0.6H, J = 10.6 Hz, J = 8.1 Hz, H-2_{α}), 3.36 (ddd, 1H, J = 15.5 Hz, J = 10.6 Hz, J = 4.8 Hz, H-2_{β}), 2.28 (br, 1H, H-6/H-7), 2.02 (m, 3H, H-1_{α}, H-6/H-7), 1.86 (m, 1H, H-6/H-7), 1.61 (m, 1H, H-1_{β}), 1.44, 1.38 (2s, 9H, O*t*Bu), 1.21 (m, 3H, CH_{3,Ester}).
**¹³C-NMR** (125 MHz, CDCl₃): δ (ppm) = 171.98 (C=O), 169.43 (C=O), 146.91 (C=O_{Boc}), 129.43, 129.17 (CH_{olef.}, C-9), 129.05, 128.70 (CH_{olef.}, C-8), 79.69 (C_{q,Boc}), 62.31, 62.09 (CH, C-3a), 61.17, 61.03 (CH_{2,Ester}), 59.61, 59.53 (CH, C-5), 57.26, 57.17 (CH, C-7a), 46.87, 4627 (CH₂, C-2), 33.09 (CH₂, C-6/C-7), 31.29, 30.81 (CH₂, C-1), 28.44, 28.15 (CH₃, *t*Bu), 27.21, 27.06 (CH₂, C-6/C-7), 14.10 (CH_{3,Ester}).

### (2S)-5-Oxo-pyrrolidin-2-carbonsäure-tert-butylester (206)

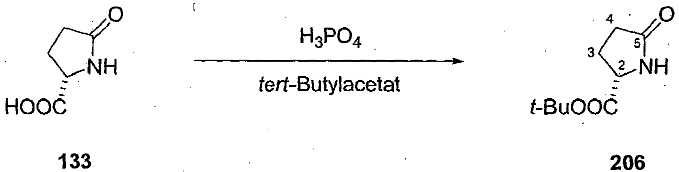

30.0 g *L*-Pyroglutaminsäure **133** (232 mmol) wurden in 500 ml *tert*-Butylessigester suspendiert und mit 12 ml 60%iger Perchlorsäure versetzt. Nach 15 h Rühren bei RT wurde die resultierende klare Lösung auf 0 °C gekühlt und durch Zugabe von 30.0 g festem NaHCO₃ auf einen pH-Wert von 4-5 gebracht. Nach Abfiltrieren des Feststoffes wurde das Filtrat vollständig eingeengt, wodurch man 25.4 g (136 mmol, 58 %) *tert*-Butylester **206** als weißen Feststoff erhielt, der ohne weitere Aufreinigung umgesetzt wurde. Eine analytische Probe wurde durch Säulenchromatographie an Kieselgel (Eluent: EE) gewonnen.
**M (C₉H₁₅NO₃)** =**185.2203.**
**R_{f}** = 0.25 (SiO₂, EE).
Smp.: 97 - 98 °C. Lit.: 98 - 99 °C.
**¹H-NMR** (300 MHz, CDCl₃): δ (ppm) = 6.13 (br, 1H, NH), 4.06-4.14 (m, 1H, H-2), 2.22-2.43 (m, 3H, H-3_{α}, H-4), 2.04-2.20 (m, 1H, H-3_{β}), 1.44 (s, 9H, O*t*Bu).
**¹³C-NMR** (75 MHz, CDCl₃): δ (ppm) = 177.97 (C=O_{Lactam}), 171.07 (C=O_{Ester}), 82.18 (C_{q,Boc}),56.07 (CH, C-2), 29.34 (CH₂, C-4), 27.87 (CH₃, O*t*Bu), 24.75 (CH₂, C-3).
**IR** (ATR) *ṽ* (cm⁻¹) = 3239 (br), 2976 (s), 2934 (m), 1731 (vs), 1695 (vs), 1457 (s), 1419 (s), 1392 (s), 1366 (vs), 1227 (vs), 1147 (vs), 1037 (m), 1009 (m), 971 (m), 953 (m), 885 (w), 845 (s), 810 (s).
**MS** (GCMS-EI, 70 eV): m/z (%) = 186 ([MH⁺], <1), 142 (<1), 84 (100), 57 (38).

### (2S)-1-(tert-Butoxycarbonyl)-5-oxo-pyrrolidin-2-carbonsäure-tert-butylester (44)

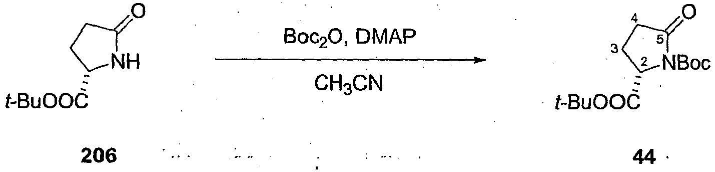

Eine Lösung von 20.0 g Lactam **206** (108 mmol) in 200 ml Acetonitril (abs.) wurde bei 0 °C mit 25.6 ml Boc₂O (120 mmol, 1.1 Äq.) und 100 mg DMAP (0.81 mmol, 0.75 mol-%) versetzt und anschließend über Nacht bei RT gerührt, wobei sich die Lösung intensiv orange färbte. Zur Aufarbeitung wurde die Lösung vollständig eingeengt und der verbleibende Rückstand in 250 ml eines 1:1-Gemisches aus Ethylacetat/Cyclohexan aufgenommen und über eine kurze Kieselgelsäule filtriert. Nach Entfernen des Lösungsmittels im Vakuum blieb ein gelblicher öliger Rückstand, der aus Diethylether/Pentan zur Kristallisation gebracht werden konnte. Man erhielt 25.9 g *N*-Boc-Lactam **44** (90.7 mmol, 84 %) in Form kleiner weißer Kristalle.
**M (C₁₄H₂₃NO₅) = 285.3362.**
**R_{f}**=0.20 (SiO₂, EE/CH 1:2).
Smp.: 52 - 53 °C. Lit.: 55 - 56 °C.
**¹H-NMR** (300 MHz, CDCl₃): δ (ppm) = 4.45 (dd, 1H, J = 9.3 Hz, J = 2.6 Hz, H-2), 2.17-2.66 (m, 3H, H-3_{α}, H-4), 1.9-2.0 (m, 1 H, H-3_{β}), 1.48 (s, 9H, O*t*Bu), 1.46 (s, 9H, O*t*Bu).
**¹³C-NMR** (75 MHz, CDCl₃): δ (ppm) =173.27 (C=O_{Lactam}), 170.07 (C=O_{Ester}), 148.94 (C=O_{Boc}), 82.84 (Cq, *t*Bu), 81.86 (Cq, *t*Bu), 59.25 (CH, C-2), 30.78 (CH₂, C-4), 27.59 (CH₃, O*t*Bu), 21.30 (CH₂, C-3).
**IR** (ATR) *ṽ* (cm⁻¹) =2977 (s), 2931 (m), 1790 (vs), 1738 (vs), 1715 (vs), 1476 (m), 1457 (m), 1392 (m), 1367 (vs), 1307 (vs), 1285 (vs), 1254 (vs), 1223 (s), 1148 (vs), 1045 (m), 1021 (m), 962 (w), 912 (w), 842 (s), 774 (m), 643 (w).
**MS** (GCMS-EI, 70 eV): m/z (%) = 270 ([M⁺-CH₃], 6), 239 (4), 227 (9), 207 (4), 185 (3), 171 (6), 153 (2), 143 (15),129 (5), 101 (4), 97 (7), 87 (72), 74 (100), 57 (12), 55 (14).
**[α]_{D}²⁰** = -35.3° (c = 1.167, CHCl₃). Lit.: -35.7° (c = 0.9, CHCl₃).

### (2S,5R/S)-1-(tert-Butoxycarbonyl)-5-hydroxy-pyrrolidin-2-carbonsäure-tert-butylester (207)

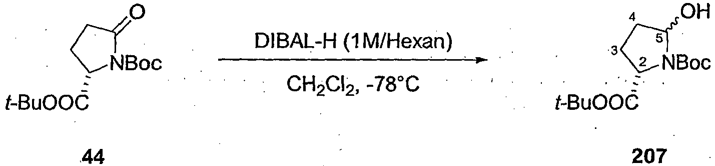

Zu einer Lösung von 13.0 g (45.6 mmol) 44 in 150 ml THF (abs.) wurden bei -78 °C über einen Zeitraum von 30 min. 100 ml DIBAL-H (1M/Hexan, 100 mmol, 2.2 Äq.) zugetropft und die Lösung nach beendeter Zugabe noch zwei Stunden gerührt. Dann wurde das überschüssige Reagenz durch Zugabe von 7 ml Isopropanol zerstört (Wasserstoff-entwicklung!). Man gab weiterhin 200 ml K-Na-Tartrat-Lösung (1.77M) zu und ließ über Nacht langsam auf RT auftauen. Anschließend wurde der Ansatz in einen Scheidetrichter überführt und mit 300 ml MTBE und 100 ml Wasser verdünnt. Nach Phasentrennung wurde die wäßrige Phase mit 2x 100 ml MTBE extrahiert und anschließend die vereinigten organischen Phasen über MgSO₄ getrocknet. Nach Filtration über einen kurze Kieselgelsäule wurde das Lösungsmittel im Vakuum entfernt, und man erhielt 12.2 g α-Hydroxycarbamat 207 (42.3 mmol, 93%) als farbloses Öl, das ohne weitere Aufreinigung umgesetzt wurde. Eine analytische Probe wurde durch Säulenchromatographie an Kieselgel (EE/CH 1:2) gewonnen.
**M (C₁₄H₂₅NO₅) = 287.3520.**
**R_{f}** = 0.14/0.28 (SiO₂, EE/CH 1:2), 2 Diastereomere.
**¹H-NMR** (300 MHz, CDCl₃): (Diastereomeren-/Rotamerengemisch) δ (ppm) = 5.37-5.60 (m, 1H, H-5), 4.02-4.25 (m, 1H, H-2), 3.75 (br, 0.4H, OH), 3.52 (br, 0.4H, OH), 3.14 (br, 0.2H, OH), 1.73-2.48 (m, 4H, H-3, H-4), 1.35-1.45 (m, 18H, 2x *t*Bu)
**¹³C-NMR** (75 MHz, CDCl₃): (Diastereomeren-/Rotamerengemisch) δ (ppm) = 172.06,171.53 (C=O_{Ester}), 154.21,153.83 (C=O_{Boc}), 82.38, 82.09, 81.78 (CH, C-5), 81.15, 80.94, 80.61, 80.55 (C_{q}, 2x *t*Bu), 59.91, 59.82 (CH, C-2), 33.51, 32.17, 31.74, 30.87 (C-4), 28.33, 28.18, 27.87 (CH₃, 2x *t*Bu), 27.77, 26.99, 26.81 (CH₂, C-3).
**IR** (ATR) *ṽ* (cm⁻¹) = 3460 (br), 2974 (m), 2928 (w), 1740 (s), 1701 (vs), 1477 (m), 1454 (m), 1365 (vs), 1325 (m), 1255 (m), 1220 (m), 1149 (vs), 1080 (m), 1061 (m), 1042 (m), 1009 (m), 973 (m), 914 (m), 843 (m), 774 (m), 758 (m).
MS (GCMS-EI, 70 eV): m/z (%) = 186 (2), 158 (2),130 (12),86 (18), 68 (7), 57 (43), 41 (100).

### (2S,5R/S)-1-(tert-Butoxycarbonyl)-5-ethoxy-pyrrolidin-2-carbonsäure-tert-butylester (45)

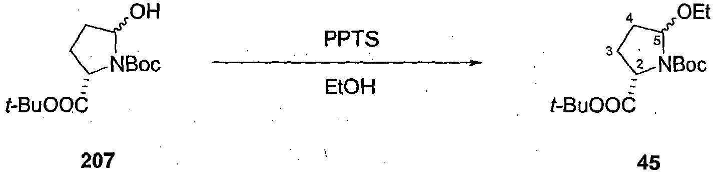

Eine Lösung von 12.1 g 207 (42.1 mmol) in 100 ml EtOH (abs.) wurde mit 200 mg PPTS (0.80 mmol, 1.9 mol-%) versetzt und bei RT über Nacht gerührt. Anschließend wurde das Lösungsmittel im Vakuum vollständig entfernt und der Rückstand in 250 ml eines 1:2-Gemisches aus EE/CH aufgenommen. Nach Filtration über einen kurze Kieselgelsäule wurde das Eluat eingeengt, worauf man 12.8 g α-Methoxycarbamat 45 (40.5 mmol, 96 %) als leicht gelbliches Öl erhielt. Eine analytische Probe wurde durch Säulenchromatographie an Kieselgel (EE/CH 1:2) gewonnen.
**M (C₁₆H₂₉NO₅) = 315.4052.**
**R_{f}** = 0.39/0.52 (SiO₂, EE/CH 1:2), zwei Diastereomere.
**¹H-NMR** (300 MHz, CDCl₃): (Diastereomeren-/Rotamerengemisch) δ (ppm) = 5.34 (d, 0.35H, J = 4.7 Hz, H-5), 5.30 (d, 0.35H, J = 4.7 Hz, H-5), 5.21 (d, 0.1 H, J = 4.4 Hz, H-5), 5.14 (d, 0.2H, J = 4.9 Hz, H-5), 4.02-4.23 (m, 1H, H-2), 3.38-3.80 (m, 2H, CH_{2,Ethyl}), 1.58-2.48 (m, 4H, H-3, H-4), 1.35-1.45 (m, 18H, 2x O*t*Bu), 1.08-1.16 (m, 3H, CH_{3,Ethyl}).
**¹³C-NMR** (75 MHz, CDCl₃): (Diastereomeren/Rotamerengemisch) δ (ppm) = 171.77, 171.68, 171.49 (C=O_{Ester}). 154.44, 154.17, 153.79 (C=O_{Boc}), 87.86,86.96,86.84 (CH, C-5), 80.86, 80.57, 80.25, 80.16 (C_{q}, 2x *t*Bu), 64.10,63.47,62.83,62.27 (CH_{2,Ethyl}), 60.31,59.95,59.82 (CH, C-2), 33.07, 32.38, 31.33, 30.39, 27.04, 26.92 (CH₂, C-3, C-4), 28.26, 28.18, 28.14, 27.92, 27.84 (CH₃, 2x *t*Bu), 15.41, 15.18 (CH_{3,Ethyl}).
**IR** (ATR) *ṽ* (cm⁻¹) = 2973 (s), 2929 (m), 2873 (w), 1739 (s), 1700 (vs), 1477 (m), 1455 (m), 1363 (vs), 1326 (s), 1255 (s), 1219 (s), 1150 (vs), 1121 (vs), 1078 (vs), 1031 (m), 997 (m), 971 (s), 945 (m), 892 (w), 842 (s), 772 (m).
**MS** (GCMS-EI, 70 eV): m/z (%) = 270 ([M⁺ -OEt], 2), 214 (12), 186 (4), 170 (6), 158 (15), 142 (4), 114 (100), 96 (2), 84 (2), 68 (40), 57 (79).

### (2S,5R/S)-5-Allyl-1-(tert-butoxycarbonyl)-pyrrolidin-2-carbonsäure-tert-butylester (46)

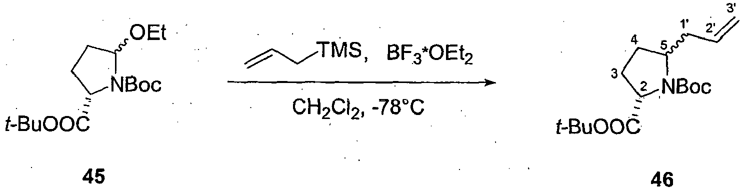

Eine Lösung von 12.6 g α-Methoxycarbamat 45 (40.0 mmol) und 16.0 ml Allyltrimethyl-silan (100 mmol, 2 5 Äq.) in 100 ml CH₂Cl₂ (abs.) wurde auf-78 °C gekühlt und tropfenweise mit 10.1 ml BF₃·OEt₂ (80.0 mmol, 2 Äq.) versetzt. Nach beendeter Zugabe wurde noch 30 min. gerührt und die Vollständigkeit der Reaktion per Dünnschicht-chromatogramm (EE/CH 19) überprüft. Dann wurden 10 ml ges. NaHCO₃-Lsg. zugegeben und der Ansatz auf RT gebracht Nach Überführung in einen Scheidetrichter mit 200 ml MTBE wurden die Phasen getrennt und die wäßrige Phase mit 2x 100 ml MTBE extrahiert Die vereinigten organischen Phasen wurden mit 100 ml halbkonz. NaCl-Lsg. gewaschen, über MgSO₄ getrocknet und eingeengt. Der Rückstand wurde säulenchromatographisch an Kieselgel aufgereinigt (EE/CH 1:9), wobei man 9.62 g Allyl-Substitutionsprodukt **46** (30.9 mmol, 77%) als farbloses Öl erhielt (Epimerengemisch, *cis*/*trans* 75:25)
**M (C₁₇H₂₉NO₄) = 311.4165.**
**R_{f}**=0.30 (SiO₂, EE/CH 1:9)
**¹H-NMR** (300 MHz, CDCl₃). (Diastereomeren-/Rotamerengemisch) δ (ppm) = 5 72 (m, 1H, H-2'_{olef.}), 5.01 (m, 2H, H-3'_{olef.}), 4.08 (m, 1H, H-2/H-5), 3.82 (br, 1H, H-2/H-5), 2.31-2.76 (m, 1H, H-1'_{α}),1.55-2.22 (m, 5H, H-1'_{β}, H-3, H-4),1.40 (m, 18H, 2x *t*Bu)
**¹³C-NMR** (75 MHz, CDCl₃): (Diastereomeren-/Rotamerengemisch) δ (ppm) = 172.33, 172-13, 172.04 (C=O_{Ester}), 154.24, 153.90, 153.75, 153.63 (C=O_{Boc}), 135.48, 135.19, 135.12 (CH_{olef.}, C-2'), 117.04, 116.99, 116.63 (CH_{2,olef.}, C-3'), 80.72, 80.66 (Cq, *t*Bu), 79.62, 79.50 (Cq, *t*Bu), 60.74, 60 58 (CH, C-2/C-5), 58.06, 57.42 (CH, C-2/C-5), 39.11, 38 97, 3814, 38.10 (CH₂, C-1'), 29.34, 28.80, 28.42 (CH₂, C-3/C-4), 28.31, 28.25 (CH₃, *t*Bu), 27.93, 27.86 (CH₃, *t*Bu), 27.42, 26.62 (CH₂, C-3/C-4).
**IR** (ATR) *ṽ* (cm⁻¹) = 3075 (w), 2973 (s), 2929 (m), 1739 (vs), 1695 (vs), 1639 (m), 1477 (s), 1454 (s), 1387 (vs), 1363 (vs), 1294 (s), 1255 (s), 1216 (s), 1149 (vs), 1103 (vs), 1031 (s), 995 (s), 969 (s), 948 (s), 910 (vs), 856 (s), 842 (s), 771 (s), 633 (m)
**MS** (GCMS-EI, 70 eV): m/z (%) = 270 ([M⁺-C₃H₅], 8), 210 (5),182 (8),170 (29), 154 (61), 114 (100), 110 (40), 96 (3), 68 (21), 57 (81)
**[α]_{D}²⁰** = -34.6° (c = 0.985, CHCl₃). Lit. *cis:* -24.3° (c = 4.36, CHCl₃), *trans*: -69.3° (c =1.07, CHCl₃).

### (2S,5R/S)-1-(tert-Butoxycarbonyl)-5-(2-hydroxyethyl)-pyrrolidin-2-carbonsäure-tert-butylester (184)

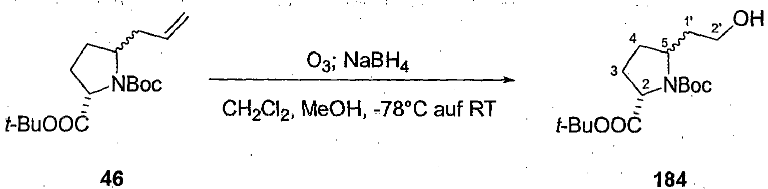

Durch eine Lösung von 4.50 g **46** (14.5 mmol) in einem Gemisch aus 38 ml CH₂Cl₂ und 75 ml MeOH wurde bei -78 °C Ozon geleitet, bis eine erkennbare Blaufärbung eintrat. Dann wurde die Reaktion abgebrochen und das überschüssige Ozon durch Spülen mit Sauerstoff verdrängt. Anschließend wurde die farblose Reaktionslösung mit 1.10 g NaBH₄ (28.9 mmol, 2 Äq.) versetzt und über Nacht gerührt, wobei sie auf RT auftaute. Nach Überprüfung der vollständigen Reaktion durch Dünnschichtchromatogramm (EE/CH 1:2) wurde der Ansatz vollständig eingeengt, in 200 ml MTBE aufgenommen und über wenig Kieselgel filtriert. Der eingeengte Rückstand wurde chromatographisch an Kieselgel aufgereinigt (EE/CH 1:2), wobei man 3.96 g des Alkohols **184** (12.6 mmol, 87 %) als farbloses Öl isolierte (Epimerengemisch).
**M (C₁₆H₂₉NO₅) = 315.4052.**
**R_{f}** =0.22 (SiO₂, EE/CH 1:2).
**¹H-NMR** (300 MHz, CDCl₃): (Diastereomeren-/Rotamerengemisch) δ (ppm) = 3.92-4.34 (m, 3H, H-2, H-5, OH), 3.45-3.80 (m, 2H, H-2'),1.49-2.39 (6H, m, H-3, H-4, H-1'), 1.40, 1.41, 1.42, 1.43 (4s, 18H, 2x *t*Bu).
**¹³C-NMR** (75 MHz, CDCl₃): (Diastereomeren-/Rotamerengemisch) ö (ppm) = 172.21 (C=OEster), 155.86, 155.54 (C=O_{Boc}), 81.03, 80.98, 80.67, 80.49 (C_{q,Boc}), 60.76, 60.68 (CH, C-2), 59.05, 58.85 (CH₂, C-2'), 54.58,54.20 (CH, C-5), 39.11, 37.54 (CH₂, C-3/C-4), 30.41, 29.07, 28.94, 28.77 (CH₂, C-3/C-4), 28.23, 27.96 (CH₃, 2x *t*Bu).
**IR** (ATR) *ṽ* (cm⁻¹) = 3452 (br), 2973 (s), 2934 (m), 2877 (m), 1737 (vs), 1673 (vs), 1477 (s),1454 (s), 1390 (vs),1364 (vs),1340 (s),1297 (s), 1255 (s),1215 (s),1150 (vs), 1116 (vs),1069 (s), 987 (s), 928 (m), 899 (m), 884 (m), 854 (m), 840 (m), 772 (m), 736 (m).
**MS** (DIP-EI, 70 eV): m/z (%) = 315 ([M⁺], <1), 214 (13),186 (4),158 (10),114 (100), 68 (8), 57 (49).
**[α]_{D}²⁰**=-43.3°(c=0.915, CHCl₃). Lit: *cis*: -48.2° (c=0.51, CHCl₃), *trans*: -31.2°
(c=1.12, CHCl₃).

### (2S,5R/S)-1-(tert-Butoxycarbonyl)-5-[2-(2-nitro-phenylselanyl)-ethyl]-pyrrolidin-2-carbonsäure-tert-butylester (47)

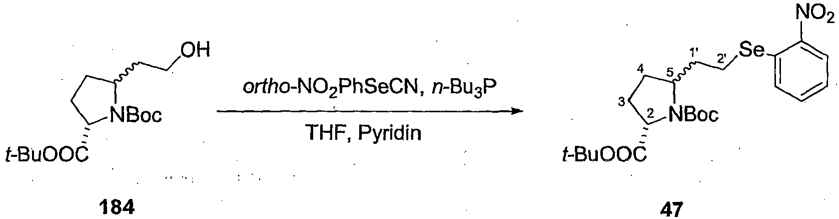

Zu einer Lösung von 4.18 g Alkohol **184** (13.3 mmol) in 50 ml THF (abs.) und 17 ml Pyridin (abs.) wurden 3.31 g *ortho*-Nitrophenylselenocyanat (14.6 mmol, 1.1 Äq.) hinzugefügt. Unter Kühlung im Eisbad wurden 4.30 ml *n*-Tributylphophin (17.3 mmol, 1.3 Äq.) langsam zugetropft, wobei eine dunkelrote Lösung entstand. Nach 30 min. Rühren bei RT wurde der Ansatz mit 200 ml MTBE verdünnt und über wenig Kieselgel filtriert. Nach Einengen des Filtrats erhielt man einen bräunlich-gelben Rückstand, der säulen-chromatographisch an Kieselgel (EE/CH 1:4) aufgereinigt wurde. Man isolierte 6.06 g Selenylether 47 (12.1 mmol, 92 %) als intensiv gelb gefärbtes Öl.
**M (C₂₂H₃₂N₂O₆Se) = 499.4594.**
**R_{f}=** 0.14 (SiO₂, EE/CH 1:4).
**¹H-NMR** (300 MHz, CDCl₃): (Diastereomeren-/Rotamerengemisch)δ (ppm) =8.20-8.28 (m, 1H, PhH), 7.43-7.67 (m, 2H, PhH), 7.20-7.33 (m, 1H, PhH), 3.92-4.33 (m, 2H, H-2, H-5), 2.73-3.12 (m, 2H, H-1'/H-2'), 1.60-2.32 (m, 6H, H-3, H-4, H-1'/H-2'), 1.44, 1.43, 1.42, 1.40 (4s, 18H, 2x *t*Bu).
**¹³C-NMR** (75 MHz, CDCl₃): (Diastereomeren-/Rotamerengemisch) δ (ppm) = 172.28, 171.94 (C=O_{Ester}). 154.19, 154.15 (C=O_{Boc}), 146.68(C_{q,ar}), 133.82 (C_{q,ar}), 133.60 (CHₐᵣ),129.95, 129.85, 129.21, 128.99, 128.67 (CHₐᵣ), 126.29, 125.22, 125.06 (CHₐᵣ), 80.99, 80.91 (C_{q}, *t*Bu), 80.09, 79.94, 79.86 (C_{q}, *t*Bu), 60.60,60.43 (CH, C-2/C-5), 58.62, 58.29, 57.83 (CH, C-2/C-5), 34.00, 33.55, 33.34 (CH₂), 30.33, 29.93 (CH₂), 28.99, 28.74 (CH₂), 28.40, 28.29 (CH₃, *t*Bu), 27.96 (CH₃, *t*Bu), 27.83, 27.64 (CH₂), 22.95, 22.75, 22.53 (CH₂).

### (2S,5R/S)-1-(tert-Butoxycarbonyl)-5-vinyl-pyrrolidin-2-carbonsäure-tert-butylester (185)

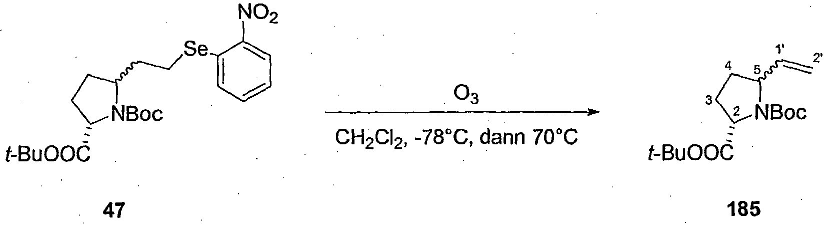

Durch eine Lösung von 5.80 g Selenylether **47** (11.6 mmol) in 150 ml CH₂Cl₂ wurde bei -78 °C Ozon geleitet, bis eine erkennbare Grünfärbung eintrat. Dann wurde die Reaktion abgebrochen und das überschüssige Ozon durch tropfenweise Zugabe von 1-Hexen entfernt (insgesamt 30 ml), wobei die Farbe von grün nach gelb umschlug. Anschließend wurde der Ansatz, ohne auf Raumtemperatur aufgetaut zu sein, zu 200 ml Hexan bei 50 °C zugetropft und nachfolgend 30 min. zum Rückfluss erhitzt. Die orangefarbene Lösung wurde eingeengt und der verbleibende, intensiv orangefarbene Rückstand doppelt säulenchromatographisch gereinigt (CH₂Cl₂/MeOH 100:1, anschließend EE/CH 1:7). Man erhielt 3.14 g Olefin **185** (10.6 mmol, 90 %) als gelbliches Öl (Epimerengemisch).
**M (C₁₆H₂₇NO₄) = 297.3899.**
**R_{f}** = 024 (SiO₂, EE/CH 1:7).
**¹H-NMR** (300 MH₄ CDCl₃): (Diastereomeren-/Rotamerengemisch) δ (ppm) = 5.62-5.94 (m, 1H, H-1'), 4.96-5.42 (m, 2H, H-2'), 4.05-4.55 (m, 2H, H-2, H-5), 1.60-2.22 (m, 4H, H-3, H-4),1.45,1.43 (2s, 9H, *t*Bu), 1.41 (br, 9H, *t*Bu).
**¹³C-NMR** (75 MHz, CDCl₃): (Diastereomeren-/Rotamerengemisch) δ (ppm) = 172.05, 171.99, 171.92 (C=O_{Ester}), 154.24, 154.08, 153.66, 153.54 (C=O_{Boc}), 139.21, 138.60, 138.46, 138.03 (CH_{olef.}, C-1'), 114.88, 114.52, 113.77, 113.60 (CH_{2,olef.}, C-2'), 80.79, 79.80, 79.67 (C_{q,Boc}), 60.95, 60.49, 60.30, 60.14, 59.58, 59.32 (CH, C-2, C-5), 31.51, 30.81, 29.88, 28.95 (CH₂, C-3/C-4), 28.28 (CH₃, *t*Bu), 27.94 (CH₃, *t*Bu), 27.29 (CH₂, C-3/C-4).
**IR** (ATR) *ṽ* (cm⁻¹) = 3077 (w), 2974 (s), 2928 (m), 2873 (w), 1739 (vs), 1696 (vs), 1643 (w), 1477 (m), 1454 (m), 1387 (vs), 1364 (vs), 1325 (m), 1293 (m), 1255 (s), 1214 (s), 1150 (vs), 1067 (m), 1024 (w), 988 (m), 957 (m), 912 (s), 875 (w), 856 (m), 843 (m), 770 (m), 685 (w).
**MS** (GCMS-EI, 70 eV): m/z (%) = (297 [M⁺], <1), 241 (2), 224 (1), 196 (28),168 (9),140 (100),114 (2), 96 (82), 79 (5), 67 (6), 57 (83).
[**α]_{D}²⁰**=-54.6° (c=0.935, CHCl₃). Lit.: cis: -56.0° (c =1.1, CHCl₃), *trans:* -50.6° (c = 0.77, CHCl₃).

### (2S,5/R)-5-Vinyl-pyrrolidin-2-carbonsäure-tert-butylester (186a)

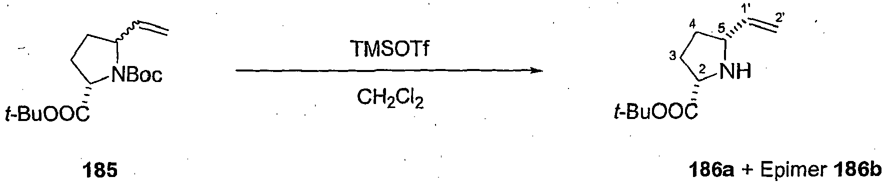

Eine Lösung von 800 mg **185** (2.69 mmol) in 10 ml CH₂Cl₂ (abs.) wurde bei 0 °C tropfenweise mit 0.53 ml TMSOTf (2.74 mmol, 1.01 Äq.) versetzt. Nach 5 min. wurde die Vollständigkeit der Reaktion per DC (CH_{z}Cl₂/MeOH 25:1) überprüft und die Reaktionslösung durch Zugabe von 2 ml ges. NaHCO₃-Lsg. gequencht. Zur Aufarbeitung wurde die organische Phase mit 50 ml Et₂O verdünnt, von der wäßrigen Phase abgetrennt, über MgSO₄ getrocknet und eingeengt Der Rückstand wurde durch sorgfältige Chromatographie an Kieselgel (CH₂Cl₂/MeOH 25:1) gereinigt, wobei zunächst das *trans*-Isomer eluiert wurde, dicht gefolgt von dem erwünschten *cis*-Isomer. Man isolierte 104 mg einer Mischfraktion (0.53 mmol, 20 %) und 270 mg reines *cis*-Isomer (1.37 mmol, 51 %) jeweils als leicht gelbliches Öl.
**M (C₁₁H₁₉NO₂)=197.2741.**
**R_{f}**=0.16/0.22 (*cis*/trans-Isomer, SiO₂, CH₂Cl₂/MeOH 25:1).
**¹H-NMR** (500 MHz, CDCl₃): δ (ppm) = 5.82 (ddd, 1H, J = 17.2 Hz, J = 10.2 Hz, J = 7.1 Hz, H-1'), 5.13 (d, 1 H, J = 17.1 Hz, H-2'_{α}), 4.99 (d, 1H, J = 10.0 Hz, H-2'_{β}), 3.61 (dd, 1H, J = 7.4 Hz, J = 5.8 Hz, H-2), 3.54 (dd, 1H, J = 14.0 Hz, J = 7.0 Hz, H-5), 2.10 (s, 1H, NH), 1.98-2.08 (m, 1H, H-3_{α}), 1.81-1.91 (m, 2H, H-3_{β}, H-4_{α}) 1.44 (s, 10H, H-4_{β}, *t*Bu).
**¹³C-NMR** (125 MHz, CDCl₃): ö (ppm) =174.46 (C=O), 140.23 (CH_{olef.}, C-1'), 114.97 (CH_{2,olef.}, C-2'), 80.97 (Cq, *t*Bu), 62.24 (CH, C-5), 60.76 (CH, C-2), 31.92 (CH2, C-4), 30.39 (C-3), 28.03 (CH₃, *t*Bu).
**IR** (ATR) *ṽ* (cm⁻¹) = 3075 (w), 2974 (m), 2870 (w), 1726 (vs), 1640 (w), 1477 (w), 1457 (w), 1425 (w), 1391 (w), 1367 (s), 1282 (w), 1226 (s), 1156 (vs), 1102 (m), 1034 (w), 991 (m), 917 (m), 848 (m), 757 (m).
**MS** (GCMS-EI, 70eV): m/z (%) = 197 ([M⁺], <1), 114 (1), 96 (100), 79 (12), 68 (7), 57 (6).
**HRMS** (ESI, C₁₁H₂₀NO₂) ber.: 198.1494, gef.: 198.149.
**[α]_{λ}²⁰** = -35.8° (589 nm), -41.6° (546 nm), -81.7° (405 nm), -104.2° (365 nm), -130.4° (334 nm) (c= 1.025, CHCl₃).

### (2S,2'S,3'R,5R)-5-Vinyl-1-[1'-((tert-butoxycarbonyl)-3'-vinyl-pyrrolidin-2'-yl)-carbonyl]-pyrrolidin-2-carbonsäure-tert-butylester (187)

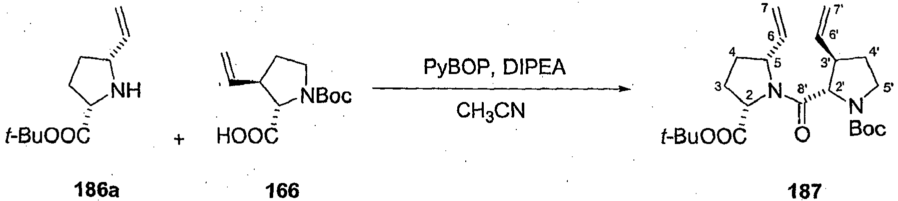

Eine Lösung von 200 mg Carbonsäure **166** (0.83 mmol, 1 Äq.) und 200 mg Amin **186a** (1.01 mmol, 1.24 Äq.) in 5 ml Acetonitril (abs.) wurde bei 0 °C mit einer Lösung von 512 mg PyBOP (0.98 mmol, 1.19 Äq.) in 5 ml Acetonitril (abs.) versetzt, gefolgt von 180 µl DIPEA (1.03 mmol, 1.24 Äq.). Nach Rühren über Nacht bei Raumtemperatur wurde das Lösungmittel vollständig entfernt und der verbleibende Rückstand in 20 ml MTBE und 5 ml Wasser aufgenommen. Nach Phasentrennung wurde die wäßrige Phase zusätzlich mit MTBE (3x 3 ml) extrahiert und die vereinigten organischen Phasen über MgSO₄ getrocknet Nach säulenchromatographischer Aufreinigung an Kieselgel erhielt man 282 mg Dipeptid **187** (0.67 mmol, 81 %) als farbloses Öl.
**M (C₂₃H₃₆N₂O₅) = 420.5424.**
**R_{f}**=0.19 (SiO₂, EE/CH 1:3).
**¹H-NMR** (500 MHz, CDCl₃): (Rotamerengemisch) δ (ppm) = 5.91 (m, 1H, H-6), 5.70 (m, 1H, H-6'), 5.40 (dd, 1H, J = 172 Hz, J = 5.4 Hz, H-7_{α}), 5.11 (m, 1H, H-7_{β}), 4.99 (m, 2H, H-7'), 4.87 (t, 0.6H, J = 6.3 Hz, H-5), 4.54 (t, 0.4H, J = 6.3 Hz, H-5), 4.36 (t, 1H, J = 8.0 Hz, H-2), 4.24 (d, 0.6H, J = 2.1 Hz, H-2'), 4.15 (s, br, 0.4H, H-2'), 3.66 (m, 0.4H, H-5_{α}'), 3.53 (m, 0.6H, H-5_{α}'), 3.37 (m, 1H, H-5_{β}'), 2.82 (m, 1H, H-3'), 2.37 (m, 1 H, H4_{α}'), 1.72-223 (m, 4H, H-3, H-4), 1.66 (m, 1 1H, H-4_{β}'), 1.44, 1.43 (2s, 9H, *t*Bu), 1.39, 1.38 (2s, 9H, *t*Bu).
**¹³C-NMR** (125 MHz, CDCl₃): (Rotamerengemisch) δ (ppm) =172.26, 172.15 (C=O), 171.30, 171.05 (C=O), 154.45, 153.63 (C=O_{Boc}), 139.05 (CH_{olef.}, C-6), 138.68 (CH_{olef.}, C-6'), 116.76/ 116.37 (CH_{2,olef.}, C-7),114.64,114.61 (CH_{2,olef.}, C-7'), 81.17, 80.85 (Cq), 79.58, 79.34 (Cq), 61.79, 61.57 (CH, C-2'), 61.04 (CH, C-2), 60.91, 60.80 (CH, C-5), 46.95, 46.18 (CH, C-3'), 45.90, 45.56 (CH₂, C-5'), 32.81, 32.58 (CH₂, C-3/C-4), 29.66 (CH₂, C-4'), 28.52, 28.47 (CH₃, *t*Bu), 28.37 (CH₂, C-4'), 27.96 (CH₃, *t*Bu), 26.99, 26.96 (CH₂, C-3/C-4).
**IR** (ATR) *ṽ* (cm⁻¹) = 3079 (w), 2973 (s), 2930 (m), 2876 (m), 1737 (s), 1690 (vs), 1656 (vs), 1477 (m), 1390 (vs), 1364 (vs), 1320 (m), 1301 (m), 1255 (m), 1212 (m),1155 (vs), 1116 (s),1067 (m), 1030 (m), 987 (m), 911 (s), 864 (m), 841 (m), 770 (m), 734 (m).
**MS** (DIP-EI, 70 eV): m/z (%) = 420 ([M⁺], <1), 347 (4), 308 (3), 291 (2), 263 (5), 222 (5), 196 (11),168 (6),140 (100), 124 (3), 96 (94), 79 (7), 67 (8), 57 (58).
**HRMS** (EI, C₂₃H₃₆N₂O₅): ber.: 420.2624, gef.: 420.262.
**[α]_{λ}²⁰** = -6.7° (589 nm), -8.3° (546 nm), -18.1° (405 nm), -24.7° (365 nm), -34.0° (334 nm) (c=0.710, CHCl₃).

### (3aS,5S,7aR,9aR)-3-(tert-Butoxycarbonyl)-4-oxo-1,2,3,3a,4,5,6,7,7a,9a-decahydro-3,4a-diaza-cyclopenta[f]azulene-5-carbonsäure-tert-butylester (188)

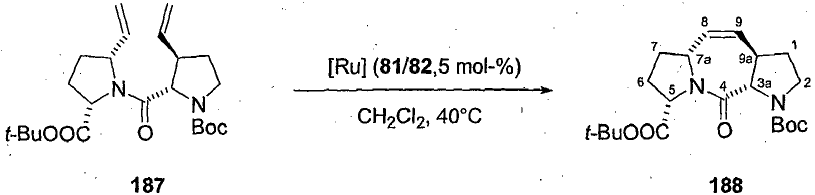

Eine Lösung von 250 mg Dipeptid 187 (0.59 mmol) und 25 mg [Ru] (81) (29.45 µmol, 5 mol-%) in 8 ml CH₂Cl₂ (abs.) wurde unter Argon-Schutzatmospäre über Nacht unter Rückfluss erhitzt, bis die Reaktion It. DC (EE/CH 1:1) vollständig war. Nach Abkühlen auf RT wurde die Reaktion zunächst mit 1 ml Ethyl-Vinyl-Ether versetzt und 30 min. gerührt. Dann wurden 365 mg Tris-(hydroxymethyl)-phosphin (2.94 mmol,100 Äq. bezogen auf Katalysator) und 200 µl DIPEA zugegeben, worauf sich die Farbe der Lösung sichtbar von braun zu hellgelb änderte. Zur Aufarbeitung wurden 50 ml MTBE und 5 ml Wasser zugegeben; die Phasen getrennt und die organische Phase über MgSO₄ getrocknet. Nach säulench-romatographischer Aufreinigung an Kieselgel (EE/CH 1:1) erhielt man 210 mg des Tricyclus 188 (0.54 mmol, 92 %) als farbloses Öl, das allmählich zu kleinen farblosen Kristallen erstarrte.
**M (C₂₁H₃₂N₂O₅) = 392.4893.**
**R_{f}**=0.20 (SiO₂, EE/CH 1:1).
**Smp.:147** - 149 °C.
**¹H-NMR** (500 MHz, CDCl₃): (Rotamerengemisch) δ (ppm) = 5.84 (d, 1H, J = 11.2 Hz H-8), 5.58 (d, 1H, J = 11.1 Hz, H-9), 4.68 (m, 2H, H-5, H-7a), 4.37 (d, 0.6H, J = 10.8 Hz, H-3a), 4.32 (d, 0.4H, J = 10.7 Hz, H-3a), 3.69 (m, 1H, H-2_{α}), 3.43 (m, 1H, H-2_{β}), 2.98 (m, 1H, H-9a), 2.32 (m, 1H, H-6/H-7), 2.07 (m, 3H, H-1_{α}, H-6/H-7)), 1.88 (m, 1H, H-6/H-7), 1.66 (m, 1H, H-1_{β}), 1.48,1.47 (2s, 9H, O*t*Bu), 1.44, 1.42 (2s, 9H, O*t*Bu).
**¹³C-NMR** (125 MHz, CDCl₃): (Rotamerengemisch) δ (ppm) = 171.11, 170.94 (C=O), 169.49, 169.30 (C=O), 154.68, 154.18 (C=O_{Boc}), 129.53, 129.32 (CH_{olef.}, C-9), 128.95, 128.60 (CH_{olef.}, C-8), 81.34, 81.13 (C_{q}, O*t*Bu), 79.67, 79.60 (C_{q}, O*t*Bu), 62.27, 62.05 (CH, C-3a), 60.22, 60.15 (CH, C-5/C-7a), 57.22, 57.11 (CH, C-5/C-7a), 46.90, 46.28 (CH₂, C-2), 41.96, 41.35 (CH, C-9a), 33.01 (CH₂, C6/C7), 31.32, 30.85 (CH₂, C-1), 28.43, 28.14, 27.90 (CH₃, O*t*Bu), 27.41, 27.24 (CH₂, C-6/C-7).
**IR** (ATR) *ṽ* (cm⁻¹) = 2972 (m), 2928 (m), 2866 (w), 2245 (w), 1738 (s), 1700 (vs), 1677 (vs), 1476 (m), 1408 (vs), 1389 (vs), 1364 (vs), 1340 (s), 1327 (s),1255 (s),1218 (s), 1159 (vs), 1121 (s), 1072 (m), 920 (m), 833 (m), 771 (m), 730 (s), 654 (w).
**MS** (DIP-EI, 70 eV): m/z (%) = 392 ([M⁺], 3), 336 (4), 319 (9), 291 (13), 263 (10), 235 (84), 207 (25),193 (8),163 (28),146 (15),134 (7),120 (12), 94 (9), 82 (12), 69 (9), 57 (100).
**HR-MS** (EI, C₂₁H₃₂N₂O₅): ber.: 392.2311, gef.: 392.231.
**[α]_{λ}²⁰** = -212.6° (589 nm), -252.1° (546 nm), -514.6° (405 nm), -672.6° (365 nm), -860.7° (334 nm) (c = 1.015, CHCl₃).

### (3aS,SS,7aR,9aR)-3-(Fluorenyl-9-methoxycarbonyl)-4-oxo-1,3a,4,5,6,7,7a,9a-octahydro-2H-3,4a-diaza-cyclopenta[f]azulene-5-carbonsäure (85)

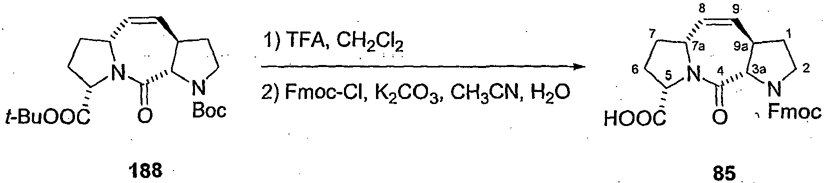

150 mg Metathese-Produkt **188** (0.38 mmol) wurden in 2 ml CH₂Cl₂ gelöst, auf 0 °C gekühlt und tropfenweise mit 2 ml TFA (99%) versetzt. Dann wurde der Ansatz auf RT gebracht und eine Stunde gerührt. Anschließend wurden alle flüchtigen Bestandteile im Vakuum entfernt, worauf man einen gelblichen Rückstand erhielt, der im ÖPV langsam erstarrte.

Das Rohprodukt wurde in einem Gemisch aus 4 ml Acetonitril und 3 ml 10%iger K₂CO₃-Lösung aufgenommen, im Eisbad auf 0 °C gekühlt und mit einer Lösung aus 200 mg Fmoc-Cl (0.77 mmol, 2 Äq.) in 2 ml Acetonitril versetzt. Nach Rühren über Nacht wurde der Ansatz wiederum auf 0 °C gekühlt, mit 50 CH₂Cl₂ verdünnt und mit 1 M HCl *vorsichtig* auf pH = 4 gebracht. Nach Phasentrennung wurde die wäßrige Phase mit 3x 10 ml CH₂Cl₂ extrahiert, bevor die vereinigten organischen Phasen über MgSO₄ getrocknet und eingeengt wurden. Der Rückstand wurde chromatographisch unter Verwendung einer etwas kürzeren Kieselgelsäule (CH₂Cl₂/MeOH 9:1, ggf. Gradient bis 8:2) gereinigt, wobei man 150 mg Fmoc-geschütztes Diprolin 85 (0.33 mmol, 86 %) in Form eines gelblichen Feststoffes erhielt.
**M (C₂₇H₂₆N₂O₅) = 458.5058.**
**R_{f}** = 0.16 (SiO₂, CH₂Cl₂/MeOH 9:1).
**Smp.**:163°C (Zersetzung).
**¹H-NMR** (500 MHz, CD₃OD): (Rotamerengemisch) δ (ppm) = 7.76-7-82 (m, 2H, Hₐᵣ), 7.48-7.65 (m, 2H, Hₐᵣ), 725-7.43 (m, 4H, Hₐᵣ), 5.75 (d, 0.4H, J = 11.1 Hz, H-8), 5.66 (d, 0.6H, J = 11.0 Hz, H-8), 5.55 (d, 0.4H, J = 11.0 Hz, H-9), 5.47 (d, 0.6H, J = 11.1 Hz, H-9), 4.73 (dd, 0.6H, J = 10.8 Hz, J = 5.3 Hz, CH_{2,Fmoc}), 4.65 (br, 0.4H, H-7a), 4.16-4.58 (m, 3.6H, CH_{2,Fmoc,} H-5, H-3a, H-7a, CH_{Fmoc}), 4.12 (t, 0.7H, J = 4.4 Hz, CH_{Fmoc}), 3.80 (d, 0.7H, J = 10.4 Hz, H-3a), 3.61 (dd, 0.6H, J = 8.1 Hz, J = 10.1 Hz, H-2_{α}), 3.54 (m, 0.4H, H-2_{α}), 3.13-3.30 (m, 1H, H-2_{β}), 2.75-2.95 (br, 1H, H-9a), 2.21-2.32 (m, 1H, H-7_{α}), 1.90-2.14 (m, 3H, H-6, H-1_{α}), 1.70-1.90 (br, 1H, H-7_{β}), 1.45-1.56 (m, 0.4H, H-1_{β}), 1.25-1.40 (m, 0.6H, H-1_{β}).
**¹³C-NMR** (125 MHz, CD₃OD): (Rotamerengemisch) δ (ppm) = 178.12, 178.01 (C=O_{C.säure}), 172.69, 172.37 (C=O),156.97,156.80 (C=OF_{moc}), 145.94, 145.51, 145.10, 144.93 (C_{q,ar}), 142.78, 142.71, 142.59 (C_{q,ar}), 130.56, 130.32 (CH_{olef.}), 129.43, 129.37 (CH_{olef.}), 128.85, 128.81, 128.71 (CHₐᵣ), 12824,128.17, 128.10 (CHₐᵣ), 126.19, 126.11, 125.96, 125.81 (CHₐᵣ), 120.99, 120.90, 120.80 (CHₐᵣ), 68.57, 67.29 (CH_{2,Fmoc}), 63.60, 63.34 (CH, C-3a), 59.16, 59.05 (CH, C-7a), 49.33, (CH, CH_{Fmoc}), 48.50 (CH, C-5), 48.22,48.13 (CH₂, C-2), 43.09, 42.46 (CH, C-9a), 34.13, 33.97 (CH₂, C-7), 32.17, 31.62 (CH₂, C-1), 28.31 (CH₂, C-6).
**IR** (ATR) *ṽ* (cm⁻¹) = 3419 (br), 2956 (m), 2880 (m), 2364 (w), 2238 (w), 1681 (vs, br), 1449 (vs), 1416 (vs), 1350 (s), 1327 (s), 1300 (m), 1240 (m), 1220 (m), 1169 (s), 1120 (s), 1021 (m), 985 (m), 908 (s), 839 (m), 759 (vs), 736 (vs).
MS (DIP-EI, 70 eV): m/z (%) = 236 [M⁺ -Fmoc], <1), 178 (Fluoren, 100), 152 (42), 126 (6), 98 (5), 89 (55), 76 (67), 63 (18), 51 (6).
**HR-MS** (ESI, C₂₇H₂₆N₂NaO₅) ber.:481.1740, gef.: 481.173.
**[α]λ²⁰**=-135.5° (589 nm), -160.4° (546 nm), -318.3 (405 nm), -406.0° (365 nm), absorbiert bei 334 nm (c = 0.86, MeOH).

Die genannten Verbindungen zeigen eine überraschend gute Affinität zu Proteinen, die die Src-Homologie-3-Domänen, WW-Domänen, Ena-VASP-Homologie-1-Domänen, GYF-Domänen, UEV-Domänen und/oder Profilin aufweisen. Diese überraschend gute Affinität ist einsetzbar, um virale oder bakterielle Erkrankungen zu diagnostizieren oder zu behandeln, in denen Proteine involviert sind, die die genannten Domänen aufweisen. Neben den genannten Infektionskrankheiten sind derartige Proteine auch bei neurodegenerativen Erkrankungen und Tumorerkrankungen involviert. Versuche in Testorganismen zeigen die gute Verträglichkeit der erfindungsgemäßen Verbindungen, auch wenn sie mit anderen Molekülen kombiniert verwendet werden.

### 8 Abkürzungen

- abs.: absolut
- Äq.: Äquivalent(e)
- Ar: Aryl
- ATR: Attenuated Total Internal Reflectance
- 9-BBN: 9-Borabicyclononan
- ber.: berechnet
- Bn: Benzyl
- Boc: *tert-*Butoxycarbonyl
- Boc₂O: Di-*tert*-butyldicarbonat (Boc-Anhydrid)
- CH: Cyclohexan
- Cy: Cyclohexyl
- DC: Dünnschichtchromatogramm
- DCE: Dichlorethan (1,2-)
- DCM: Dichlormethan
- DIBAL-H: Diisobutylaluminiumhydrid
- DIC: Diisopropylcarbodiimid
- DIP: Direkteinlass (Massenspektrometrie)
- DIPEA: Diisopropylethylamin
- DMAP: 4-*N,N*-Dimethylaminopyridin
- DMF: *N,N*-Dimethylformamid
- DMP: 2,2-Dimethoxypropan
- DMS: Dimethylsulfid
- DMSO: Dimethylsulfoxid
- dr: Diastereomerenverhältnis (diastereomeric ratio)
- EE: Ethylacetat
- ee: Enantiomerenüberschuss (enantiomeric excess)
- EI: Elektronenstoßionisation
- ESI: Elektronensprayionisation
- Et: Ethyl
- Et₂O: Diethylether
- EtOH: Ethanol
- EVH1: Ena-VASP-Homologie-1-Domäne
- FGI: Funktionelle-Gruppen-Chemie (function group inversion)
- Fmoc: Fluorenyl-9-methoxycarbonyl
- FMP: Forschungsinstitut für Molekulare Pharmakologie (Berlin-Buch)
- GC-MS: Gaschromatographie mit angeschlossener Massenspektrometrie
- gef.: gefunden
- ges.: gesättigt
- HPLC: High Performance Liquid Chromatography
- HRMS: hochaufgelöste Massenspektrometrie
- IR: Infrarotspektroskopie
- konz.: konzentriert
- LiHMDS: Lithium-hexamethyldisilazid
- M: molare Masse
- MCPBA: *meta*-Chlorperbenzoesäure
- Me: Methyl
- MeOH: Methanol
- Ms: Mesyl (Methansulfonyl)
- MS: Massenspektrometrie
- MTBE: Methyl-*tert*-butylether
- NaHMDS: Natrium-hexamethyldisilazid
- NME: *N*-Methylephedrin
- NMR: Magnetische Kernresonanzspektroskopie
- NOE: Nuclear Overhauser Effect
- *o*: *ortho*
- Ph: Phenyl
- PPII: Polyprolin-Helix Typ 11
- POM-Cl: Chlormethylpivaloat
- PPTS: Pyridinium-*para*-toluolsulfonat
- RCM: Ringschlussmetathese
- R_{f}: Retentionsfaktor
- [Ru]ₗₗ: Grubbs-ll-Katalysator **81**
- [Ru]_{gr}: modifizierter (grüner) Grubbs-Hoveyda-Katalysator nach Blechert 82
- *s*: sekundär
- Smp.: Schmelzpunkt
- Su: Succinimid
- TBAF: Tetrabutylammoniumfluorid
- TBS: *tert*-Butyldimethylsilyl
- *t*-Bu: *tert*-Butyl
- Tf: Trifluormethansulfonyl
- TFA: Trifluoressigsäure
- THF: Tetrahydrofuran
- TMEDA: Tetramethylethylendiamin
- TMS: Trimethylsilyl
- TMSOTf: Trimethylsilyl-trifluormethansulfonat
- TPS: *tert*-Butyldiphenylsilyl
- Ts: Tosyl (*para*-Toluolsulfonyl)
- TsOH: *para*-Toluolsulfonsäure
- Z: Benzyloxycarbonyl- (auch Cbz)

Ein-Buchstaben-Code und Drei-Buchstaben-Code natürlicher proteinogener Aminosäuren:

| | | | | | |
|---|---|---|---|---|---|
| A | Ala | Alanin | M | Met | Methionin |
| C | Cys | Cystein | N | Asn | Asparagin |
| D | Asp | Asparaginsäure | P | Pro | Prolin |
| E | Glu | Glutaminsäure | Q | Gln | Glutamin |
| F | Phe | Phenylalanin | R | Arg | Arginin |
| G | Gly | Glycin | S | Ser | Serin |
| H | His | Histidin | T | Thr | Threonin |
| I | Ile | Isoleucin | V | Val | Valin |
| K | Lys | Lysin | W | Trp | Tryptophan |
| L | Leu | Leucin | Y | Tyr | Tyrosin |

## Patentansprüche

1. Verbindungen gemäß der allgemeinen Formel 1 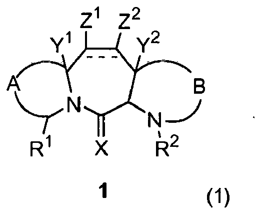 mit einem gesättigten oder ungesättigten zentralen siebengliedrigen Ring, wobei
X = O und/oder S sind;
A, B = Ringbrücken sind;
Y¹, Y² = H, Alkyl, Fluoralkyl, Aryl und/oder Heteroaryl sind;
Z¹, Z² = H, Carbonyl, OH, O-Alkyl, O-Acyl, N-R¹R² mit R¹ bzw. R² = H, Alkyl, Acyl, Sulfonyl, Alkyl, Acyl, Fluoralkyl, Aryl und/oder Heteroaryl sind;
R¹ = Alkyl, Acyl, Alkoxycarbonyl, Aryloxycarbonyl und/oder Aminocarbonyl (CONH₂, CONHR, CONH-Peptidyl, (mit R)) ist;
R² = H, Alkyl, Aryl, Alkylcarbonyl, Arylcarbonyl, Alkoxycarbonyl, Aminocarbonyl, Aryloxycarbonyl, Alkylsulfonyl, Arylsulfonyl, Aminoacyl und/oder Peptidyl sind.

2. Verbindung nach Anspruch 1
**dadurch gekennzeichnet, dass**
A und/oder B 5 und/oder 6 Ringatome sind, wobei die durch A und B repräsentierten Ringglieder ausgewählt sind aus der Gruppe umfassend C-, O-, S- und/oder N-Atome.

3. Verbindung nach Anspruch 1 oder 2 gemäß der allgemeinen Formel 2 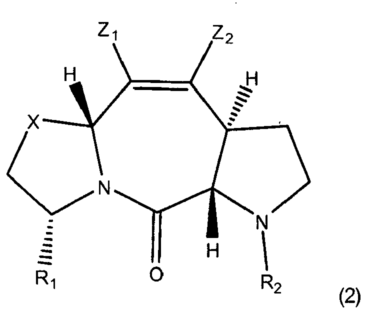
mit Z¹, Z² wie für Struktur 1 angegeben und der im Formelbild 2 gezeigten Konfiguration,
mit R¹, R² = Alkyl, Acyl, Hetaryl, Sulfonyl,
mit X = -CH₂-, -O-, -S- und -NH-R.

4. Verbindung nach Anspruch 1 bis 3,
**dadurch gekennzeichnet, dass**
der bevorzugten Ausführungsform gemäß der in Formel 3 gezeigten Strukturen entsprechen 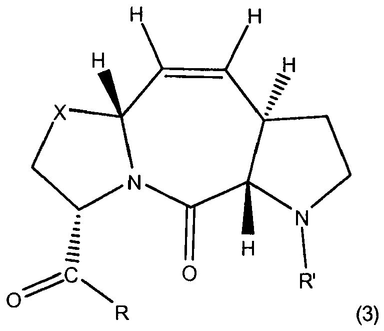
mit X = -CH₂-, -O-, -S-,
mit R = NH-R", -O-R", mit R" = Peptidyl, substituierte Alkyle, Hetaryl,
mit R' = Acyl, Peptidyl, Sulfonyl.

5. Verbindung gemäß einem der Ansprüche 1 bis 4 als pharmazeutischer Wirkstoff.

6. Verbindung gemäß einem der Ansprüche 1 bis 5, wobei die Verbindung ein Ligand für eine Domäne ausgewählt aus der Gruppe umfassend Src-Homologie-3-Domänen, WW-Domänen, Ena-VASP-Homologie-1-Domänen, GYF-Domänen, UEV-Domänen und/oder Profilin ist.

7. Verbindung nach dem vorhergehenden Anspruch, wobei die Verbindung gemäß den Ansprüchen 1 bis 5 ein Poly-Prolin-Mimetikum ist.

8. Verbindung nach einem der Ansprüche 1 bis 4 zur Verwendung als Arzneimittel zur Behandlung von Krankheiten, die mit der Modifikation von intrazellulären Signaltransduktionsvorgängen assoziiert sind, die durch Poly-Prolin-Helix-Strukturen vermittelt werden, ausgewählt aus der Gruppe umfassend bakterielle und/odervirale Infektionskrankheiten, neurodegenerative Erkrankungen und/oder Tumorerkrankungen.

9. Verbindung nach dem vorhergehenden Anspruch zur Verwendung als Arzneimittel,
**dadurch gekennzeichnet, dass**
die bakteriellen Erkrankungen durch Bakterien ausgelöst werden, ausgewählt aus der Gruppe umfassend Legionellen, Streptokokken, Staphylokokken, Klebsiellen, Haemophilis influenzae, Rickettsien (Fleckfieber), Mykobakterien, Mykoplasmen, Ureaplasmen, Neisserien (Meningitis, Waterhouse-Friedrichsen-Syndrom, Gonorrhoe), Pseudomonaden, Bordetellen (Pertussis), Corynobakterien (Diphtherie), Chlamydien, Campylobacter (Durchfall), Escherichia coli, Proteus, Salmonellen, Shigellen, Yersinien, Vibrionen, Enterokokken, Clostridien, Borrelien, Treponema pallidum, Brucellen, Francisellen und/oder Leptospira, insbesondere Listerien und/oder die virale Infektionskrankheit eine Hepatitis und/oder HIV-Erkrankung ist.

10. Verbindung nach dem vorhergehenden Anspruch zur Verwendung als Arzneimittel,
**dadurch gekennzeichnet, dass**
die Listerien ausgewählt sind aus der Gruppe umfassend L. monocytogenes Sv1/2a, L. monocytogenes Sv4b F2365, L. monocytogenes Sv4b H7858, 178 contigs, L. monocytogenes Sv1/2a F6854,133 contigs, L. monocytogenes Sv4b, L. monocytogenes Sv4a, L. innocua Sv6a, L. welshimeri Sv6b, L. seeligeri Sv1/2b und/oder L. ivanovii Sv5.

11. Verbindung nach Anspruch 8 zur Verwendung als Arzneimittel,
**dadurch gekennzeichnet, dass**
die neurodegenerative Erkrankung ausgewählt ist aus der Gruppe umfassend Morbus Alzheimer, Morbus Parkinson, Morbus Huntington und/oder amyotrophische Lateralslklerose (ALS).

12. Verbindung nach Anspruch 8 zur Verwendung als Arzneimittel,
**dadurch gekennzeichnet, dass**
die Tumorerkrankung ein Karzinom, ein Sarkom, ein neuroendokriner Tumor, ein hämoonkologischer Tumor, ein dysontogenetischer Tumor und/oder ein Mischtumor ist.

13. Verbindung nach einem der vorhergehenden Ansprüche zur Verwendung als Arzneimittel zur Modulation von onkogener Proteinaktivität oder zur Modulierung von Signaltransduktionswegen.

14. Verfahren zur Identifizierung eines Inhibitors für die Bindung zwischen dem ersten Molekül, das eine Domäne ausgewählt aus der Gruppe umfassend Src-Homologie-3-Domänen, WW-Domänen, Ena-VASP-Homologie-1-Domänen, GYF-Domänen, UEV-Domänen und/oder Profilin umfasst, und einem zweiten Molekül, das an diese Domänen bindet, wobei das zweite Molekül ein Mittel ist, welches mindestens eine Verbindung gemäß einem der Ansprüche 1 bis 5 umfasst, umfassend das Inkubieren von einem oder mehreren Mitteln, aus denen ein solcher Inhibitor ausgewählt werden soll, mit einem ersten Molekül und dem zweiten Molekül, unter Bedingungen, die für die Bindung und den Nachweis einer oder mehrerer Verbindungen, die die Bindung des ersten Moleküls an das zweite Molekül inhibieren, geeignet sind.

15. Verfahren zur Identifizierung einer Verbindung, die die Bindung eines Moleküls, das eine Domäne ausgewählt aus der Gruppe umfassend Src-Homologie-3-Domänen, WW-Domänen, Ena-VASP-Homologie-1-Domänen, GYF-Domänen, UEV-Domänen und/oder Profilin umfasst, mit einer Verbindung gemäß einem der Ansprüche 1 bis 5, beeinflusst, umfassend die folgenden Schritte:
(a) In-Kontakt-Bringen der Src-Homologie-3-Domänen, WW-Domänen, Ena-VASP-Homologie-1-Domänen, GYF-Domänen, UEV-Domänen und/oder Profilin mit einer Verbindung gemäß einem der Ansprüche 1 bis 5 unter Bedingungen, die geeignet sind für die Bindung, in Anwesenheit einer Kandidatenverbindung und Messen des Umfangs der Bindung zwischen der Domäne und der Verbindung; und
(b) Vergleichen des in Schritt (a) gemessenen Umfangs der Bindung mit dem Umfang der Bindung, von dem bekannt ist oder bestimmt wurde, dass er zwischen der Domäne und der Verbindung in Abwesenheit der Kandidatenverbindung auftritt, wobei der Unterschied zwischen dem im ersten Schritt gemessenen Umfang der Bindung und dem Umfang der Bindung, von dem bekannt ist oder bestimmt wurde, dass er zwischen der Domäne und dem Liganden in Abwesenheit der Kandidatenverbindung auftritt, darauf hinweist, dass die Kandidatenverbindung eine Verbindung ist, die die Bindung zwischen der Domäne und der Verbindung gemäß einem der Ansprüche 1 bis 5 beeinflusst.

## Claims

1. Compounds according to general formula 1 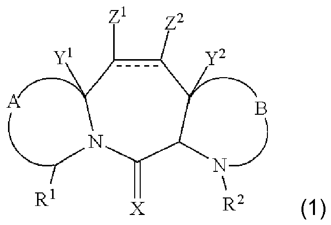 with a saturated or unsaturated central seven-membered ring, whereby
X is O and/or S;
A, B are ring bridges;
Y¹, Y² are H, alkyl, fluoroalkyl, aryl and/or heteroaryl;
Z¹, Z² are H, carbonyl, OH, O-alkyl, O-acyl, N-R¹R² with R¹ and/or R² = H, alkyl, acyl, sulfonyl, alkyl, fluoroalkyl, aryl and/or heteroaryl;
R¹ is alkyl, acyl, alkoxycarbonyl, aryloxycarbonyl and/or aminocarbonyl (CONH₂, CONHR, CONH-peptidyl, (with R));
R² is H, alkyl, aryl, alkylcarbonyl, arylcarbonyl, alkoxycarbonyl, aminocarbonyl, aryloxycarbonyl, alkylsulfonyl, arylsulfonyl, aminoacyl and/or peptidyl.

2. The compound according to claim 1,
**characterized in that**
A and/or B are 5 and/or 6 ring atoms, whereby the ring members represented by A and B are selected from the group comprising C, O, S and/or N atoms.

3. The compound according to claim 1 or 2 according to the general formula 2 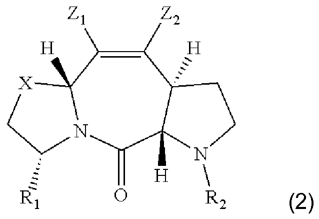
with Z¹, Z² as represented in general formula 1 and with a configuration as shown in formula 2;
with R¹, R² = alkyl, acyl, hetaryl, sulfonyl,
with X = CH₂₋, -O-, -S- and -NH-R.

4. The compound according to claims 1 to 3,
**characterized in that**
the preferred embodiments according to the structures shown in formula 3 correspond to: 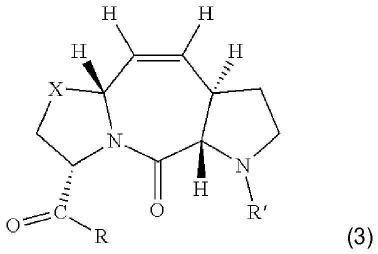
with X = -CH₂-, -O-, -S-;
with R = NH-R", -O-R", with R" = peptidyl, substituted alkyls, hetaryl;
with R' = acyl, peptidyl, sulfonyl.

5. The compound according to one of claims 1 to 4 as a pharmaceutical active substance.

6. The compound according to one of claims 1 to 5, whereby the compound is a ligand for a domain selected from the group comprising Src homology 3 domains, WW domains, Ena/VASP homology 1 domains, GYF domains, UEV domains and/or profilin.

7. The compound of the preceding claim, whereby the compound according to the claims 1 to 5 is a polyproline mimetic.

8. The compound according to one of claims 1 to 4 for use as a medicament for the treatment of diseases associated with a modification of intracellular signal transduction processes mediated by polyproline helical structures, selected from the group comprising bacterial and/or viral infectious diseases, neurodegenerative diseases and/or tumor diseases.

9. The compound of the preceding claim for use as a medicament,
**characterized in that**
the bacterial diseases are induced by bacteria selected from the group comprising legionellas, streptococci, staphylococci, klebsiellas, *Haemophilis influenzae,* rickettsiae (typhoid fever), mycobacteria, mycoplasmas, ureaplasmas, neisseriae (meningitis, Waterhouse-Friedrichsen syndrome, gonorrhea), pseudomonads, bordetellas (pertussis), *corynebacteria* (diphtheria), chlamydiae, campylobacteria (diarrhea), *Escherichia coli, proteus,* salmonellas, shigellas, yersiniae, vibrions, enterococci, clostridiae, borreliae, *Treponema pallidum,* brucellas, francisellas and/or *Leptospira,* particularly listeriae and/or the viral infectious disease is a hepititits and/or HIV-disease.

10. The compound of the preceding claim for use as a medicament,
**characterized in that**
the listeriae are selected from the group comprising L. *monocytogenes* Sv1/2a, L. *monocytogenes* Sv4b F2365, *L. monocytogenes* Sv4b H7858,178 contigs, *L. monocytogenes* Sv1/2a F6854,133 contigs, *L. monocytogenes* Sv4b, *L. monocytogenes* Sv4a, *L. innocua* Sv6a, *L. welshimeri* Sv6b, *L. seeligeri* Sv1/2b and/or *L. ivanovii* Sv5.

11. The compound of claim 8 for use as a medicament,
**characterized in that**
the neurodegenerative disease is selected from the group comprising Alzheimer's disease, Parkinson's disease, Huntington's disease and/or amyotrophic lateral sclerosis (ALS).

12. The compound of claim 8 for use as a medicament,
**characterized in that**
the tumor disease is a carcinoma, a sarcoma, a neuroendocrine tumor, a hemo-oncologic tumor, a dysontogenetic tumor and/or a mixed tumor.

13. The compound according to one of the preceding claims for use as a medicament for the modulation of oncogenic protein activity or modulation of signal transduction pathways.

14. A method of identifying an inhibitor of binding between a first molecule, which comprises a domain selected from the group comprising Src homology 3 domains, WW domains, Ena/VASP homology 1 domains, GYF domains, UEV domains and/or profilin, and a second molecule, which binds to said domain, the second molecule is an agent comprising at least one compound according to one of claims 1 to 5, said method comprising incubation of one or more agents, from which such an inhibitor is to be selected, with the first molecule and the second molecule under conditions suitable for binding and detection of one or more compounds which inhibit binding of the first molecule to the second molecule.

15. A method of identifying a compound, which influences binding of a molecule comprising a domain selected from the group comprising Src homology 3 domains, WW domains, Ena/VASP homology 1 domains, GYF domains, UEV domains and/or profilin with a compound according to one of claims 1 to 5, said method comprising the following steps:
(a) contacting the Src homology 3 domains, WW domains, Ena/VASP homology 1 domains, GYF domains, UEV domains and/or profilin with a compound according to one of claims 1 to 5 under conditions suitable for binding in the presence of a candidate compound and measuring the extent of binding between the domain and the compound; and
(b) comparing the extent of binding measured in (a) with an extent of binding which is known or has been determined to exist between the domain and the compound in the absence of a candidate compound, whereby the difference between the extent of binding measured in (a) and the extent of binding which is known or has been determined to exist between the domain and the ligand in the absence of a candidate compound, indicates that the candidate compound is a compound that influences binding between the domain and the compound according to one of claims 1 to 5.

## Revendications

1. Composés répondant à la formule générale 1 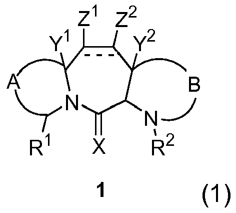 comprenant un cycle central à sept membres, saturé ou insaturé, formule dans laquelle :
X représente un atome d'oxygène et/ou un atome de soufre ;
A, B représentent des ponts cycliques ;
Y1, Y2 représentent un atome d'hydrogène, un groupe alkyle, un groupe fluoroalkyle, un groupe aryle et/ou un groupe hétéroaryle ;
Z1, Z2 représentent un atome d'hydrogène, un groupe carbonyle, un groupe OH, un groupe O-alkyle, un groupe O-acyle, un groupe N-R1R2 dans lequel R1 respectivement R2 représente un atome d'hydrogène, un groupe alkyle, un groupe acyle, un groupe sulfonyle, un groupe alkyle, un groupe acyle, un groupe fluoroalkyle, un groupe aryle et/ou un groupe hétéroaryle ;
R1 représente un groupe alkyle, un groupe acyle, un groupe alcoxycarbonyle, un groupe aryloxycarbonyle et/ou un groupe aminocarbonyle (un groupe CONH2, un groupe CONHR, un groupe CONH-peptidyle, (où R)) ;
R2 représente un atome d'hydrogène, un groupe alkyle, un groupe aryle, un groupe alkylcarbonyle, un groupe arylcarbonyle, un groupe alcoxycarbonyle, un groupe aminocarbonyle, un groupe aryloxycarbonyle, un groupe alkylsulfonyle, un groupe arylsulfonyle, un groupe aminoacyle et/ou un groupe peptidyle.

2. Composé selon la revendication 1, **caractérisé en ce que** A et/ou B représentent 5 et/ou 6 atomes cycliques, les membres cycliques représentés par A et B étant choisis parmi le groupe comprenant des atomes de carbone, d'oxygène, de soufre et/ou d'azote.

3. Composé selon la revendication 1 ou 2, répondant à la formule générale 2 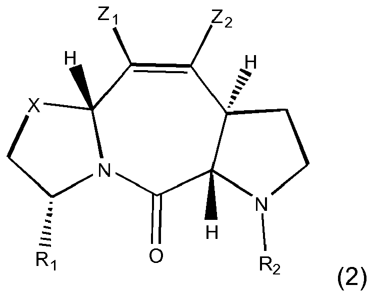
dans laquelle Z1, Z2 sont tels qu'indiqués pour la structure 1 et possèdent la configuration représentée dans la formule développée 2 ;
dans laquelle R1, R2 représentent un groupe alkyle, un groupe acyle, un groupe hétaryle, un groupe sulfonyle ;
X représente un groupe -CH2-, un atome d'oxygène, un atome de soufre et un groupe -NH-R.

4. Composé selon les revendications 1 à 3, **caractérisé en ce que** les structures répondant à la formule 3 correspondent à la forme de réalisation préférée : 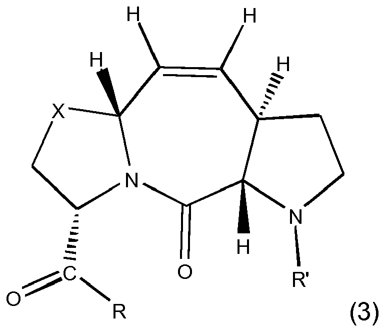
dans laquelle X représente un groupe -CH2-, un atome d'oxygène, un atome de soufre ;
R représente un groupe NH-R", un groupe -O-R", où R" représente un groupe peptidyle, des groupes alkyle substitués, un groupe hétaryle ;
R' représente un groupe acyle, un groupe peptidyle, un groupe sulfonyle.

5. Composé selon l'une quelconque des revendications 1 à 4, à titre de substance active pharmaceutique.

6. Composé selon l'une quelconque des revendications 1 à 5, dans lequel le composé représente un ligand pour un domaine choisi parmi le groupe comprenant les domaines d'homologie 3 Src, les domaines WW, les domaines d'homologie 1 Ena/VASP, les domaines GYF, les domaines UEV et/ou la profiline.

7. Composé selon la revendication précédente, dans lequel le composé selon les revendications 1 à 5 est un mimétique de la polyproline.

8. Composé selon l'une quelconque des revendications 1 à 4, pour son utilisation comme médicament pour le traitement de maladies qui sont associées à la modification de processus intracellulaires de transduction de signaux qui sont déterminés par des structures d'hélice polyproline, choisies parmi le groupe comprenant des maladies infectieuses d'origine bactérienne et/ou d'origine virale, des maladies neurodégénératives et/ou des maladies tumorales.

9. Composé selon la revendication précédente pour son utilisation comme médicament, **caractérisé en ce que** les maladies bactériennes sont déclenchées par des bactéries choisies parmi le groupe comprenant des légionnelles, des streptocoques, des staphylocoques, des klebsiellles, Hemophilis influenzae, des rickettsies (typhus), des mycobactéries, des mycoplasmes, des uréaplasmes, des Neisseria (méningite, syndrome de Waterhouse-Friedrichsen, blennorragie), des Pseudomonas, des bordetelles (Pertussis), des corynebactéries (diphtérie), des chlamydes, Campylobacter (diarrhée), Escherichia coli, Proteus, des salmonelles, des shigelles, des yersinies, des vibrions, des entérocoques, des clostridies, des borrélies, Treponema pallidum, des brucelles, des franciselles et/ou Leptospira, en particulier les listeria et/ou la maladie infectieuse d'origine virale, l'hépatite et/ou la maladie à VIH.

10. Composé selon la revendication précédente pour son utilisation comme médicament, **caractérisé en ce que** les listeria sont choisies parmi le groupe comprenant L. monocytogenes Sv1/2a, L. monocytogenes Sv4b F2365, L. monocytogenes Sv4b H7858, 178 contigs, L. monocytogenes Sv1/2a F6854, 133 contigs, L. monocytogenes Sv4b, L. monocytogenes Sv4a, L. innocua Sv6a, L. welshimeri Sv6b, L. seeligeri Sv1/2b et/ou L. ivanovii Sv5.

11. Composé selon la revendication 8 pour son utilisation comme médicament, **caractérisé en ce que** la maladie neurodégénérative est choisie parmi le groupe comprenant la maladie d'Alzheimer, la maladie de Parkinson, la maladie d'Huntington et/ou la sclérose latérale amyotrophique (ALS).

12. Composé selon la revendication 8 pour son utilisation comme médicament, **caractérisé en ce que** la maladie tumorale est un carcinome, un sarcome, une tumeur neuroendocrinienne, une tumeur hémato-oncologique, une tumeur dysontogénétique et/ou une tumeur mixte.

13. Composé selon l'une quelconque des revendications précédentes pour son utilisation comme médicament pour la modulation de l'activité protéique oncogène ou pour la modulation de voies de transduction de signaux.

14. Procédé pour l'identification d'un inhibiteur pour la liaison entre la première molécule qui comprend un domaine choisi parmi le groupe comprenant les domaines d'homologie 3 Src, les domaines WW, les domaines d'homologie 1 Ena/VASP, les domaines GYF, les domaines UEV et/ou la profiline, et une deuxième molécule qui se lie à ces domaines, la deuxième molécule représentant un agent qui comprend au moins un composé selon l'une quelconque des revendications 1 à 5, comprenant l'incubation d'un ou de plusieurs agents à partir desquels un tel inhibiteur doit être sélectionné, avec une première molécule et la deuxième molécule, dans des conditions qui sont appropriées pour la liaison et le décèlement d'un ou de plusieurs composés qui inhibent la liaison de la première molécule à la deuxième molécule.

15. Procédé pour l'identification d'un composé qui influence la liaison d'une molécule qui comprend un domaine choisi parmi le groupe comprenant les domaines d'homologie 3 Src, les domaines WW, les domaines d'homologie 1 Ena/VASP, les domaines GYF, les domaines UEV et/ou la profiline, avec un composé selon l'une quelconque des revendications 1 à 5, comprenant les étapes suivantes dans lesquelles :
(a) on met les domaines d'homologie 3 Src, les domaines WW, les domaines d'homologie 1 Ena/VASP, les domaines GYF, les domaines UEV et/ou la profiline en contact avec un composé selon l'une quelconque des revendications 1 à 5 dans des conditions qui sont appropriées pour la liaison, en présence d'un composé candidat, et on mesure l'ampleur de la liaison entre les domaines et le composé ; et
(b) on compare l'ampleur de la liaison mesurée à l'étape (a) à l'ampleur de la liaison à partir de laquelle on sait ou on a déterminé qu'elle intervient entre les domaines et le composé en l'absence du composé candidat, la différence entre la portée de la liaison mesurée dans la première étape et la portée de la liaison à partir de laquelle on sait ou on a déterminé qu'elle intervient entre les domaines et le ligand en l'absence du composé candidat, indiquant que le composé candidat représente un composé qui influence la liaison entre les domaines et le composé selon l'une quelconque des revendications 1 à 5.
